# EUROPEAN PATENT SPECIFICATION

(11) **EP 1 403 251 B1**
(45) Date of publication and mention of the grant of the patent: **18.07.2007**
(21) Application number: 02738821.4
(22) Date of filing: 27.06.2002
(51) Int. Cl.: C07D 213/74, C07D 405/14, C07D 401/14, A61K 31/44, A61K 31/4427, A61K 31/496, A61K 31/4523, A61K 31/551, A61K 31/4439, A61K 31/4545, A61K 31/443, A61P 37/00

(54) **BIS(2-ARYL-5-PYRIDYL) DERIVATIVE**
BIS(2-ARYL-5-PYRIDYL)DERIVATE
DERIVE DE BIS(2-ARYL-5-PYRIDYLE)

(30) Priority: 29.06.2001 US 893680
(43) Date of publication of application: 31.03.2004
(73) Proprietor: Kowa Co., Ltd., Nagoya-shi, Aichi 460-8625 (JP)
(72) Inventor: ISHIWATA, Hiroyuki, ICHIKAWA-SHI, Chiba 272-0824 (JP); SATO, Seiichi, SUGINAMI-KU, Tokyo 167-0043 (JP); KABEYA, Mototsugu, HIGASHIMURAYAMA-SHI, Tokyo 189-0022 (JP); ODA, Soichi, Nishikasugai-gun Aichi 452-0055 (JP); SUDA, Makoto, Tokyo 189-0022 (JP); SHIBASAKI, Manabu, CHIBA-SHI, Chiba 260-0851 (JP)
(74) Representative: Hartz, Nikolai
(86) International application number: PCT/JP2002/006493
(87) International publication number: WO 2003/002538

(56) References cited:
- EP-A1- 0 926 138
- EP-A1- 1 052 238
- WO-A1-98/18782

## Description

### Technical Field

This invention relates to novel bis(2-aryl-5-pyridyl) derivatives or salts thereof, and also to medicines which comprise the bis (2-aryl-5-pyridyl) derivatives or salts thereof as active ingredients and are useful for the prevention or treatment of allergic immune diseases.

### Background Art

IgE, a class of immunoglobulin (Ig), is a allergen-specific molecule produced by IgE producing cells differentiated from B cells, when triggered by contact of immunocompetent cells with an allergen in the body.

IgE is produced in a target organ of allergy, and binds to a receptor on surfaces of mast cells or basophils as principal effector cells in an allergic reaction (sensitized state). From the mast cells stimulated as a result of intrusion of the allergen into the body after the sensitization and its reaction with the specific IgE, histamine, a leucotriene, a prostaglandin, an allergic chemical transmitter such as PAF, or a detrimental enzyme such as tryptase is released, and an immediate-response allergic reaction such as increased vasopermeability, smooth muscle constriction or vasodilation is provoked. From the stimulated mast cells, a cytokine capable of directly activating other immune system cells, such as IL-4, is also secreted. As a result, eosinophils, basophils or the like infiltrate tissues, and an allergic chemical transmitter or a histodetrimental protein such as MBP, which is secreted by these inflammatory cells, induces a delayed allergic reaction and protracts and worsen an allergic symptom.

Hence, the abnormal production of IgE is associated deeply with various allergic immune diseases, such as asthma, atopic dermatitis, allergic rhinitis, inflammatory colitis, contact-type dermatitis and allergic eye diseases. These diseases, however, are widely known to be preventable and curable if such production is effectively blocked (Emerging therapeutic targets in asthma and allergy: modulation of IgE. Emerging Therapeutic Targets (1999) 3: 229-240 ; Anti-IgE as novel therapy for the treatment of asthma. Curr. Opin. Plum. Med. (1999) 5: 76-80 ; Treatment of allergic asthma with monoclonal anti-IgE antibody. N. Eng. J. Med. (1999) 341: 1966-1973 ; Anti-IgE antibody therapy for asthma. N. Eng. J. Med. (1999) 341: 2006-2008).

From the foregoing, IgE is considered to be a substance which takes part at the stage of very beginning upon onset of an allergic disease. With the aim of developing antiallergic agents, some low molecular compounds with IgE antibody production inhibiting activity have been found and reported to date [WO98/04058, WO98/07702, WO98/16497, JP10-324631A, WO99/19291, WO99/35140, WO99/38829, WO99/42446, JP11-269192A, WO00/05198, "Yakuri to Chiryo (Basic Pharmacology & Therapeutic)" **22**(3), 1369 (1994), JP1-106818A, JP7-17506B, JP8-92216A, JP8-109177A, WO96/11682, JP59-167564A]. These compounds, however, involve problems such as low water solubility, and it is the current circumstance that the aim has not been fully achieved.

An object of the present invention, therefore, is to provide a compound having excellent IgE antibody production inhibiting activity and also a medicine comprising the compound as an active ingredient.

### Disclosure of the Invention

Under such circumstances, the present inventors have proceeded with extensive research. As a result, it has been found that compounds represented by the below-described formula (1) have excellent IgE antibody production inhibiting activity and also good water solubility and hence, are useful as medicines for the prevention or treatment of allergic immune diseases, leading to the completion of the present invention.

Accordingly, the present invention provides a bis (2-aryl-5-pyridyl) derivative represented by the following formula (1) or a salt thereof: 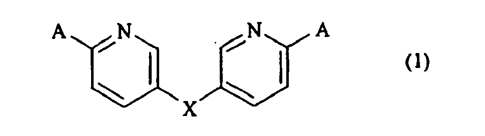 wherein A represents a substituted or unsubstituted aromatic hydrocarbon group, and X represents a group selected from the following formulas (2) to (4) : 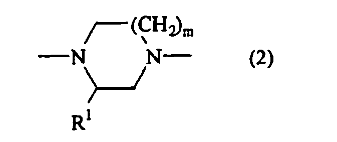 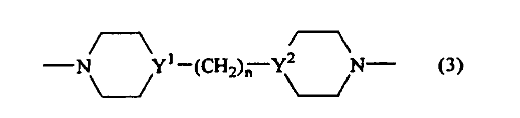 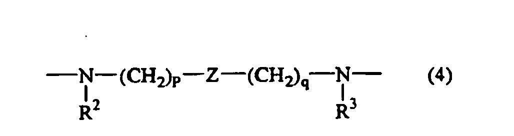 wherein in the formula (2), R¹ represents a hydrogen atom or a di(lower alkyl)amino(lower alkyl) group, and m stands for an integer of 1 or 2; in the formula (3), Y¹ and Y² represent a nitrogen atom or a CH group, and n stands for an integer of 0 to 6; in the formula (4), R² and R³ represent a hydrogen atom or a lower alkyl group, Z represents a single bond, a substituted methylene group, a substituted imino group, an oxygen atom or a cycloalkylene group, and p and q stand for an integer of 0 to 6.

The present invention also provides a medicine comprising as an active ingredient the bis(2-aryl-5-pyridyl) derivative or the salt thereof.

The present invention also provides use of the bis(2-aryl-5-pyridyl) derivative and the salt thereof for the manufacture of a medicine.

The present invention also provides a medicinal composition comprising the bis(2-aryl-5-pyridyl) derivative or the salt thereof and a pharmacologically acceptable carrier.

The present invention also provides a treatment method for an allergic immune disease, which comprises administering the bis(2-aryl-5-pyridyl) derivative or the salt thereof.

### Best Modes for Carrying Out the Invention

The lower alkyl moieties in "lower alkyl groups", "lower alkoxy groups", "halogeno(lower alkoxy)groups", "lower alkoxy(lower alkoxy) groups", "hydroxy(lower alkoxy) groups", "(lower alkoxy)carbonyl groups", "lower alkanoyl groups", "lower alkanoyloxy groups", (lower alkyl)thio groups" "(lower alkyl) amino groups", "(lower alkyl)sulfonylamino groups" as used herein are linear, branched or cyclic alkyl groups having 1 to 6 carbon atoms. Their examples can include methyl, ethyl, n-propyl, isopropyl, n-butyl, isobutyl, sec-butyl, tert-butyl, pentyl, hexyl, cyclopropyl, and cyclohexyl. On the other hand, illustrative of "halogen atoms" are fluorine atom, chlorine atom, bromine atom, and iodine atom.

In the formula (1), the aromatic hydrocarbon group represented by A may preferably be one having 6 to 14 carbon atoms, with a phenyl or naphthyl group being more preferred and a phenyl group being particularly preferred.

These groups may contain 1 to 3 substituents. Examples of such substituents can include lower alkyl groups, lower alkoxy groups, halogeno(lower alkoxy)groups, lower alkoxy(lower alkoxy) groups, hydroxy(lower alkoxy) groups, carboxyl groups, (lower alkoxy)carbonyl groups, unsubstituted or (lower alkyl)- and/or (lower alkoxy)-substituted carbamoyl groups, lower alkanoyl groups, formyl groups, lower alkanoyloxy groups, halogen atoms, hydroxy groups, cyano groups, (lower alkyl)thio groups, amino groups, mono- or di-(lower alkyl)amino groups, (lower alkyl)sufonylamino groups, pyrrolidinyl groups, and alkylenedioxy groups.

Preferred specific examples of these substituents can include methyl, methoxy, ethoxy, n-propoxy, isopropoxy, trifluoroethoxy, methoxyethoxy, hydroxyethoxy, F, Cl, hydroxy, cyano, methylthio, dimethylamino, pyrrolidinyl, formyl, carboxy, ethoxycarbonyl, t-butoxycarbonyl, propionyloxy, N-methyl-N-methoxycarbamoyl, acetyl, and methylenedioxy.

Among the groups represented by X, the group of the formula (2) contains the di(lower alkyl)amino(lower alkyl) group represented by R¹. As the di(lower alkyl)amino group in the di(lower alkyl)amino(lower alkyl) group, a dimethylamino group or the like is particularly preferred.

In the formula (3), n may preferably range from 2 to 4.

In the formula (4), the substituted methylene group represented by Z contains one or two substituents, examples of which may include lower alkyl, hydroxy, pyrrolidinyl and benzyloxy groups. The substituted imino group represented by Z contains lower alkyl groups. Illustrative of the cycloalkylene represented by Z are cyclopentylene, cyclohexylene, and cycloheptylene.

Further, p and q may preferably range from 0 to 4.

Preferred specific examples of the bis (2-aryl-5-pyridyl) derivative (1) according to the present invention can include
N,N'-bis[2-(3,4,5-trimethoxyphenyl)-5-pyridyl]-N,N'-dimethylethylenediamine dimethanesulfonate,
N,N'-bis[2-(4-hydroxy-3,5-dimethoxyphenyl)-5-pyridyl]-N,N'-dimethyl-ethylenediamine dimethanesulfonate,
1,4-bis[2-(3,4,5-trimethoxyphenyl)-5-pyridyl]hexahydro-1,4-diazepine dimethanesulfonate,
N,N'-bis[2-(3,4,5-trimethoxyphenyl)-5-pyridyl]-N,N'-diethylethylenediamine dimethanesulfonate,
N,N'-bis[2-(3,4,5-trimethoxyphenyl)-5-pyridyl]-N,N'-dimethy1-1,3-propanediamine dimethanesulfonate,
N,N'-bis[2-(3,4,5-trimethoxyphenyl)-5-pyridyl]-N,N',2,2-tetramethyl-1,3-propanediamine dimethanesulfonate,
2-hydroxy-N,N'-bis[2-(3,4,5-trimethoxyphenyl)-5-pyridyl]-N,N'-dimethyl-1,3-propanediamine,
N,N-bis[N-[2-(3,4,5-trimethoxyphenyl)-5-pyridyl]-N-methyl-2-aminoethyl]methylamine trihydro-chloride,
N,N-bis[N-[2-(3,4,5-trimethoxyphenyl)-5-pyridyl]-N-methyl-3-aminopropyl]methylamine,
bis[N-[2-(3,9,5-trimethoxyphenyl)-5-pyridyl]-2-aminoethyl]ether dimethane-sulfonate,
2-dimethylaminomethyl-1,4-bis[2-(3,4,5-trimethoxyphenyl)-5-pyridyl]piperazine trihydrochloride,
N,N'-bis[2-(3,4,5-trimethoxyphenyl)-5-pyridyl]-N,N'-dimethy1-1,6-hexanediamine dimethanesulfonate,
N,N-bis[N-[2-[3,5-dimethoxy-4-(2,2,2-trifluoroethoxy)phenyl]-5-pyridyl]-N-methyl-3-aminopropyl]methylamine trihydro-chloride.

The compound (1) according to the present invention can be produced, for example, in accordance with the reaction steps of the below-described production processes, although no particular limitation is imposed on its production process.

### (Production Process 1)

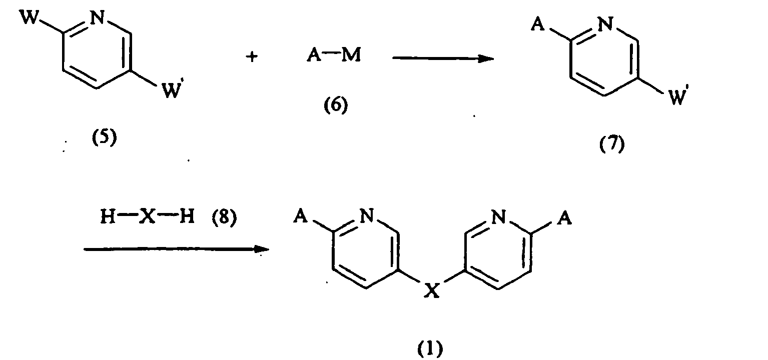

wherein A and X have the same meanings as defined above, W and W' represent a halogen atom or -OSO₂(CᵣF₂ᵣ₊₁) in which r stands for an integer of 0 to 4, and M represents dihydroxyboron, di (lower alkoxy)boron, di(loweralkyl)boron, dihalo(lower alkyl)silicon, halogenated zinc, tri(lower alkyl)tin, halogenated magnesium or the like.

Described specifically, the compound (1) according to the present invention can be produced by adding the compound (6) and a catalyst to a solution or suspension of the compound (5), reacting these compounds optionally in the presence of a ligand and a base to obtain the compound (7) and then reacting the compound (7) with the compound (8).

### (1) Synthesis of the compound (7) (cross-coupling reaction)

The compound (7) can be obtained by adding the compound (6), the catalyst and, if necessary, the ligand and the base to the solution or suspensionof the compound (5) and then reacting the compound (5) with the compound (6) at room temperature to 200°C for 0.5 to 100 hours [Metal-catalyzed Reactions; Diederich, F., Stang, P.J., Eds.; Wiley-VHC: Weinheim (1998). Stanforth, S.P. Tetrahedron, 54, 263-303 (1998)].

Illustrative of a solvent usable in the above reaction are benzene, toluene, xylene, diethyl ether, tetrahydrofuran, dimethoxyethane, dioxane, acetonitrile, dimethylformamide, N-methylpiperidone, methanol, ethanol, and water. Examples of the catalyst can include
tetrakis(triphenylphosphine)-palladium(0),
tris(bisbenzylideneacetone)dipalladium(0), palladium acetate(II), palladium chloride(II),
dichloro-bis(triphenylphosphine)palladium(II),
dichloro[1,2-bis(diphenylphosphine)ethane]palladium(II),
dichloro[1,4-bis(diphenylphosphino)butane]palladium(II),
dichloro[1,1-bis(diphenylphosphino)ferrocene]palladium(II),
tetrakis-triphenylphosphine nickel(0) and
bis(acetylacetonato)nickel (II).

Examples of the ligand, on the other hand, can include
tri(t-butyl)phosphine, triphenylphosphine,
tri(o-tolyl)-phosphine, tri(2-furyl)phosphine,
2,2'-bis(diphenyl-phosphino)-1,1'-binaphthyl,
1,2-bis(diphenylphosphino)-ethane,
1,3-bis(diphenylphosphino)propane,
1,4-bis(diphenylphosphino)butane, and
1,1'-bis(diphenyl-phosphino)ferrocene. Examples of the base can include sodium acetate, potassium acetate, sodium carbonate, sodium hydrogencarbonate, potassium phosphate, sodium hydroxide, potassium hydroxide, barium hydroxide, sodium methoxide, sodium ethoxide, cesium fluoride, fluorinated tributyl-ammonium, and triethylamine.

The reaction can be conducted preferably by following the report by Stille et al. [Stille, J.K., Angew. Chem. Int. Ed. Engl. 25, 508-524 (1986)], the report by Suzuki et al. [Miyaura, N.; Suzuki, A. Chem. Rev., 95, 2457-2483 (1995)] or the report by Mitchell et al. [Mitchell, M.B. ; Wallbank, P.J., Tetrahedron Lett., 32, 2273-2276 (1991)], that is, by using a compound (6) in which M is a tri(lower alkyl)tin and applying conditions of tetrakis(triphenylphosphine)palladium(0)/toluene/100 to 150°C /10 to 30 hours; by using a compound (6) in which M is a dihydroboron and applying conditions of tetrakis(triphenyl-phosphine)palladium(0)/sodium carbonate (or potassium carbonate)/water-ethanol(or methanol)-toluene/60 to 100°C/0.5 to 3 hours; or by using a compound (6) in which M is a dihydroxyboron an applying conditions of dichloro[1,2-bis(diphenylphosphino)ethane]palladium(II)[or dichloro[1,4-bis(diphenylphosphino)butane]palladium(II)]/sodium carbonate(or potassium carbonate)/water-ethanol(or methanol)-toluene/80 to 120°C/1 to 24 hours.

### (2) Synthesis of the compound (1) (condensation reaction)

The compound (1) can be obtained by adding the compound (8), a catalyst, a base and, if necessary, a ligand to a solution of the compound (7) and then reacting the compound (8) with the compound (7) at room temperature to 200°C for 0.5 to 100 hours, preferably at 80 to 120°C for 5 to 15 hours [Yang, B.H., Buchwald, S.L., J. Organomet. Chem., 576, 125-146 (1999) ; Hartwig. J.F., Angew. Chem. Int. Ed. Engl., 37, 2046-2067 (1998); Nishiyama, M., Yamamoto, T., Koike, Y., Tetrahedron Lett., 39, 617-620 (1998)].

Illustrative of solvents usable here are toluene, xylene, dimethoxyethane, tetrahydrofuran, and dioxane. Examples of the catalyst can include
tetrakis(triphenylphosphine)-palladium(0),
tris(bisbenzylideneacetone)dipalladium(0), palladium(II) acetate, and
dichloro[1,1'-bis(diphenyl-phosphino)ferrocene]palladium(II), with tetrakis(triphenyl-phosphine)palladium(0) and
tris(bisbenzylideneacetone)-dipalladium(0) being particularlypreferred. Examples of the base can include sodium t-butoxide, potassium t-butoxide, potassium phosphate, potassium carbonate, cesium carbonate, lithium tetramethyldisilazide, triethylamine, and 1,8-diazabicyclo[5,4,0]-undecene (DBU), with sodium t-butoxide, potassium t-butoxide and potassium carbonate being particularly preferred. Examples of the ligand can include
tri(t-butyl)phosphine, triphenylphosphine,
tri(o-tolyl)-phosphine, tri(2-furyl)phosphine,
2,2'-bis(diphenyl-phosphino)-1,1'-binaphthyl,
1,2-bis(diphenylphosphine)-ethane,
1,3-bis(diphenylphosphino)propane,
1,4-bis(diphenylphosphino)butane, and
1,1'-bis(diphenyl-phosphino)ferrocene.

### (Production Process 2)

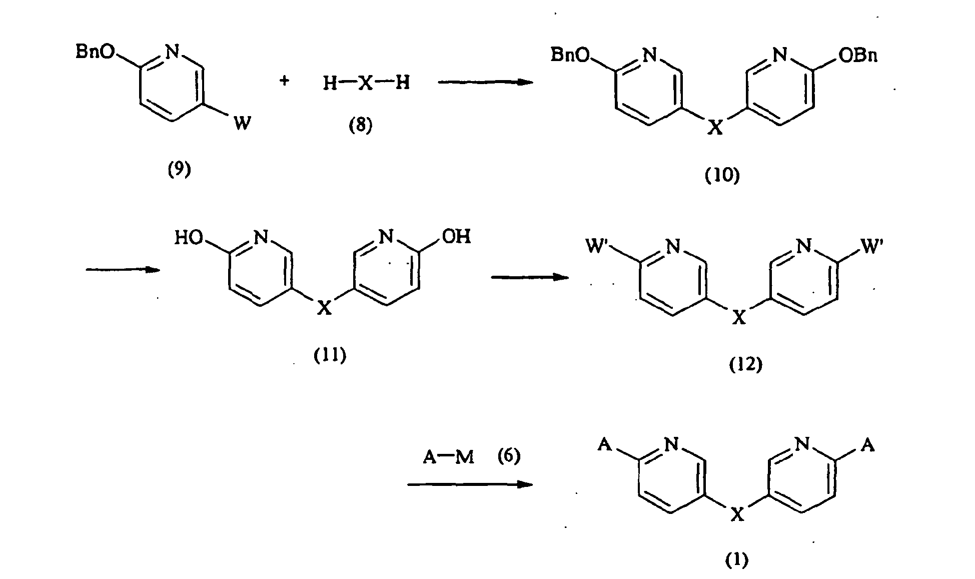

wherein A, X, W, W' and M have the same meanings as defined above.

Described specifically, the compound (1) can be produced by subjecting the compound (9) and the compound (8) to a condensation reaction to obtain the compound (10), debenzylating the compound (10) into the compound (11), converting the hydroxyl groups of the compound (11) to obtain the compound (12), and then reacting the compound (12) with the compound (6).

### (1) Synthesis of the compound (10) (condensation reaction)

The compound (10) can be obtained by reacting the 2-benzyloxy-5-halopyridine (9) with the compound (8) under similar conditions as in the reaction between the compound (7) and the compound (8) in the production process 1.

### (2) Synthesis of the compound (11) (debenzylating reaction)

The compound (11) can be obtained by adding an acid to a solution of the compound (10) and then reacting them at -20 to 100°C for 0.5 to 100 hours, preferably at 30 to 60°C for 1 to 6 hours.

Illustrative of a solvent usable here are chloroform, methylene chloride, dichloromethane, and ethyl acetate. Examples of the acid can include hydrogen chloride and trifluoroacetic acid. It is also possible to use the acid as a solvent.

### (3) Synthesis of the compound (12) (conversion of hydroxyl groups into eliminative groups)

The compound (12) can be obtained by adding a base and ClSO₂(CₛF₂ₛ₊₁) or [SO₂(CₛF₂ₛ₊₁)]₂ (s: an integer of 0 to 4) to a solution or suspension of the compound (11) and stirring the resulting mixture at -78 to 100°C for 5 minutes to 10 hours, preferably at -78 to 0°C for 10 minutes to 2 hours.

Illustrative of a solvent usable here are methylene chloride, chloroform, dichloroethane, benzene, toluene, xylene, diethyl ether, tetrahydrofuran, dimethoxyethane, dioxane, and acetonitrile. Examples of the base can include sodium acetate, potassium carbonate, sodium hydrogencarbonate, potassium phosphate, triethylamine, N,N-diisopropyl-ethylamine and pyridine, with triethylamine and N,N-diisopropylethylamine being preferred.

As an alternative process, the compound (12) can also be obtained by reacting the compound (11) with a halogenating agent at room temperature to 200°C for 0.5 minutes to 200 hours, preferably at 50 to 120°C for 5 to 12 hours. A solvent can also be used as needed.

Examples of the halogenating agent can include phosphorus pentachloride, phosphorus oxychloride, phosphorus tribromide, and phosphorus oxybromide, with phosphorus oxychloride-phosphorus pentachloride being particularly preferred. Examples of the solvent can include chloroform, methylene chloride and dichloroethane.

### (4) Synthesis of the compound (1) (cross-coupling reaction)

The compound (1) can be obtained by reacting the compound (12) with the compound (6) under similar conditions as in the cross-coupling between the compound (5) and the compound (6) in the production process 1.

### (Production Process 3)

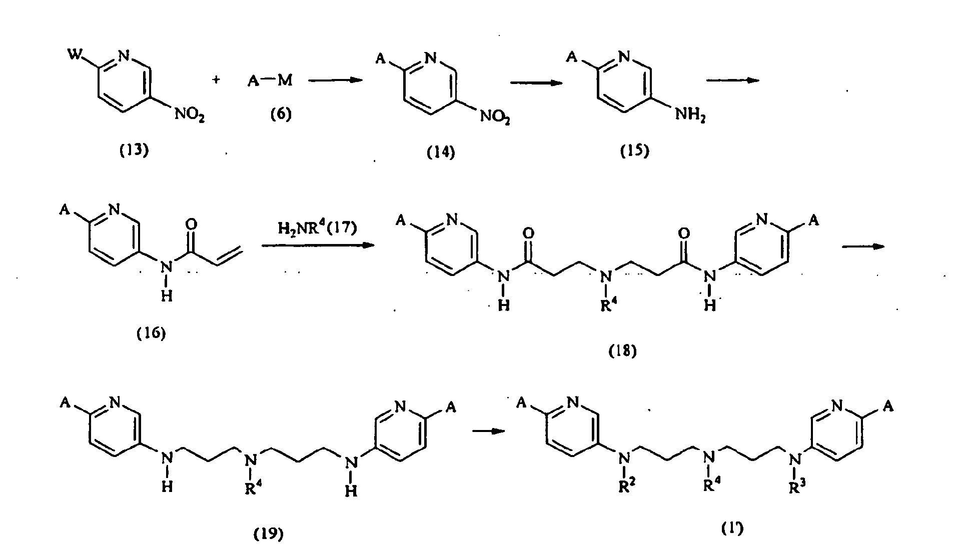

wherein A, W, M, R² and R³ have the same meanings as defined above, and R⁴ represents a lower alkyl group.

Among the compounds (1), the compounds (1') in each of which X is a group of the formula (4), p and q are 3, and Z is a substituted imino group can be produced in accordance with the reaction steps of the above-described production process 3.

### (1) Synthesis of the compound (14) (cross-coupling reaction)

The compound (14) can be obtained by reacting the 2-halo-5-nitropyridine (13) with the compound (6) under similar conditions as in the cross-coupling reactionbetween the compound (5) and the compound (6) in the production process 1.

### (2) Synthesis of the compound (15) (reduction of the nitro group)

The compound (15) can be obtained by reducing the compound (14) in a manner known *per se* in the art. As a specific reducing reaction, a catalyst and a hydrogen source are added to a solution of the compound (14), followed by a reaction at room temperature to 100°C for 5 minutes to 100 hours, preferably at 50 to 100°C for 0.5 to 3 hours (process A) ; or an acid and a metal or metal salt are added to a solution or suspension of the compound (14), followed by a reaction at room temperature to 100°C for 5 minutes to 100 hours, preferably at 50 to 100°C for 0. 5 to 3 hours (process B).

As specific conditions for the process A, illustrative of a solvent are tetrahydrofuran, dimethoxyethane, dioxane, ethyl acetate, methanol, ethanol; illustrative of the catalyst are palladium on charcoal, platinum black, platinum, and Raney nickel; and illustrative of the hydrogen source are hydrogen, ammonium formate, and hydrazine.

As specific conditions for the process B, illustrative of a solvent are ethanol and water; illustrative of the acid are hydrochloric acid and acetic acid; and illustrative of the metal or metal salt are zinc powder, tin powder, iron powder, and stannous chloride.

### (3) Synthesis of the compound (16) (acylating reaction)

The compound (16) can be obtained by adding a base and acryloyl chloride to a solution of the compound (15) and reacting them at -78 to 50°C for 5 minutes to 100 hours, preferably at -30 to 30°C for 10 minutes to 1 hour.

Illustrative of a solvent usable here are methylene chloride, chloroform, dichloroethane, benzene, toluene, xylene, diethyl ether, tetrahydrofuran, dimethoxyethane, dioxane, and acetonitrile, with methylene chloride and tetrahydrofuran being particularly preferred. Illustrative of the base are sodium acetate, potassium carbonate, sodium hydrogencarbonate, potassium phosphate, triethylamine, and N,N-diisopropylethylamine, with triethylamine and N,N-diisopropylethylamine being particularly preferred.

### (4) Synthesis of the compound (18) (Michael addition)

The compound (18) can be obtained by reacting the compound (16) with the amine (17) at 0 to 200°C for 0.5 to 100 hours, preferably at 20 to 100°C for 1 to 15 hours.

Here, a solvent may be used if necessary. Illustrative of the solvent are methylene chloride, chloroform, dichloroethane, benzene, toluene, xylene, diethyl ether, tetrahydrofuran, dimethoxyethane, dioxane, acetonitrile, and dimethylformamide.

### (5) Synthesis of the compound (19) (reducing reaction)

The compound (19) can be obtained by adding a reducing agent to a solution of the compound (18) and reacting them at 0 to 100°C for 5 minutes to 100 hours, preferably at 20 to 80°C for 30 minutes to 3 hours.

Illustrative of a solvent usable here are toluene, diethyl ether, tetrahydrofuran, and dimethoxyethane. Illustrative of the reducing agent are lithium aluminum hydride, diisobutylaluminum hydride, and borane.

### (6) Synthesis of the compound (1') (alkylating reaction)

The compound (1') can be obtained by adding a reducing agent and an acid to a solution of the compound (19) and a carbonyl compound and reacting them at -20 to 50°C for 0.5 to 100 hours, preferably at 0 to 30°C for 1 to 15 hours.

Illustrative of the carbonyl compound employed here are formaldehyde, paraformaldehyde, lower alkylaldehydes, and di(lower alkyl)ketone. Illustrative of a solvent are methylene chloride, chloroform, tetrahydrofuran, dimethoxyethane, dioxane, acetonitrile, methanol, and ethanol. Illustrative of the reducing agent are lithium aluminum hydride, sodium borohydride, sodium cyanoborohydride, and borane, Illustrative of the acid are hydrochloric acid, acetic acid, and trifluoroacetic acid.

### (Production Process 4)

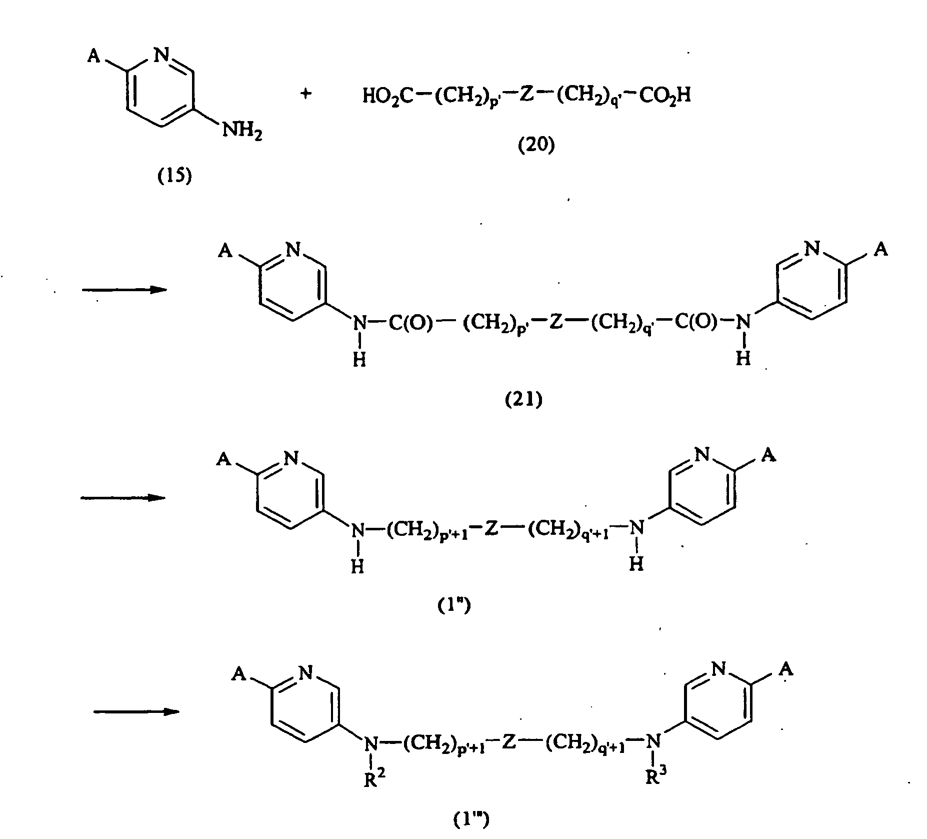

wherein A, Z, R² and R³ have the same meanings as defined above, and p' and q' denote an integer of 1 to 5.

Among the compound (1), the compounds (1") and (1"') in each of which X is a group of the formula (4) and p and q are 2 to 6 can be produced in accordance with the reaction steps of the production process 4.

### (1) Synthesis of the compound (21) (amidating reaction)

The compound (21) can be obtained by condensing the intermediate (15) in the production process 3 and the dicarboxylic acid (20) in a manner known *per se* in the art.

Described specifically, the intermediate 15 and the dicarboxylic acid (20) can be reacted in a solvent, in the presence of a condensing agent, in the presence of a base added as needed, at 0 to 100°C for 0.5 to 30 hours (process A) ; or the intermediate (15) and a reactive derivative of the dicarboxylic acid (20) are reacted in a solvent, in the presence of a base added as needed, at 0 to 100°C for 0.5 to 30 hours (process B). In each of the process A and the process B, it is preferred to conduct the reaction at 0 to 50°C for 0.5 to 5 hours.

Illustrative of the solvents employed in these reactions are dimethylformamide, tetrahydrofuran, dioxane, acetonitrile, methylene chloride, and dichloroethane. Illustrative of the bases are organic bases such as pyridine, triethylamine and N,N-diisopropylethylamine; and inorganic bases such as sodium carbonate and sodium hydrogencarbonate.

Further, examples of the condensing agent used in the process A can include 1,3-dicyclohexylcarbodiimide, 1-cyclohexyl-3-morpholinoethylcarbodiimide, 1-(3-dimethyl-aminopropyl)-3-ethylcarbodiimide, 1,1'-carbonyldiimidazole, diethyl phosphorocyanidate, diphenyl phophorylazide, bis(2-oxo-3-oxazolidine)phosphinyl chloride, and 2-chloro-1-methylpyridium iodide. Examples of the reactive derivative of the dicarboxylic acid (20) in the process B can include acid halides such as acid chlorides, acid azides, mixed acid anhydrides with pivalic acid or the like, and active esters such as cyanomethyl esters and p-nitrophenyl esters.

### (2) Synthesis of the compound (1") (reducing reaction)

The compound (1") can be obtained by reacting the compound (21) under similar conditions as in the reducing reaction of the compound (18) in the process 3.

### (3) Synthesis of the compound (1"') (alkylation)

The compound (1"') can be obtained by reacting the compound (1") under similar conditions as in the alkylation of the compound (19) in the process 3.

The intermediate and target compound obtained in the above reactions, respectively, can be isolated and purified by subjecting them to purification procedures commonly employed in organic synthesis chemistry, for example, filtration, extraction, washing, drying, concentration, recrystallization, one or more of various chromatographic treatments, and the like. Further, the intermediate can be provided for use in the next reaction without specifically purifying it.

In addition, they may also be obtained in the form of solvates with reaction solvents, recrystallization solvents or the like, especially as hydrates. Further, the compound (1) according to the present invention may include various isomers depending on the kinds and combination of the substituents. It is to be noted that the present invention encompasses all of such isomers.

The compound (1) obtained as described above can be converted into an acid addition salt or a basic salt by a method known *per se* in the art. No particular limitation is imposed on such salts insofar as they are pharmacologically acceptable salts. When the compound (1) is a basic compound, examples of its pharmacologically acceptable salt can include mineral acid salts such as the hydrochloride, sul f ate and nitrate; and organic acid salts such as the methanesulfonate, acetate, oxalate and citrate. When the compound (1) is an acidic compound, on the other hand, examples of its pharmacologically acceptable salt can include alkali metal salts such as the sodium and potassium salts; alkaline earth metal salts such as the calcium and magnesium salts; and organic base salts such as the pyridine, picoline and triethylamine salts.

As the bis (2-aryl-5-pyridyl) derivative (1) according to the present invention has excellent IgE antibody production inhibiting activity and also, IL-4 production inhibiting activity and IL-5 production inhibiting activity as will be demonstrated in tests to be described subsequently herein, it is useful as medicines for the prevention or treatment of various allergic diseases - for example, asthma, atopic dermatitis, allergic rhinilis, inflammatory colitis, contact skin diseases and allergic eye diseases - and also as an IgE antibody production inhibitor.

The medicine according to the present invention comprises as an active ingredient the bis(2-aryl-5-pyridyl) derivative or the salt thereof. By adding pharmacologically acceptable, inorganic or organic carriers, the bis(2-aryl-5-pyridyl) derivative or the salt thereof can be formulated into medicinal compositions, for example, various oral preparations or parenteral preparations such as solid, semi-solid or liquid preparations by methods known *per se* in the art.

Illustrative of preparations for oral administration are tablets, pills, granules, soft or hard capsules, triturates, subtilized granules, powders, emulsions, syrups, pellets, and elixirs. On the other hand, illustrative of preparations for parenteral administration are injections, drips, infusions, ointments, lotions, tonics, sprays, suspensions, medicinal oils, emulsions, suppositories, and instillations.

To formulate such preparations, methods known *per se* in the art can be followed. Active ingredients according to the present invention can be used in combination with pharma-cologically acceptable surfactants, excipients, coloring matters, flavoring agents, preservatives, stabilizers, buffering agents, suspending agents, isotonicities and the like as needed.

The dosage of the medicine according to the present invention varies inter alia depending on the medicine, the target disease to be treated or prevented, the administration method, the treatment period, and the age, sex and weight of the patient. Nonetheless, it is preferred to administer the medicine at a daily dosage of from 0.01 to 1,000 mg/kg.weight in terms of the compound represented by the formula (1). This dosage can be administered at once or in several portions, for example, 2 to 6 portions in a day.

### Examples

The present invention will next be describe further based on Examples. It should, however, be borne in mind that the present invention is not limited to or by the following Examples.

### Referential Example 1

### N,N'-bis(2-trifluoromethanesulfonyloxy-5-pyridyl)-N,N'-dimethylethylenediamine

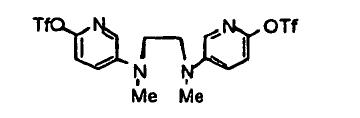

Under an argon gas atmosphere, tri-t-butylphosphine (5.0 mL, 20 mmol), sodium t-butoxide (10.78 g, 112 mmol), 2-benzyloxy-5-bromopyridien [J. Am. Chem. Soc., 71, 70-73 (1949)] (29.20 g, 110 mmol) and a solution of N,N'-dimethyl-ethylenediamine (4.40 g, 50.0 mmol) in o-xylene (30 mL were added to a solution of bis(dibenzylideneacetone)palladium (2.86 g, 5.00 mmol) in o-xylene (80 mL). After stirred under heating at 120°C for 3 hours, water was added, and the resulting mixture was extracted with chloroform. The organic layer was washed with water, dried over anhydrous sodium sulfate, and then concentrated under reduced pressure. The residue was purified by chromatography on a silica gel column, whereby N,N'-bis(2-benzyloxy-5-pyridyl)-N,N'-dimethylethylenediamine was obtained as pale yellow crystalline powder (meltingpoint: 95.0-97.0°C) (18.59g, yield: 81%).

A solution of N,N'-bis(2-benzyloxy-5-pyridyl)-N,N'-dimethylethylenediamine (18.50 g, 400 mmol) in trifluoroacetic acid (105 mL) was stirred at 50°C for 4 hours. Under ice cooling, diethyl ether (630 mL) was added to the reaction mixture, followed by stirring for 1 hour. Precipitated crystals were collected by filtration and then washed with diethyl ether, whereby N,N'-bis (2-hydroxy-5-pyridyl)-N,N'-dimethyl-ethylenediamine ditrifluoroacetate was obtained as yellow crystalline powder (melting point: 180.0-182.0°C) (17.24 g, yield 86%).

N,N'-Bis(2-hydroxy-5-pyridyl)-N,N'-dimethylethylene-diamine ditrifluoroacetate (18.00 g, 35.8 mmol) was suspended in methylene chloride (350 mL), followed by the addition of N,N-diisopropylethylamine (48 mL, 280 mmol). The resulting mixture was cooled to -10°C, to which trifluoromethanesulfonic anhydride (21 mL, 120 mmol) was added. After stirred for 20 minutes, N,N-diisopropylethylamine (12 mL, 70 mmol) and trifluoromethanesulfonic anhydride (21 mL, 120 mmol) were added. After stirred further at -10°C for 30 minutes, N,N-diisopropylethylamine (5.0 mL, 29 mmol) and trifluoro-methanesulfonic anhydride (5.0 mL, 28mmol) were added further, and the thus-obtained mixture was stirred for 1 hour in an ice bath. A saturated aqueous solution of sodium carbonate (180 mL) and sodium carbonate (18 g, 0.17 mmol) were added, and the resulting mixture was extracted with methylene chloride. The organic layer was washed with a saturated aqueous solution of sodium chloride (hereinafter called "brine"), dried over anhydrous sodium sulfate, and then concentrated under reduced pressure. The residue was purified by chromatography on a silica gel column to obtain crude crystals. The crude crystals were recrystallized from chloroform-hexane, whereby the title compound was obtained as pale brown needles (melting point: 146.0-148.0°C) (13.44 g, yield: 70%).

### Referential Example 2

### 5-Chloro-2-(3,4,5-trimethoxyphenyl)pyridine

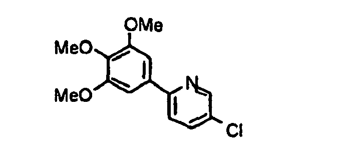

Under an argon gas atmosphere, 3,4,5-trimethoxy-phenylboric acid (5.05 g, 24.0 mmol), dichloro(diphenylphosphino)ethane]palladium (574.0 mg, 1.00 mmol) and a 2.0 M aqueous solution of sodium carbonate (20 mL, 40 mmol) were added to a solution of 2.5-dichloropyridine (2.95 g, 20.0 mmol) in ethanol-toluene (1:5, 54.0mL). After stirred at 100°C for 3 hours, water was added, and the resulting mixture was extracted with ethyl acetate. The organic layer was dried over anhydrous sodium sulfate and then concentrated under reduced pressure. The residue was purified by chromatography on a silica gel column, whereby the title compound was obtained as colorless crystalline powder (melting point: 61.0-63.0°C) (4.13 g, yield: 73%).

### Referential Example 3

### 4-Ethoxy-3,5-dimethoxyphenylboric acid

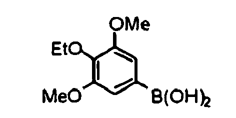

To a solution of 4-iodo-2,6-dimethoxyphenol (JP61-207432A) (100.0 mg, 0.360 mmol), ethyl iodide (84.0 mg, 0.540 mmol)and a 50% dispersion of sodium hydride in mineral oil (19.0 mg, 0.40 mmol) were added. After stirred at room temperature for 1 hour, the reaction mixture was poured into 0.10M hydrochloric acid, and the resulting mixture was extracted with diethyl ether. The organic layer was washed with a saturated aqueous solution of sodium hydrogencarbonate and brine, dried with anhydrous sodium sulfate, and then concentrated under reduced pressure. The residue was purified by chromatography on a silica gel column, whereby 4-ethoxy-1-iodo-3,5-dimethoxybenzene was obtained as colorless amorphous powder (102.0 mg, yield: 93%).

Under an argon atmosphere, a 1.60 M hexane solution of n-butyllithium (2.5 mL, 4.1 mmol) was added to anhydrous tetrahydrofuran (7. 0 mL) heldunder stirring in a dry ice-acetone bath, followed by gradual dropwise addition of a solution of 4-ethoxy-1-iodo-3,5-dimethoxybenzene (570.0 mg, 1.85 mmol) in anhydrous tetrahydrofuran (1.5 mL). After the thus-obtained mixture was stirred for 20 minutes in the dry ice-acetone bath, triisopropyl borate (0.50 mL, 2.2 mmol) was added, followed by stirring for further 20 minutes. The reaction mixture was stirred at room temperature for 1 hour, and then concentrated under reduced pressure. To the residue, a 0.1 M aqueous solution of sodium hydroxide (4.4 mL) was added. The thus-obtained aqueous solution was washed with chloroform. Concentrated hydrochloric acid was added to the solution to acidify the same, followed by extraction with chloroform. The organic layer was washed with brine, dried over anhydrous sodium sulfate, and then concentrated under reduced pressure. Chloroform-hexane was added to the residue and the resulting precipitate was collected by filtration, whereby the title compound was obtained as colorless amorphous powder (160.0 mg, yield: 39%).

### Referential Example 4

### 3,5-Dimethoxy-4-(2,2,2-trifluoroethoxy)phenylboric acid

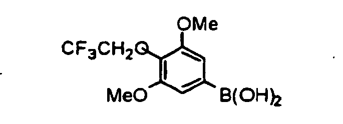

To a solution of methyl syringate (2.10 g, 9.90 mmol) in dimethylformamide (21.0 mL), potassium carbonate (13.60 g, 9.90 mmol) and 2,2,2-trifluoroethyl trifluoromethanesulfonate (purity: 25%) [J. Org. Chem., 30, 4322-4324 (1965)](10.90 g, 12 mmol) were added. After the resulting mixture was stirred at room temperature for 1 hour and then at 50°C for 3 hours, water was added. The resulting mixture was extracted with diethyl ether. The organic layer was washed with brine, dried over anhydrous sodium sulfate and then concentrated under reduced pressure, whereby an oil (2.69 g) containing methyl 3,5-dimethoxy-4-(2,2,2-trifluoroethoxy)benzoate was obtained.

To a solution of the oil (2.69 g, 9.1 mmol), which had been obtained by the above-described procedures, in methanol (14 mL), a 5.0 M aqueous solution of sodium hydroxide (14 mL, 70 mmol) was added, and the resulting mixture was stirred at 80°C for 30 minutes. The reaction mixture was concentrated under reduced pressure to remove methanol. Concentrated hydrochloric acidwas added to the residue to acidify the same, and the resulting mixture was extracted with chloroform. The organic layer was washed with brine, dried over anhydrous sodium sulfate and then concentrated under reduced pressure, whereby an oil (2.56 g) containing 3,5-dimethoxy-4-(2,2,2-trifluoroethoxy)benzoic acid was obtained.

To a solution of the oil (2.45 g, 7.8 mmol), which had been obtained by the above-described procedures, in t-butyl alcohol (37 mL), triethylamine (0.93 g, 9.2 mmol) and diphenylphosphoryl azide (2.53 g, 9.20 mmol) were added. After stirred at 100°C for 2 hours, the reaction mixture was concentrated under reduced pressure. A solution of the residue in ethyl acetate was washed with a saturated aqueous solution of sodium hydrogencarbonate and brine, dried over anhydrous sodium sulfate and then concentrated under reduced pressure. The residue was purified by chromatography on a silica gel column, whereby N-t-butoxycarbonyl-3,5-dimethoxy-4-(2,2,2-trifluoroethoxy)-aniline was obtained as a colorless oil (1.10 g, yield: 36% based on the methyl syringate).

N-t-butoxycarbonyl-3,5-dimethoxy-4-(2,2,2-trifluoro-e thoxy) aniline (1.10 g, 3.13 mmol) was added to a 10% methanol solution of hydrogen chloride (20 mL, 55 mmol), and the resulting solution was stirred at 50°C for 30minutes. The reaction mixture was concentrated under reduced pressure, whereby crude 3,5-dimethoxy-4-(2,2,2-trifluoroethoxy)aniline hydrochloride was obtained.

The crude 3,5-dimethoxy-4-(2,2,2-trifluoroethoxy)-aniline hydrochloride obtained by the above-described procedures was suspended in water (16 mL), and to the resulting suspension, concentrated hydrochloric acid (0.26mL, 3.1mmol) was added under ice cooling. Under ice cooling, a solution of sodium nitrite (227.0 mg, 3.29 mmol) in water (2.0 mL) was added dropwise over about 5 minutes, and after the resulting mixture was stirred for 15 minutes, a solution of potassium iodide (562.0 mg, 3.44 mmol) in water (2.0 mL) was added. The reaction mixture was stirred at room temperature for 1 hour and then at 50°C for 15 minutes, followed by extraction with ethyl acetate. The organic layer was washed with brine, dried over anhydrous sodium sulfate, and then concentrated under reduced pressure. The residue was purified by chromatography on a silica gel column, whereby 1-iodo-3,5-dimethoxy-4-(2,2,2-trifluoro-ethoxy)benzene was obtained as a colorless oil [1.00 g, yield: 88% based on N-t-butoxycarbonyl-3,5-dimethoxy-4-(2,2,2-trifluoroethoxy)aniline].

Applying the synthesis procedures of 4-ethoxy-3,5-dimethoxyphenylboric acid from 4-ethoxy-1-iodo-3,5-dimethoxybenzene in Referential Example 3, the title compound was obtained as colorless amorphous powder (369.0 mg, yield: 48%) from 1-iodo-3,5-dimethoxy-4-(2,2,2-trifluoroethoxy)-benzene (1.00 g, 2.76 mmol)

### Referential Example 5

### 4- (2-Methoxyethoxy)-3,5-dimethoxyphenylboric acid

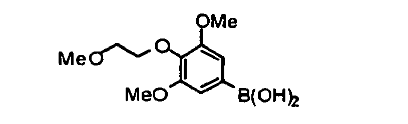

To a solution of 4-iodo-2,6-dimethoxyphenol (JP61-207432A) (280.0 mg, 1.00mmol) indimethylformamide (3.0 mL), potassium carbonate (184.0 mg, 1.10 mmol) and ethyl bromoacetate (184.0 mg, 1.10 mmol) were added. After the resulting mixture was stirred at room temperature for 1 hour and then at 50°C for 1 hour, water was added to the reaction mixture. The resulting mixture was extracted with diethyl ether. The organic layer was washed with brine, dried over anhydrous sodium sulfate and then concentrated under reduced pressure, whereby an oil (351.0 mg) containing 4-ethoxy-carbonylmethyloxy-1-iodo-3,5-dimethoxybenzene was obtained.

Under ice cooling, lithium aluminum hydride (11.0 mg, 0.29 mmol) was added to a solution of the oil (155.0 mg, approx. 0.42 mmol), which had been obtained by the above-described procedures, in anhydrous diethyl ether (2.0 mL). After the resulting mixture was stirred at room temperature for 1 hour, 3.0 M hydrochloric acid was added to the reaction mixture, and the water layer was extracted with diethyl ether. The organic layer was washed with a saturated aqueous solution of sodium hydrogencarbonate and brine, dried over anhydrous sodium sulfate, and then concentrated under reduced pressure. The residue was purified by chromatography on a silica gel column, whereby 4-(2-hydroxyethoxy)-1-iodo-3,5-dimethoxybenzene was obtained as a colorless oil (83.0 mg, yield: 59% based on 4-iodo-2,6-dimethoxyphenol).

Under ice cooling, methyl iodide (0.010 mL, 0.16 mmol) and a 50% dispersion of sodium hydride in mineral oil (12.0 mg, 0.24 mmol) were added to a solution of 4-(2-hydroxyethoxy)-1-iodo-3,5-dimethoxybenzene (39.0 mg, 0.120 mmol) in anhydrous tetrahydrofuran (1.0 mL), and the resulting mixture was stirred at room temperature for 1 hour. Water was added to the reaction mixture, and the thus-obtained mixture was extracted with diethyl ether. The organic layer was washed with 3.0 M hydrochloric acid, a saturated aqueous solution of sodium hydrogencarbonate and brine, dried over anhydrous sodium sulfate, and then concentrated under reduced pressure. The residue was purified by chromatography on a silica gel column, whereby 1-iodo-3,5-dimethoxy-4-(2-methoxyethoxy)benzene was obtained as a colorless oil (40.0 mg, yield: 98%).

Applying the synthesis procedures of 4-ethoxy-3,5-dimethoxyphenylboric acid from 4-ethoxy-1-iodo-3,5-dimethoxybenzene in Referential Example 3, the title compound was obtained as colorless amorphous powder (276.0 mg, yield: 54%) from 1-iodo-3,5-dimethoxy-4-(2-methoxyethoxy)benzene (673.0 mg, 2.00 mmol).

### Referential Example 6

### 4-[2-(t-butyldimethylsiloxy)ethoxy]-3,5-dimethoxyphenylboric acid

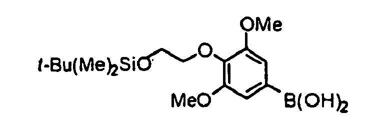

To a solution of 4-(2-hydroxyethoxy)-1-iodo-3,5-dimethoxybenzene (2.76g, 8.52 mmol), which had been synthesized by the process shown in Referential Example 5, in dimethylformamide (25 mL), imidazole (1.16 g, 17.0 mmol) and t-butylchlorodimethylsilane (1.61 g, 10.7 mmol) were added, and the resulting mixture was stirred at 50°C for 3 hours. Water was added to the reaction mixture, followed by extraction with ethyl acetate-hexane (1:1). The organic layer was washed with water and brine, dried over anhydrous sodium sulfate, and then concentrated under reduced pressure. The residue was purified by chromatography on silica gel column, whereby 4-[2-(t-butyldimethylsiloxy)ethoxy]-1-iodo-3,5-dimethoxyben zene was obtained as a colorless oil (3.56 g, yield: 95%).

Applying the synthesis procedures of 4-ethoxy-3,5-dimethoxyphenylboric acid from 4-ethoxy-1-iodo-3,5-dimethoxybenzene in Referential Example 3, the title compound was obtained as colorless amorphous powder (1.15 mg; quantitative) from 4-[2-(t-butyldimethylsiloxy)ethoxy]-1-iodo-3,5-dimethoxybenzene (1.42 g, 3.24 mmol).

### Referential Example 7

### 3, 5-Dimethoxy-4-(N-methoxy-N-methylcarbamoyl)phenylboric acid

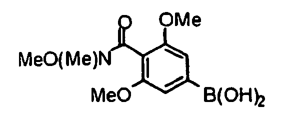

To a solution of 4-iodo-2, 6-dimethoxybezoic acid (308. 0 mg, 1.00 mmol) in methylene chloride (5.0 mL), dimethyl-formamide (0.020 mL, 0.26 mmol) and oxalyl chloride (0.13 mL, 1.5 mmol) were added, and the resulting mixture was stirred for 30 minutes. Under ice cooling, the reaction mixture was poured into a solution of N, O-dimethylhydroxyamine hydrochloride (488.0 mg, 5.00 mmol) and N, N-diisopropylethylamine (1.74 mL, 10.0 mmol) in methylene chloride (5.0 mL). An ice bath was removed, and the mixture was stirred at room temperature for 30 minutes. 0.1 M Hydrochloric acid was added to the reaction mixture, and the resulting mixture was extracted with chloroform. The organic layer was washed with a 0.1 M aqueous solution of sodium hydroxide and brine, dried over anhydrous sodium sulfate, and then concentrated under reduced pressure. Hexane was added to the residue and the resulting precipitate was collectedby filtration, whereby crude 4-iodo-N,2,6-trimethoxy-N-methylbenzamide was obtained as pale yellow crystalline powder (305.0 mg, yield: approx. 87%).

Applying the synthesis procedures of 4-ethoxy-3,5-dimethoxyphenylboric acid from 4-ethoxy-1-iodo-3,5-dimethoxybenzene in Referential Example 3, the title compound was obtained as pale yellow amorphous powder (91.0 mg, yield: 36% based on 4-iodo-2,6-dimethoxybenzoic acid) from the crude 4-1-iodo-N,2,6-trimethoxy-N-methylbenzamide (287.0 mg, approx. 0.82 mmol).

### Referential Example 8

### (4-Acetyl-3,5-dimethoxyphenyl)trimethyltin

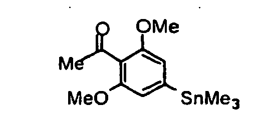

Aluminumchloride (87.05g, 653.0mmol) wasgraduallyadded to a solution of 3, 5-dimethoxyaniline (20.0 g, 131.0 mmol) and acetic anhydride (30.9 mL, 328 mmol) in methylene chloride (650 mL), said solution having been held under stirring in a sodium chloride-ice bath. The resulting mixture was stirred in the sodium chloride-ice bath for 20 minutes and then at room temperature for 1 hour. The reaction mixture was poured into ice water, and the resulting mixture was extracted with chloroform. The organic layer was washed with a saturated aqueous solution of sodium hydrogencarbonate and brine, dried over anhydrous sodium sulfate, and then concentrated under reduced pressure. The residue was purified by chromatography on a silica gel column to obtain crude crystals. The crude crystals were recrystallized from diethyl ether-hexane, whereby 4-acetamido-2,6-dimethoxyacetophenone was obtained as pale yellow crystalline powder (melting point 184.0-187.0°C) (9.42 g, yield: 26%).

To a suspension of 4-acetamido-2,6-dimethoxyaceto-phenone (9.42 g, 39.7 mmol) in ethanol (400 mL), a 5.0 M aqueous solution of sodium hydroxide (80 mL, 400 mL) was added, and the resulting mixture was stirred at 95°C for 3 hours. The reaction mixture was concentrated under reduced pressure, water was added to the residue, and the resulting mixture was extracted with chloroform. The organic layer was washed with brine, dried over anhydrous sodium sulfate, and then concentrated under reduced pressure. The residue was suspended in diethyl ether-hexane (1:4) and collected by filtration, whereby 4-amino-2,6-dimethoxyacetophenone was obtained as pale yellow crystalline powder (melting point: 155.0-158.0°C) (7.58 g, yield: 98%).

To a suspension of 4-amino-2,6-dimethoxyacetophenone (7.58 g, 38.8 mmol) in water (155 mL), said suspension having been held under stirring in the sodium chloride-ice bath, concentrated hydrochloric acid (6.5 mL, 78 mmol) was added to provide a homogeneous solution. To the solution held under stirring in the sodium chloride-ice bath, a solution of sodium nitrite (2.81g, 40.7 mmol) inwater (8.0 mL) was gradually added, and the resulting mixture was stirred in an ice bath for 15 minutes. A solution of potassium iodide (7.08 g, 42.7 mmol) in water (8.0 mL) and ethyl acetate (48 mL) were added, and the thus-obtained mixture was stirred at room temperature for 1 hour and then at 50°C for 15 minutes. After the mixture was allowed to cool down, the water layer was extracted with ethyl acetate. The organic layers were combined, washed with a saturated aqueous solution of sodium hydrogencarbonate, a 10% aqueous solution of sodium thiosulfate and brine, dried over anhydrous sodium sulfate, and then concentrated under reduced pressure. The residue was purified by chromatography on a silica gel column, whereby 4-iodo-2,6-dimethoxyacetophenone was obtained as colorless crystallinepowder (melting point: 124.0-129.0°C) (9.50g, yield: 80%).

Under a nitrogen gas atmosphere, hexamethylditin (609.0 mg, 1.86 mmol) and tetrakis(triphenylphosphine)palladium (215.0 mg, 0.190 mmol) were added to 4-iodo-2,6-dimethoxyacetophenone (287.0 mg, 1.86 mmol) in a solution of anhydride 1,4-dioxane, and the resulting mixture was stirred at 110°C for 4 hours. After the mixture was allowed to cool down, water was added. The resultant mixture was extracted with benzene. The organic layer was dried over anhydrous sodium sulfate and then concentrated under reduced pressure. The residue was purifiedby chromatography on a silica gel column, whereby the title compound was obtained as a colorless oil (450.0 mg, yield: 71%).

### Referential Example 9

### N,N'-Bis(2-chloro-5-pyridyl)-N,N'-dimethylethylenediamine

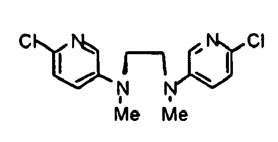

To phosphorus oxychloride (16.4 mL), N,N'-bis(2-hydroxy-5-pyridyl)-N,N'-dimethylethylenediamine ditrifluoroacetate (824.0 mg, 1.64 mmol) synthesized by the method described in Referential Example 1 and phosphorus pentachloride (171.0 mg, 0.820mmol) were added. The resulting mixture was stirred at 100°C for 8 hours. After allowed to cool down, the reaction mixture was poured into ice water. Sodium hydroxide was added to the resulting mixture to basify the same, followed by extraction with chloroform. The organic layer was dried over anhydrous sodium sulfate and then concentrated under reduced pressure, whereby crude crystals of the title compound was obtained as pale brown crystalline powder (melting point: 173.0-179.0°C) (439.0 mg, yield: approx. 86%).

### Referential Example 10

### 2-Benzyloxy-N,N'-dimethyl-1,3-propanediamine

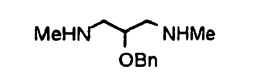

To a solution of 2-hydroxy-1,3-propanediamine (0.90 g, 10 mmol) in tetrahydrofuran (40 mL), triethylamine (3. 94 g, 39 mmol) was added. To the resulting solution, Under ice cooling, a solution of ethyl chloroformate (3.58 g, 33 mmol) in tetrahydrofuran (10 mL) was added dropwise under ice cooling. The resulting mixture was stirred at room temperature for 15 hours, and was then filtered to remove insoluble matter. The filtrate was dried over anhydrous sodium sulfate and then concentrated under reduced pressure, whereby an oil (1.95 g) containing N,N'-bis(ethoxycarbonyl)-2-ethoxycarbonyloxy-1,3-propanediamine was obtained.

Under ice cooling, lithium aluminum hydride (780.0 mg, 20.7 mmol) was added to a solution of the oil (1.95 g, approx. 6.5 mmol), which was obtained by the above-described procedures, in tetrahydrofuran (40 mL). An ice bath was removed, and the mixture was stirred at 80°C for 17 hours. The reaction mixture was allowed to cool down, followed by the addition of anhydrous sodium sulfate (25.0 g). Insoluble matter was filtered off using Celite, and the filtrate was concentrated under reduced pressure. The residue was purified by chromatography on a silica gel column, whereby 2-hydroxy-N,N'-dimethyl-1,3-propanediamine was obtained as a pale yellow oil (277.0 mg, yield: 23% based on 2-hydroxy-1,3-propanediamine).

To a solution of 2-hydroxy-N,N'-dimethyl-1,3-propane-diamine (165.0 mg, 1.39 mmol) in acetonitrile (5.0 mL), triethylamine (564.0 mg, 5.58 mmol) and di-t-butyl dicarbonate (912.0 mg, 4.18 mmol) were added. After stirred at room temperature for 15 hours, 0.10 M hydrochloric acid was added. The thus-obtained mixture was extracted with chloroform. The organic layer was washed with brine, dried over anhydrous sodium sulfate, and then concentrated under reduced pressure. The residue was purified by chromatography on a silica gel column, whereby N,N'-bis(t-butoxycarbonyl)-2-hydroxy-N,N'-dimehtyl-1,3-propanediamine was obtained as a pale yellow oil (163.0 mg, yield: 36%).

Under ice cooling, benzyl bromide (230.0 mg, 1.35 mmol) and a 50% dispersion of sodium hydride in mineral oil (35.0 mg, 0.81 mmol) were added to a solution of N,N'-bis(t-butoxy-carbonyl)-2-hydroxy-N,N'-dimehtyl-1,3-propanediamine (87.0 mg, 0.27 mmol) in tetrahydrofuran (4.0 mL). The reaction mixture was stirred at room temperature for 15 hours and then concentrated under reduced pressure. Water was added to the residue, and the resulting mixture was extracted with ethyl acetate. The organic layer was washed with brine, dried over anhydrous sodium sulfate, and the concentrated under reduced pressure. The residue was purified by chromatography on a silica gel column, whereby 2-benzyloxy-N,N'-bis(t-butoxy-carbonyl)-N,N'-dimethyl-1,3-propanediamine was obtained as a colorless oil (54.0 mg, yield: 49%).

To a solution of 2-benzyloxy-N,N'-bis(t-butoxy-carbonyl)-N,N'-dimethyl-1,3-propanediamine (73.0 mg, 0.17 mmol) in methylene chloride (2.0 mL), a 4.0 M ethyl acetate solution of hydrogen chloride (1.0 mL, 4.0 mmol) was added, and the resulting mixture was stirred at room temperature for 4 hours. The reaction mixture was concentrated under reduced pressure, ethanol (5.0 mL) was added to the residue, and the resulting mixture was concentrated under reduced pressure. An approx. 10% methanol solution of ammonia (2.0 mL, approx. 12 mmol) was added to the residue, insoluble matter was removed by filtration, and the filtrate was concentrated under reduced pressure. The residue was purified by chromatography on a silica gel column, whereby the title compound was obtained as a colorless oil (35.0 mg, yield: 98%).

### Referential Example 11

### 5-Amino-2-(3,4,5-trimethoxyphenyl)pyridine

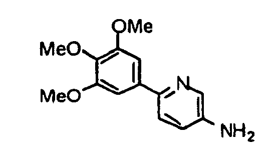

Under a nitrogen gas atmosphere, tetrakis(triphenyl-phosphine)palladium (504.6mg, 0.437 mmol) and a 2.0 M aqueous solution of sodium carbonate (25 mL, 50 mmol) were added to a solution of 2-bromo-5-nitropyridine (2.87 g, 14.1 mmol) and 3,4,5-trimethoxyphenylboric acid (3.00 g, 14.2 mmol) in ethanol-toluene (1:5, 90 mL), and the resulting mixture was stirred at 70°C for 12 hours. Water was added to the reaction mixture, and the water layer was extracted with ethyl acetate. The organic layers were combined, dried over anhydrous sodium sulfate, and then concentrated under reduced pressure. The residue was purified by chromatography on a silica gel column to obtain crude crystals. The crude crystals were recrystallized from chloroform-hexane, whereby 5-nitro-2-(3,4,5-trimethoxyphenyl)pyridine was obtained as yellow needles (melting point: 134. 0-136. 5°C) (3.31 g, yield: 81%).

To a solution of 5-nitro-2-(3,4,5-trimethoxyphenyl)-pyridine (1.50 g, 5.17 mmol) in methanol-ethyl acetate (1:4, 50 mL), 10% palladium on charcoal (158.6 mg) was added, and under a hydrogen gas atmosphere, the resulting mixture was stirred at 45°C for 2 hours and 30 minutes. Insoluble matter was filtered off using Celite, and the filtrate was concentrated under reduced pressure. The residue was recrystallized from ethyl acetate-hexane, whereby the title compound was obtained as colorless needles (melting point: 153.5-155.5°C) (1.25 g, yield: 93%).

### Referential Example 12

### N,N'-Dimethyl-3-(1-pyrrolidinyl)-1,5-pentanediamine

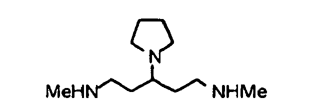

Following the process described in the publication [J. Am. Chem. Soc., 110, 6127-6179 (1988)], N,N'-dibenzyl-N,N'-dimethyl-3-(1-pyrrolidinyl)glutaramide was obtained as a colorless oil (2.81 g, yield: 90% based on 6-chloro-2-pyrone) from 6-chloro-2-pyrone (1.00 g, 7.66 mmol) and N-methyl-benzylamine (3.71 g, 30.6 mmol) via crude N,N'-dibenzyl-N,N'-dimethylglutaconamide (2.67 g).

Under a nitrogen gas atmosphere, lithium aluminum hydride (0.44 g, 12 mmol) was added to a solution of N,N'-dibenzyl-N,N'-dimethyl-3-(1-pyrrolidinyl)glutaramide (1.16 g) in anhydrous tetrahydrofuran (20 mL), said solution having been held under stirring in an ice bath, and the resulting mixture was stirred at 65°C for 2 hours. Under ice cooling, methanol (3.0 mL, 74 mmol) was added to the reaction mixture, and the ice bath was removed. Water (2.0 mL), diethyl ether (150 mL) and anhydrous magnesium sulfate (15 g) were added, and the thus-obtained mixture was stirred at room temperature for 1 hour. Insoluble matter was filtered off using Celite, and the filtrate was concentrated under reduced pressure. The residue was purified by chromatography on a silica gel column, whereby N,N'-dibenzyl-N,N'-dimethyl-3-(1-pyrrolidinyl)-1,5-pentanediamine was obtained as a colorless oil (917.3 mg, yield: 85%). To a solution of N, N' -dibenzyl-N,N'-dimethyl-3-(1-pyrrolidinyl)-1,5-pentanediamine (249.1 mg, 0.657 mmol) in methanol (3.0 mL), concentrated hydrochloric acid (0.33 mL, 4.0 mmol) and 10% palladium on charcoal (50. 0 mg) were added, and under a hydrogen gas atmosphere, the resulting mixture was stirred at 50°C for 14 hours. Insoluble matter was filtered off using a membrane filter, and the filtrate was concentrated under reduced pressure. The residue was caused to be adsorbed on an alumina column by chromatography, and was eluted with methanol-chloroform (1:4). The eluate was concentrated under reduced pressure, whereby the title compound was obtained as a colorless oil (101.9 mg, yield: 78%)

### Example 1

### N,N'-Bis[2-(2,4-dimethoxyphenyl)-5-pyridyl]-N,N'-dimethylethylenediamine dimethanesulfonate

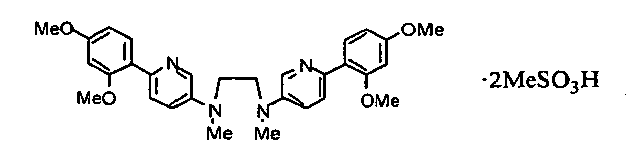

To a solution of N,N'-bis(2-trifluoromethane-sulfonyloxy-5-pyridyl)-N,N'-dimethylethylene diamine (108.0 mg, 0.200 mmol), which had been synthesized in Referential Example 1, in ethanol-toluene (1 : 6, 3.5 mL), 2, 4-dimethoxy-phenylboric acid (108.0 mg, 0.200 mmol), tetrakis-(triphenylphosphine)palladium (46.0 mg, 0.040 mmol) and a 2.0 M aqueous solution of sodium carbonate (0.20 mL, 0.40 mmol) were added. After stirred at 800°C for 2 hours, brine was added, and the resulting mixture was extracted with chloroform. The organic layer was washed with brine, dried over anhydrous sodium sulfate, and then concentrated under reduced pressure. The residue was purified by silica gel chromatography, whereby N,N'-bis[2-(2,4-dimethoxyphenyl)-5-pyridyl]-N,N'-dimethylethylenediamine was obtained as colorless amorphous powder (109.0 mg; quantitative).

To a solution of N,N'-bis[2-(2,4-dimethoxyphenyl)-5-pyridyl]-N,N'-dimethylethylenediamine (108.0 mg, 0.200 mmol) in methanol (2.0 mL), a 1.0 M methanol solution of methane-sulfonic acid (0.44 mL, 0.44 mmol) was added, and the reaction mixture was concentrated under reduced pressure. The residue was recrystallized from methanol-diethyl ether, whereby the title compound was obtained as yellow crystalline powder (melting point: 195.0-197.0°C) (61.0 mg, yield: 43%).
¹H-NMR (DMSO-d₆, 120°C) (ammonium salt NH⁺ protons were not observed) δ: 2.38(s, 6H), 3.07(s, 6H), 3.75(s, 4H), 3.85(s, 12H), 6.70(dd, J=2.4, 8.5Hz, 2H), 6.74(d, J=2.4Hz, 2H), 7.51(d, J=8.5Hz, 2H), 7.69(dd, J=2.9, 9.2Hz, 2H),7.82(d, J=9.2Hz, 2H), 8.01(d, J=2.9Hz, 2H).

### Example 2

### N,N'-Bis[2-(2,3-dimethoxyphenyl)-5-pyridyl]-N,N'-dimethylethylenediamine dimethanesulfonate

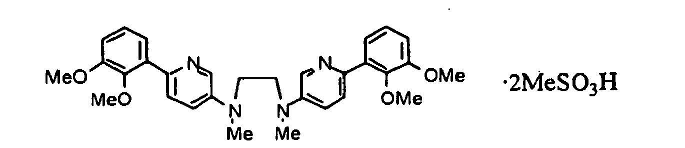

Following the procedures of Example 1, N,N'-bis[2-(2,3-dimethoxyphenyl)-5-pyridyl]-N,N'-dimethylethylene-diamine was obtained as a colorless oil (33.0 mg, yield: 31%) from N,N'-bis(2-trifluoromethanesulfonyloxy-5-pyridyl)-N,N'-dimethylethylenediamine (114.0 mg, 0.212 mmol) synthesized by similar procedures as in Referential Example 1 and 2,3-dimethoxyphenylboric acid (128.0 mg, 0.850 mmol).

To a solution of N,N'-bis[2-(2,3-dimethoxyphenyl)-5-pyridyl]-N,N'-dimethylethylenediamine (33.0 mg, 0.0641 mmol) in chloroform (3.0 mL), a 1.0 M methanol solution of methanesulfonic acid (0.13 mmol) was added, and the reaction mixture was concentrated under reduced pressure. Methanol-diethyl ether was added to the residue and the resulting precipitate was collectedby filtration, whereby the title compound was obtained as yellow crystalline powder (melting point: 191.5-199.5°C) (39.5 mg, yield: 87%).
¹H-NMR (CDCl₃) (ammonium salt NH⁺ protons were not observed) δ: 2.85 (s, 6H), 3.39 (s, 6H),3.79(s, 6H), 3.83 (s, 4H), 3.92 (s, 6H), 7.03-7.40(m, 6H), 7.61(br d, J=9.2Hz, 2H), 7.97(d, J=9.2Hz, 2H), 8.95(br s, 2H)

### Example 3

### N,N'-Bis[2-(2,6-dimethoxyphenyl)-5-pyridyl]-N,N'-dimethylethylenediamine dimethanesulfonate

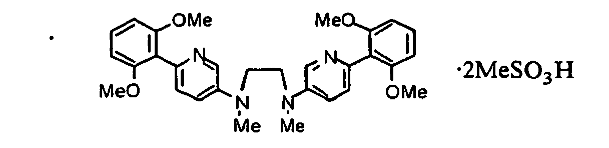

Following the procedures of Example 1, crude crystals were obtained from N,N'-bis(2-trifluoromethanesulfonyloxy-5-pyridyl)-N,N'-dimethylethylenediamine (150.0 mg, 0.279 mmol) synthesized in Referential Example 1 and 2, 6-dimethoxyphenylboric acid (150.7 mg, 1.00 mmol). The crude crystals were recrystallized from chloroform-diethyl ether-hexane, whereby N,N'-bis[2-(2,6-dimethoxyphenyl)-5-pyridyl]-N,N'-dimethylethylenediamine was obtained as colorless crystalline powder (77.3 mg, yield: 54%).

To a solution of N,N'-bis[2-(2,6-dimethoxyphenyl)-5-pyridyl]-N,N'-dimethylethylenediamine (72.3 mg, 0.141 mmol) in methanol-chloroform (9:1, 10 mL), a 1.0 M aqueous solution of methanesulfonic acid (0.35 mL, 0.35 mmol) was added, and the reaction mixture was concentrated under reduced pressure. Ethanol (5.0 mL) was added to the residue, and the resulting mixture was concentrated under reduced pressure. The residue was recrystallized fromethanol-diethyl ether, whereby the title compound was obtained as pale yellow crystalline powder (melting point: 243.0-245.0°C) (94.1 mg, yield: 95%).
¹H-NMR (CDCl₃) (ammonium salt NH⁺ protons were not observed) δ: 2.79(s, 6H), 3.28(s, 6H), 3.79(s, 4H), 3.83(s, 12H), 6.65(d, J=8.5Hz, 4H), 7.39(t, J=8.5Hz, 2H), 7.52(br dd, J=3.1, 9.2Hz, 2H), 7.69(br d, J=9.2Hz, 2H), 8.91(br d, J=3.1Hz, 2H).

### Example 4

### N,N'-Bis[2-(3,5-dimethoxyphenyl)-5-pyridyl]-N,N'-dimethylethylenediamine

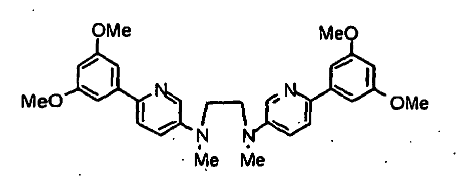

Following the procedures of Example 1, an oil was obtained from N,N'-bis(2-trifluoromethanesulfonyloxy-5-pyridyl)-N,N'-dimethylethylenediamine (108.0 mg, 0.200 mmol) synthesized in Referential Example 1 and 3, 5-dimethoxy-phenylboric acid (91.0 mg, 0. 500 mmol). Chloroform-diethyl ether-hexane was added to the oil and the resulting precipitate was collected by filtration, whereby the title compound was obtained as pale yellow crystalline powder (melting point: 139.0-141.0°C) (41.2 mg, yield: 40%).
¹H-NMR (CDCl₃) δ: 3.02(s, 6H), 3.66(s, 4H), 3.87(s, 12H), 6.46(br s, 2H), 6.99(br d, J=9.2Hz, 2H), 7.11(br s, 4H), 7.57(br d, J=9.2Hz, 2H), 8.22(br s, 2H).

### Example 5

### N,N'-Bis[2-(2, 3,5-trimethoxyphenyl)-5-pyridyl]-N,N'-dimethylethylenediamine

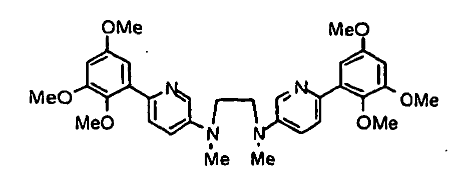

Following the procedures of Example 1, crude crystals were obtained from N,N'-bis(2-trifluoromethanesulfonyloxy-5-pyridyl)-N,N'-dimethylethylenediamine (150.0 mg, 0.280 mmol) synthesized in Referential Example 1 and 2,3,5-trimethoxy-phenylboric acid (131.0 mg, 0.620 mmol). The crude crystals were recrystallized from chloroform-hexane, whereby the title compound was obtained as pale brown needles (melting point: 171.0-172.0°C) (37.0 mg, yield: 56%).
¹H-NMR (CDCl₃) δ: 3.05(s, 6H), 3.60(s, 6H), 3.67(s, 4H), 3.85 (s, 6H), 3.88(s, 6H), 6.50(d, J=2.9Hz, 2H), 6.95(d, J= 2.9Hz, 2H), 7.01(dd, J=2.9, 8.8Hz, 2H), 7.85(d, J=8.8H z, 2H), 8.25(d, J=2.9Hz, 2H).

### Example 6

### N,N'-Bis[2-(2,4,6-trimethoxyphenyl)-5-pyridyl]-N,N'-dimethylethylenediamine dimethanesulfonate

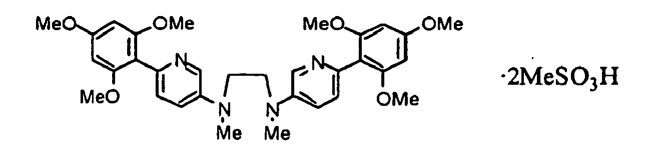

Following the procedures of Example 1, N,N'-bis[2-(2,4,6-trimethoxyphenyl)-5-pyridyl]-N,N'-dimethyl-ethylenediamine was obtained as a colorless oil (20.1 mg, yield: 13%) from N,N'-bis(2-trifluoromethanesulfonyloxy-5-pyridyl)-N,N'-dimethylethylenediamine (150.3 mg, 0.279 mmol) synthesized in Referential Example 1 and 2,4,6-trimethoxy-phenylboric acid (124.5 mg, 0.692 mmol).

To a solution of N,N'-bis[2-(2,4,6-trimethoxyphenyl)-5-pyridyl]-N,N'-dimethylethylenediamine (20.1 mg, 0.0350 mmol) in methanol-chloroform (1:1, 5 mL), a 1.0 M aqueous solution of methanesulfonic acid (0.090 mL, 0.090 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as pale yellow fine scales (melting point: 264.0-265.0°C) (15.6 mg, yield: 59%).
¹H-NMR [CD₃OD-CDCl₃(1:20)] (ammonium salt NH⁺ protons were not observed) δ: 2.77(s, 6H), 3.19(s, 6H), 3.80(s, 4H), 3.82(s, 12H), 3.88(s, 6H), 6.21(s, 4H), 7.59(dd, J=3.1, 9.3Hz, 2H), 7.69(d, J=9.3Hz, 2H), 8.50(dd, J=3.1Hz, 2H).

### Example 7

### N,N'-Bis[2-(3,4,5-trimethoxyphenyl)-5-pyridyl]-N,N'-dimethylethylenediamine dimethanesulfonate

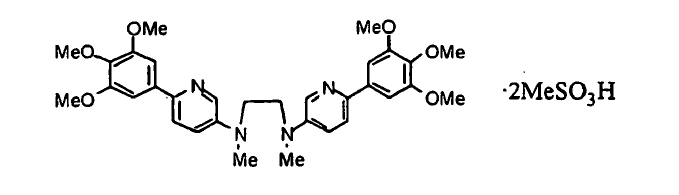

Under an argon atmosphere, tri-t-butylphosphine (101.0 mg, 0.500 mmol) was added to a solution of bis(dibenzylideneacetone)palladium (71.0 mg, 0.120 mmol) in o-xylene (3.0 mL). To the resulting solution, 5-chloro-2-(3,4,5-trimethoxyphenyl)pyridine (335.0 mg, 1.20 mmol), which had been synthesized in Referential Example 2, and N,N'-dimethylethylenediamine (44.0 mg, 0.500 mmol) and sodium t-butoxide (106.0 mg, 1.10 mmol) were added. After stirred at 120°C for 12 hours, water was added to the reaction mixture, and the water layer was extracted with chloroform. The organic layers were combined, dried over anhydrous sodium sulfate, and then concentrated under reduced pressure. The residue was purified by chromatography on a silica gel column, whereby N,N'-bis[2-(3,4,5-trimethoxyphenyl)-5-pyridyl]-N,N'-dimethylethylenediamine was obtained as pale yellow amorphous powder (167.0 mg, yield: 58%).

To a solution of N,N'-bis[2-(3,4,5-trimethoxyphenyl)-5-pyridyl]-N,N'-dimethylethylenediamine (140.0 mg, 0.240 mmol) in methanol-methylene chloride (1:1, 6.0 mL), a 1.0 M aqueous solution of methanesulfonic acid (0. 48 mL, 0. 48 mmol) was added, and the reaction mixture was concentrated under reduced pressure. Ethanol (5.0 mL) was added to the residue, and the resulting mixture was concentrated under reduced pressure. The residue was recrystallized from methanol-methylene chloride-diethyl ether, whereby the title compound was obtained as yellow crystalline powder (melting point: 247.0-249.0°C) (140.0 mg, yield: 76%).
¹H-NMR [DMSO-d₆, 120°rC] (ammonium salt NH⁺ protons were not observed) δ: 2.43(s, 6H), 3.05(s, 6H), 3.73(s, 4H), 3.76(s, 6H), 3.86(s, 12H), 7.16(s, 4H), 7.47(dd, J=3.1, 9.0Hz, 2H), 7.86(d, J=9.0Hz, 2H), 8.07(d, J=3.1Hz, 2H).

### Example 8

### N,N'-Bis[2-(4-ethoxy-3,5-dimethoxyphenyl)-5-pyridyl]-N,N'-dimethylethylenediamine dimethanesulfonate

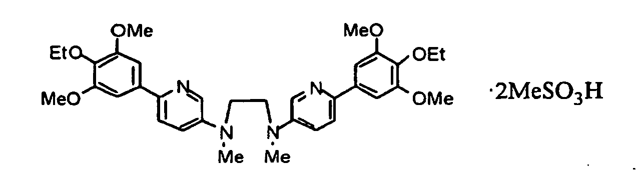

Following the procedures of Example 1, N,N'-bis[2-(4-ethoxy-3,5-dimethoxyphenyl)-5-pyridyl]-N,N'-dimethyl-ethylenediamine was obtained as a colorless oil (133.0 mg, yield: 79%) from N,N'-bis(2-trifluoromethanesulfonyloxy-5-pyridyl)-N,N'-dimethylethylenediamine (150.0 mg, 0.280 mmol) synthesized in Referential Example 1 and 4-ethoxy-3,5-dimethoxyphenylboric acid (140.0 mg, 0. 620 mmol) synthesized in Referential Example 3.

To a solution of N,N'-bis[2-(4-ethoxy-3,5-dimethoxy-phenyl)-5-pyridyl]-N,N'-dimethylethylenediamine (133.0 mg, 0.220mmol) inmethanol (2.0 mL), a 1.0 M aqueous solution of methanesulfonic acid (0.44 mL, 0.44 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (melting point: 232.0-234.0°C) (120.0 mg, yield: 54%).
¹H-NMR [CDCl₃] (ammonium salt NH⁺ protons were not observed) δ: 1.38(t, J=7.1Hz, 6H), 2.87(s, 6H), 3.32(s, 6H), 3.80(s, 4H), 3.95(s, 12H), 4.12(q, J=7.1Hz, 4H), 7.05(s, 4H), 7.70(dd, J=2.9, 9.3Hz, 2H), 7.88(d, J=9.3, Hz, 2H), 8.71(d, J=2.9Hz, 2H).

### Example 9

### N,N'-Bis[2-(3,5-dimethoxy-4-propoxyphenyl)-5-pyridyl]-N,N'-dimethylethylenediamine dimethanesulfonate

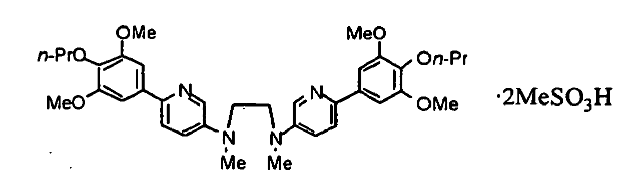

Following the procedures of Example 1, N,N'-bis[2-(3,5-dimethoxy-4-propoxyphenyl)-5-pyridyl]-N,N'-dimethyl-ethylenediamine was obtained as a colorless oil (124.0 mg, yield: 98%) from N,N'-bis(2-trifluoromethanesulfonyloxy-5-pyridyl)-N,N'-dimethylethylenediamine (108.0 mg, 0.200 mmol) synthesized in Referential Example 1 and 4-propoxy-3,5-dimethoxyphenylboric acid (105.0 mg, 0.440 mmol) synthesized by similar procedures as in Referential Example 3.

To a solution of N,N'-bis[2-(3,5-dimethoxy-4-propoxyphenyl)-5-pyridyl]-N,N'-dimethylethylenediamine (124.0 mg, 0.196 mmol) in methanol-chloroform (1:2, 3.0 mL), a 1.0 M aqueous solution of methanesulfonic acid (0.41 mL, 0.41 mmol) was added, and the reaction mixture was concentrated under reduced pressure. Ethanol (5.0 mL) was added to the residue, and the resulting mixture was concentrated under reduced pressure. The residue was recrystallized from ethanol-diethyl ether-hexane, whereby the title compound was obtained as yellow crystalline powder (melting point: 241.0-243.0°C) (91.0 mg, yield: 56%).
¹H-NMR [MDSO-d₆, 120°C] (ammonium salt NH⁺ protons were not observed) δ: 0.98(t, J=7.0Hz, 6H), 1.67(tq, J=7.0, 7.0Hz, 4H), 2.44(s, 6H), 3.06(s, 6H), 3.73(s, 4H), 3.85(s, 12H), 3.91(t, J=7.0Hz, 4H), 7.16(s, 4H), 7.47(dd, J=2.9, 9.0Hz, 2H), 7.86(d, J=9.0Hz, 2H), 8.06(d, J=2.9Hz, 2H).

### Example 10

### N,N'-Bis[2-(4-isopropoxy-3,5-dimethoxyphenyl)-5-pyridyl]-N,N'-dimethylethylenediamine dimethanesulfonate

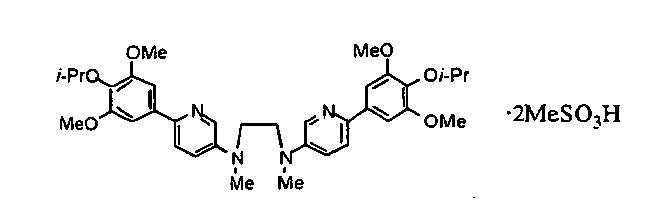

Following the procedures of Example 1, N,N'-bis[2-(4-isopropxy-3,5-dimethoxyphenyl)-5-pyridyl]-N,N'-dimethyl-ethylenediamine was obtained as a colorless oil (74.0 mg, yield: 92%) from N,N'-bis(2-trifluoromethanesulfonyloxy-5-pyridyl)-N,N'-dimethylethylenediamine (69.0 mg, 0.130 mmol) synthesized in Referential Example 1 and 4-isopropoxy-3,5-dimethoxyphenylboric acid (61.0 mg, 0.260 mmol) synthesized by similar procedures as in Referential Example 3.

To a solution of N,N'-bis[2-(4-isopropoxy-3,5-dimethoxy-phenyl)-5-pyridyl]-N,N'-dimethylethylenediamine (74.0 mg, 0.12 mmol) in methanol (2.0 mL), a 1.0 M aqueous solution of methanesulfonic acid (0.26 mL, 0.26 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (melting point: 250.0-253.0°C) (60.0 mg, yield: 57%).
¹H-NMR [CDCl₃] (ammonium salt NH⁺ protons were not observed) δ: 1.31(d, J=6.1Hz, 12H), 2.86(s, 6H), 3.32(s, 6H), 3.80(s, 4H), 3.94(s, 12H), 4.46(qq, J=6.1, 6.1Hz, 2H), 7.06(s, 4H), 7.68(dd, J=3.2, 9.3Hz, 2H), 7.87(d, J=9.3Hz, 2H), 8.74(d, J=3.2Hz, 2H).

### Example 11

### N,N'-Bis[2-[3,5-dimethoxy-4-(2,2,2-trifluoroethoxy)-phenyl]-5-pyridyl]-N,N'-dimethylethylenediamine dimethanesulfonate

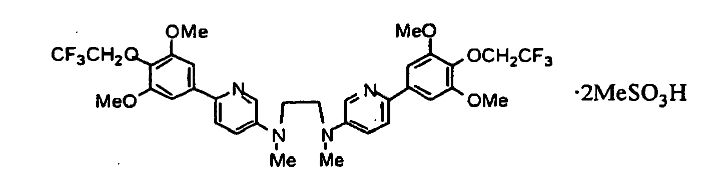

Following the procedures of Example 1, N,N'-bis[2-[3,5-dimethoxy-4-(2,2,2-trifluoroethoxy)phenyl]-5-pyridyl]-N,N'-dimethylethylenediamine was obtained (80.0 mg, yield: 46%) from N,N'-bis(2-trifluoromethanesulfonyloxy-5-pyridyl)-N,N'-dimethylethylenediamine (128.0 mg, 0.240 mmol) synthesized in Referential Example 1 and 3,5-dimethoxy-4-(2,2,2-trifluoroethoxy)phenylboric acid (146.0 mg, 0.530 mmol) synthesized in Referential Example 4.

To a solution of N,N'-bis[2-[3,5-dimethoxy-4-(2,2,2-trifluoroethoxy)phenyl]-5-pyridyl]-N,N'-dimethylethylene-diamine (80.0 mg, 0.11 mmol) in methanol (2.0 mL), a 1.0 M aqueous solution of methanesulfonic acid (0.22 mL, 0.22 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (meltingpoint: 253. 0-257.0°C) (47.0 mg, yield: 22%).
¹H-NMR [MDSO, 120°C] (ammonium salt NH⁺ protons were not observed) δ: 2.41(s, 6H), 3.04(s, 6H), 3.70(s, 4H), 3.88(s, 12H), 4.46(q, ³J_{HF}=9.0Hz, 4H), 7.22(s, 4H), 7.32(dd, J=3.2, 9.0Hz, 2H), 7.80(d, J=9.0Hz, 2H), 8.11(d, J=3.2Hz, 2H).

### Example 12

### N,N'-Bis[2-[4-(2-methoxyethoxy)-3,5-dimethoxyphenyl]-5-pyridyl]-N,N'-dimethylethylenediamine dimethanesulfonate

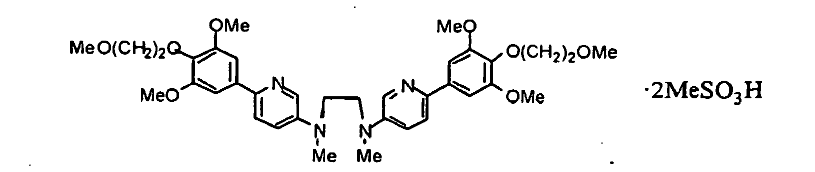

Following the procedures of Example 1, N, N' -bis [2- [4- (2-methoxyethoxy)-3,5-dimethoxyphenyl]-5-pyridyl]-N,N'-dimethylethylenediamine was obtained (139.0 mg, yield: 75%) from N,N'-bis(2-trifluoromethanesulfonyloxy-5-pyridyl)-N,N'-dimethylethylenediamine (150.0 mg, 0.280 mmol) synthesized in Referential Example 1 and 4-(2-methoxy-ethoxy)-3,5-dimethoxyphenylboric acid(158.0 mg, 0.620 mmol) synthesized in Referential Example 5.

To a solution of N,N'-bis[2-[4-(2-methoxyethoxy)-3,5-dimethoxyphenyl]-5-pyridyl]-N,N'-dimethylethylenediamine (139.0 mg, 0.21mmol) in methanol (2. 0 mL), a 1.0 M aqueous solution of methanesulfonic acid (0.42 mL, 0.42 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (melting point: 211.0-214.0°C) (127.0 mg, yield: 71%).
¹H-NMR [CDCl₃] (ammonium salt NH⁺ protons were not observed) δ: 2.86(s, 6H), 3.32(s, 6H), 3.44(s, 6H), 3.69-3.74(m, 4H), 3.80(s, 4H), 3.95(s, 12H), 4.16-4.21(m, 4H), 7.05(s, 4H), 7.69(dd, J=2.9, 9.3Hz, 2H), 7.88(d, J=9.3Hz, 2H), 8.73(d, J=2.9Hz, 2H).

### Example 13

### N,N'-Bis[2-[4-(2-hydroxyethoxy)-3,5-dimethoxyphenyl]-5-pyridyl]-N,N'-dimethylethylenediamine

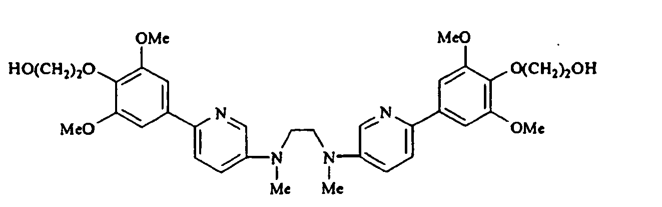

Following the procedures of Example 1, an oil (357.0 mg) which contained N,N'-bis[2-[4-[2-(t-butyldimethylsiloxy)-ethoxy]-3,5-dimethoxyphenyl]-5-pyridyl]-N,N'-dimethyl-ethylenediamine was obtained from N,N'-bis(2-trifluoro-methanesulfonyloxy-5-pyridyl)-N,N'-dimethylethylenediamine (162.0 mg, 0.300 mmol) synthesized in Referential Example 1 and 4-[2-(t-butyl-dimethylsiloxy)ethoxy]-3,5-dimethoxyphenylboric acid (374.0 mg, 1.05 mmol) synthesized in Referential Example 6.

To a solution of the oil (357.0 mg) obtained by the above-described procedures in acetonitrile (19 mL), 46% hydrofluoric acid (1.0 mL) was added. After stirred for 3 hours, a 1.0 M aqueous solution of sodium hydroxide was added to the reaction mixture, and the resulting mixture was concentrated under reduced pressure to remove the organic solvent. The residue was extracted with chloroform. The organic layer was dried over anhydrous sodium sulfate and then concentrated under reduced pressure. The residue was purified by chromatography to obtain an oil. Methanol-chloroform-diethyl ether-hexane was 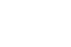 added to the oil and the resulting precipitate was collected by filtration, whereby the title compound was obtained as yellow crystalline powder (melting point: 178. 0-180. 0°C) (141.0 mg, yield: 71% based on N,N'-bis(2-trifluoro-methane-sulfonyloxy-5-pyridyl)-N,N'-dimethylethylenediamine).
¹H-NMR [CDCl₃] δ: 3.03(s, 6H), 3.53(br s, 2H), 3.67(s, 4H), 3.69-3.79(m, 4H), 3.96(s, 12H), 4.14-4.25(m, 4H), 6.99(dd, J=3.0, 8.8Hz, 2H), 7.18(s, 4H), 7.55(d, J=8.8Hz, 2H), 8.21(d, J=3.0Hz, 2H).

### Example 14

### N,N'-Bis[2-(4-hydroxy-3,5-dimethoxyphenyl)-5-pyridyl]-N,N'-dimethylethylenediamine dimethanesulfonate

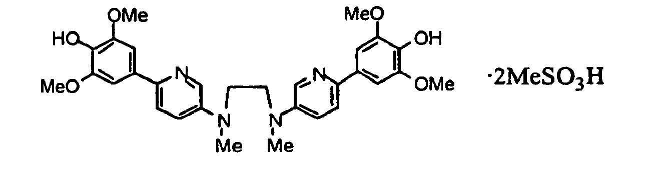

Following the procedures of Example 1, N,N'-bis[2-(4-benzyloxy-3,5-dimethoxyphenyl)-5-pyridyl]-N,N'-dimethyl-ethylenediamine was obtained (64.0 mg, yield: 63%) from N,N'-bis(2-trifluoromethanesulfonyloxy-5-pyridyl)-N,N'-dimethylethylenediamine (75.0 mg, 0.140 mmol) synthesized in Referential Example 1 and 4-benzyloxy-3,5-dimethoxyphenyl-boric acid (81.0 mg, 0.280 mmol).

To a solution of N,N'-bis[2-(4-benzyloxy-3,5-dimethoxyphenyl)-5-pyridyl]-N,N'-dimethylethylenediamine (64.0 mg, 0.090 mmol) in acetic acid (1.0 mL), 10% palladium on charcoal (43.0 mg) was added. Under a hydrogen gas atmosphere, the mixture was stirred at 50°C for 1 hour and 30 minutes. Insoluble matter was filtered off using Celite, and the filtrate was concentrated under reduced pressure. A solution of the residue in methanol-chloroform (1:10) was washed with a saturated aqueous solution of sodium hydrogencarbonate and brine, dried over anhydrous sodium sulfate, and then concentrated under reduced pressure. To a solution of the residue in methanol (2.0 mL), a 1.0 M aqueous solution of methanesulfonic acid (0.20 mL, 0.20 mmol) was added, and the reaction mixture was concentrated under reduced pressure. Ethanol (5.0 mL) was added to the residue, and the thus-obtained mixture was concentrated under reduced pressure. The residue was recrystallized from methanol-diethyl ether, whereby the title compound was obtained as yellow crystalline powder (melting point: ≥300.0°C) (20.0 mg, yield: 31% based on N,N'-bis[2-(4-benzyloxy-3,5-dimethoxyphenyl)-5-pyridyl]-N,N'-dimethylethylenediamine).
¹H-NMR [DMSO, 120°C] (neither ammonium salt NH⁺ protons nor phenol OH protons were observed) δ: 2.38(s, 6H), 3.04 (s, 6H), 3.70(s, 4H), 3.85(s, 12H), 7.15(s, 4H), 7.43(dd, J=2.9, 9.0Hz, 2H), 7.80(d, J=9.0Hz, 2H), 7.99(d, J=2.9Hz, 2H).

### Example 15

### N,N'-Bis[2-(4-butyryloxy-3,5-dimethoxyphenyl)-5-pyridyl]-N,N'-dimethylethylenediamine dimethanesulfonate

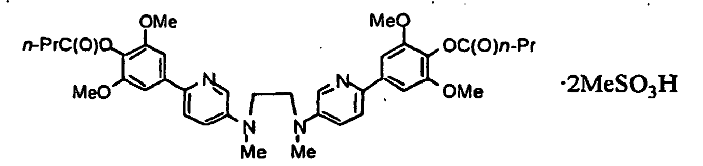

To a solution of N,N'-bis[2-(4-hydroxy-3,5-dimethoxyphenyl)-5-pyridyl]-N,N'-dimethylethylenediamine (74.0 mg, 0.135 mmol), which had been synthesized in Example 14, in pyridine (1.5 mL), butyric acid anhydride (101.0 mg, 0.640 mmol) was added. After stirred at room temperature for 12 hours, the reaction mixture was concentrated under reduced pressure. A saturated aqueous solution of sodium hydrogen-carbonate was added to the residue, and the resulting mixture was extracted with chloroform. The organic layer was washed with brine, dried over anhydrous sodium carbonate, and then concentrated under reduced pressure. To a solution of the residue in methanol (2.0 mL), a 1.0 M aqueous solution of methanesulfonic acid (0.27 mL, 0.27 mmol) was added, and the reaction mixture was concentrated under reduced pressure. Ethanol (5.0 mL) was added to the residue, and the thus-obtained mixture was concentrated under reduced pressure. The residue was recrystallized from chloroform-diethyl ether, whereby the title compound was obtained as yellow crystalline powder (melting point: 196.0-200.0°C) (70.0 mg, yield: 59%).
¹H-NMR (CDCl₃) (ammonium salt NH⁺ protons were not observed) δ: 1.07(t, J=7.3Hz, 6H), 1.82(tq, J=7.3, 7.3Hz, 4H), 2.61(t, J=7.3Hz, 4 H), 2.85(s, 6H), 3.32(s, 6H), 3.81(s, 4H), 3.92(s, 12H), 7.08(s, 4H), 7.70(dd, J=2.9, 9.3Hz, 2H), 7.89(d, J=9.3Hz, 2H), 8.72(d, J=2.9Hz, 2H).

### Example 16

### N,N'-Bis[2-(3-methoxy-4,5-methylenedioxyphenyl)-5-pyridyl]-N,N'-dimethylethylenediamine dimethanesulfonate

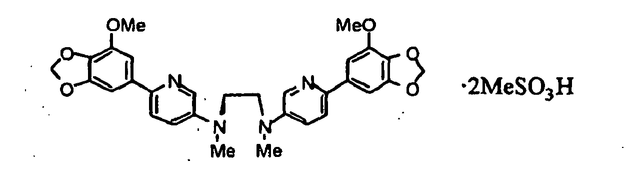

Following the procedures of Example 1, N,N'-bis[2-(3-methoxy-4,5-methylenedioxyphenyl)-5-pyridyl]-N,N'-dimethyl-ethylenediamine was obtained as a colorless oil (109.0 mg, yield: 72%) from N,N'-bis(2-trifluoromethanesulfonyloxy-5-pyridyl)-N,N'-dimethylethylenediamine (150.0 mg, 0.280 mmol) synthesized in Referential Example 1 and 3-methoxy-4, 5-methylenedioxyphenylboric acid (115.0 mg, 0.590 mmol).

To a solution of N,N'-bis[2-(3-methoxy-4,5-methylene-dioxyphenyl)-5-pyridyl]-N,N'-dimethylethylenediamine (109.0 mg, 0.200 mmol) in methanol (3.0 mL), a 1.0 M aqueous solution of methanesulfonic acid (0.40 mL, 0.40 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound (109.0 mg) was obtained as yellow crystalline powder (melting point: 275. 0-276.0°C) (yield: 79%).
¹H-NMR [CD₃OD-CDCl₃ (1:10)] (ammonium salt NH⁺ protons were not observed) δ: 2.84(s, 6H), 3.20(s, 6H), 3.85(s, 4H), 4.02(s, 6H), 6.09(s, 4H), 6.93(d, J=1.7Hz, 2H), 7.06(d, J=1.7Hz, 2H), 7.74(dd, J=2.9, 9.5Hz, 2H), 7.82(d, J=9.5Hz, 2H), 8.34(d, J=2.9Hz, 2H).

### Example 17

### N,N'-Bis[2-(3, 4, 5-triethoxyphenyl)-5-pyridyl]-N,N'-dimethylethylenediamine dimethanesulfonate

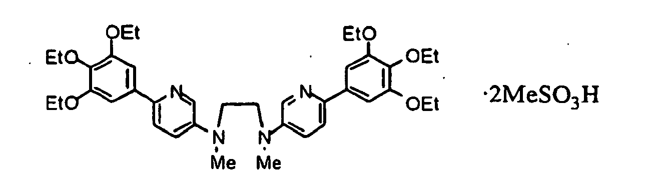

Following the procedures of Example 1, N,N'-bis[2-(3,4,5-triethoxyphenyl)-5-pyridyl]-N,N'-dimethylethylene-diamine was obtained as a pale yellow oil (123.0 mg, yield: 93%) from N,N'-bis(2-trifluoromethanesulfonyloxy-5-pyridyl)-N,N'-dimethylethylenediamine (108.0 mg, 0.200 mmol) synthesized in Referential Example 1 and 3,4,5-triethoxy-phenylboric acid (122.0 mg, 0.480 mmol).

To a solution of N,N'-bis[2-(3,4,5-triethoxyphenyl)-5-pyridyl]-N,N'-dimethylethylenediamine (123.0 mg, 0.186 mmol) in methanol (3.0 mL), a 1.0 M methanol solution of methanesulfonic acid (0.38 mL, 0.38 mmol) was added, and the reaction mixture was concentrated under reduced pressure. Chloroform-diethyl ether was added to the residue and the resulting precipitate was collected, whereby the title compound was obtained as yellow crystalline powder [melting point: 237.0°C (decomposed)] (110.0 mg, yield: 70%).
¹H-NMR [CDCl₃] (ammonium salt NH⁺ protons were not observed) δ: 1.37(t, J=7.0Hz, 6H), 1.46(t, J=6.8Hz, 12H), 2.87(s, 6H), 3.31(s, 6H), 3.79(s, 4H), 4.12(q, J=7.0Hz, 4H), 4.17 (q, J=6.8Hz, 8H), 7.01(s, 4H), 7.67(br d, J=9.0Hz, 2H), 7.82(d, J=9.0Hz, 2H), 8.69(br s, 2H).

### Example 18

### N,N'-Bis[2-(3,5-dimethoxy-4-methylthiophenyl)-5-pyridyl]-N,N'-dimethylethylenediamine dimethanesulfonate

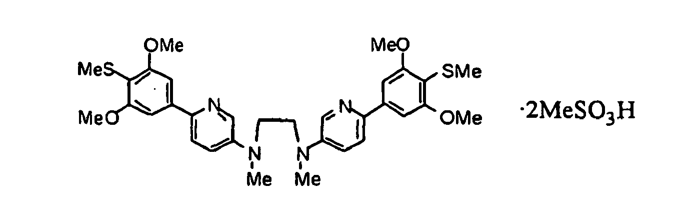

Following the procedures of Example 1, N,N'-bis[2-(3,5-dimethoxy-4-methylthiophenyl)-5-pyridyl]-N,N'-dimethylethylenediamine was obtained as a colorless viscous oil (61.0 mg, yield: 80%) from N,N'-bis(2-trifluoromethane-sulfonyloxy-5-pyridyl)-N,N'-dimethylethylenediamine (70.0 mg, 0.130 mmol) synthesized in Referential Example 1 and 3,5-dimethoxy-4-methylthiophenylboric acid (60.0 mg, 0.260 mmol).

To a solution of N,N'-bis[2-(3,5-dimethoxy-4-methyl-thiophenyl)-5-pyridyl]-N,N'-dimethylethylenediamine (61.0mg, 0.100 mmol) in methanol (2.0 mL), a 1.0 M aqueous solution of methanesulfonic acid (0.20 mL, 0.20 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (melting point: 242.0-244.0°C) (41.0 mg, yield: 51%).
¹H-NMR (CDCl₃) (ammonium salt NH⁺ protons were not observed) δ: 2.42(s, 6H), 2.86(s, 6H), 3.34(s, 6H), 3.82(s, 4H), 4.00(s, 12H), 7.01(s, 4H), 7.72(dd, J=2.9, 9.3Hz, 2H), 7.93(d, J=9.3Hz, 2H), 8.79(d, J=2.9Hz, 2H).

### Example 19

### N,N'-Bis[2-[3,5-dimethoxy-4-(1-pyrrolidinyl)phenyl]-5-pyridyl]-N,N'-dimethylethylenediamine

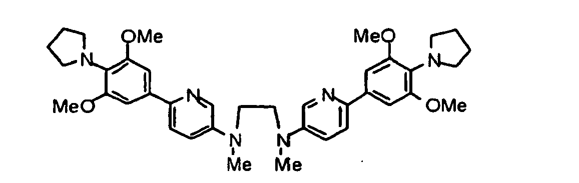

Following the procedures as in Example 1, an oil was obtained from N,N'-bis(2-trifluoromethanesulfonyloxy-5-pyridyl)-N,N'-dimethylethylenediamine (120.0 mg, 0.220 mmol) synthesized in Referential Example 1 and 3,5-dimethoxy-4-(1-pyrrolidinyl)phenylboric acid (138.0 mg, 0.550 mmol). Methanol was added to the oil and the resulting precipitate was collected by filtration, whereby the title compound was obtained as pale brown crystalline powder (melting point: 221.0-225.0°C) (81.0 mg, yield: 56%).
¹H-NMR (CDCl₃) δ: 1.89-1.97(m, 8H), 3.01(s, 6H), 3.28-3.37(m, 8H), 3.65(s, 4H), 3.91(s, 12H), 6.97(dd, J=2.9, 8.8Hz, 2H), 7.14(s, 4H), 7.54(d, J=8.8Hz, 2H), 8.21(d, J=2.9Hz, 2H).

### Example 20

### N,N'-Bis[2-(4-dimethylamino-3,5-dimethoxyphenyl)-5-pyridyl]-N,N'-dimethylethylenediamine

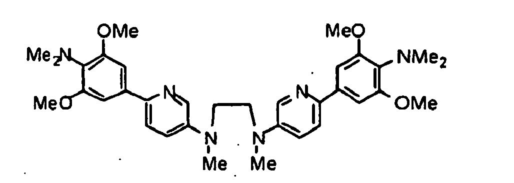

Following the procedures of Example 1, an oil was obtained from N,N'-bis(2-trifluoromethanesulfonyloxy-5-pyridyl)-N,N'-dimethylethylenediamine (129.0 mg, 0.240 mmol) synthesized in Referential Example 1 and 4-dimethylamino-3,5-dimethoxyphenylboric acid (135.0 mg, 0.600 mmol). Methanol was added to the oil and the resulting precipitate was collected by filtration, whereby the title compound was obtained as pale brown crystalline powder (melting point: 194.0-196.0°C) (84.0 mg, yield: 58%).
¹H-NMR (CDCl₃) δ: 2.85(s, 12H), 3.02(s, 6H), 3.66(s, 4H), 3.9 3(s, 12H), 6.98(dd, J=2.9, 9.0Hz, 2H), 7.12(s, 4H), 7. 55(d, J=9.0Hz, 2H), 8.22(d, J=2.9Hz, 2H).

### Example 21

### N,N'-Bis[2-(3,5-dimethoxy-4-methylphenyl)-5-pyridyl]-N,N'-dimethylethylenediamine dimethanesulfonate

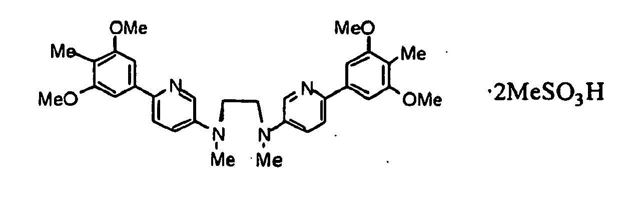

Following the procedures of Example 7, N,N'-bis[2-(3,5-dimethoxy-4-methylphenyl)-5-pyridyl]-N,N'-dimethyl-ethylenediamine was obtained as a colorless oil (37.0 mg, yield: 40%) from 5-chloro-2-(3,5-dimethoxy-4-methylphenyl)-pyridine (99.0 mg, 0. 370 mmol) synthesized by similar procedures as in Referential Example 2.

To a solution of N,N'-bis[2-(3,5-dimethoxy-4-methyphenyl)-5-pyridyl]-N,N'-dimethylethylenediamine (37.0 mg, 0.070 mmol) in methanol (2.0 mL), a 1.0 M aqueous solution of methanesulfonic acid (0. 15 mL, 0.15 mmol) was added, and the reaction mixture was concentrated under reduced pressure. Ethanol (5.0 mL) was added to the residue, and the resulting mixture was concentrated under reduced pressure. The residue was recrystallized from methanol-chloroform, whereby the title compound was obtained as yellow crystalline powder (melting point: 272.0-274.0°C (22.0 mg, yield: 44%).
¹H-NMR [CD₃OD-CDCl₃(1:10)] (ammonium salt NH⁺ protons were not observed) δ: 2.12(s, 6H), 2.83(s, 6H), 3.24(s, 6H), 3.85(s, 4H), 3.91(s, 12H), 7.23(s, 4H), 7.76(dd, J=2.9, 9.3Hz, 2H), 7.94(d, J=9.3Hz, 2H), 8.45(d, J=2.9Hz, 2H).

### Example 22

### N,N'-Bis[2-(4-fluoro-3,5-dimethoxyphenyl)-5-pyridyl]-N,N'-dimethylethylenediamine dimethanesulfonate

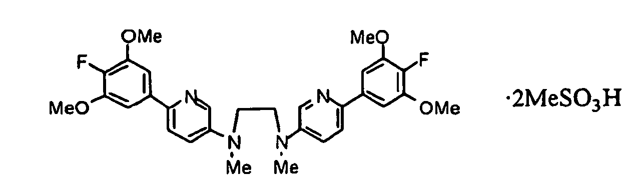

Following the procedures of Example 1, an oil was obtained from N,N'-bis(2-trifluoromethanesulfonyloxy-5-pyridyl)-N,N'-dimethylethylenediamine (150.0 mg, 0.280 mmol) synthesized in Referential Example 1 and 4-fluoro-3,5-dimethoxyphenylboric acid (167.0 mg, 0.840 mmol). Hexane was added to the oil and the resulting precipitate was collected by filtration, whereby N,N'-bis[2-(4-fluoro-3,5-dimethoxy-phenyl)-5-pyridyl]-N,N'-dimethylethylenediamine was obtained as yellow crystalline powder (160.0 mg; quantitative).

To a solution of N,N'-bis[2-(4-fluoro-3,5-dimethoxy-phenyl)-5-pyridyl]-N,N'-dimethylethylenediamine (150.0 mg, 0.270 mmol) in chloroform (30 mL), a 1.0 M aqueous solution of methanesulfonic acid (0.60 mL, 0.60 mmol) was added, and the reaction mixture was concentrated under reduced pressure. Ethanol (5.0 mL) was added to the residue, and the resulting mixture was concentrated under reduced pressure. Diethyl ether was added to the residue and the resulting precipitate was collected by filtration, whereby the title compound was obtained as yellow crystalline powder (melting point: ≥300.0°C) (181.0 mg, yield: 90%).
¹H-NMR (DMSO-d₆) (ammonium salt NH⁺ protons were not observed) δ: 2.33(s, 6H), 3.03(s, 6H), 3.77(br s, 4H), 3.89(s, 12H), 7.25 (d, ⁴J_{HF}=7.0Hz, 4H), 7.50-7.59 (m, 2H), 7.99-8.06(m, 4H).

### Example 23

### N,N'-Bis[2-(4-chloro-3,5-dimethoxyphenyl)-5-pyridyl]-N,N'-dimethylethylenediamine dimethanesulfonate

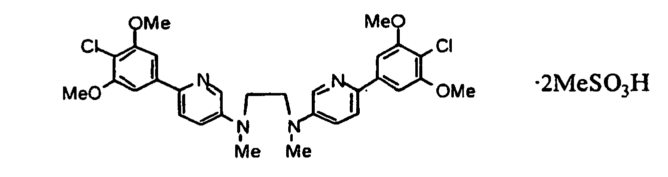

Following the procedures of Example 1, N,N'-bis[2-(4-chloro-3,5-dimethoxyphenyl)-5-pyridyl]-N,N'-dimethyl-ethylenediamine was obtained as pale yellow amorphous powder (101.0 mg, yield: 86%) from N,N'-bis(2-trifluoromethane-sulfonyloxy-5-pyridyl)-N,N'-dimethylethylenediamine (108.0 mg, 0.200 mmol) synthesized in Referential Example 1 and 4-chloro-3,5-dimethoxyphenylboric acid (95.0 mg, 0.440 mmol).

To a solution of N,N'-bis[2-(4-chloro-3,5-dimethoxy-phenyl)-5-pyridyl]-N,N'-dimethylethylenediamine (78.0 mg, 0.130 mmol) in methanol-chloroform (1:2, 50 mL), a 1.0 M aqueous solution of methanesulfonic acid (0.27 mL, 0.27 mmol) was added, and the reaction mixture was concentrated under reduced pressure. Ethanol (5.0 mL) was added to the residue, and the resulting mixture was concentrated under reduced pressure. The residue was recrystallized from methanol-chloroform-diethyl ether, whereby the title compound was obtained as yellow crystalline powder (melting point: 280.0-285.0°C) (83.0 mg, yield: 80%).
¹H-NMR (DMSO-d₆, 120°C) (ammonium salt NH⁺ protons were not observed) δ: 2.45(s, 6H), 3.05(s, 6H), 3.72(s, 4H), 3.91(s, 12H), 7.26(s, 4H), 7.37(dd, J=3.1, 9.0Hz, 2H), 7.87(d, J=9.0Hz, 2H), 8.14(d, J=3.1Hz, 2H).

### Example 24

### N,N'-Bis[2-(4-cyano-3,5-dimethoxyphenyl)-5-pyridyl]-N,N'-dimethylethylenediamine dimethanesulfonate

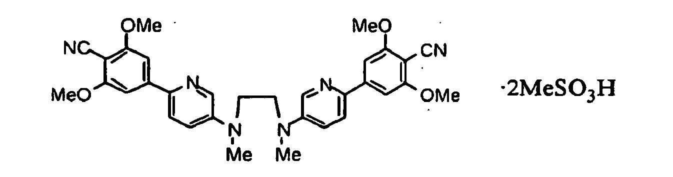

Following the procedures of Example 1, N,N'-bis[2-(4-cyano-3,5-dimethoxyphenyl)-5-pyridyl]-N,N'-dimethyl-ethylenediamine was obtained as a colorless oil (125.0 mg, yield: 79%) from N,N'-bis(2-trifluoromethanesulfonyloxy-5-pyridyl)-N,N'-dimethylethylenediamine (150.0 mg, 0.280 mmol) synthesized in Referential Example 1 and 4-cyano-3,5-dimethoxyphenylboric acid (145.0 mg, 0.700 mmol).

To a solution of N,N'-bis[2-(4-cyano-3,5-dimethoxy-phenyl)-5-pyridyl]-N,N'-dimethylethylenediamine (125.0 mg, 0.220 mmol) inmethanol (2.0 mL), a 1.0 M aqueous solution of methanesulfonic acid (0.44 mL, 0.44 mmol) was added, and the reaction mixture was concentrated under reduced pressure. Ethanol (5.0mL) was added to the residue, and the resulting mixture was concentrated under reduced pressure. Methanol (2.5 mL) was added to the residue. The resulting suspension was stirred in a bath maintained at 70°C, and then allowed to cool down. The resulting precipitate was collected by filtration, whereby the title compound was obtained as yellow crystalline powder (melting point: ≥300.0°C) (72.0 mg, yield: 43%).
¹H-NMR [CD₃OD-CDCl₃(1:10)] (ammonium salt NH⁺ protons were not observed) δ: 2.35(s, 6H), 3.04(s, 6H), 3.77(s, 4H), 3.97(s, 12H), 7.32(s, 4H), 7.28-7.34(m, 2H), 8.06(d, J=9.0Hz, 2H), 8.19(d, J=3.2Hz, 2H).

### Example 25

### N,N'-Bis[2-[3,5-dimethoxy-4-(N-methoxy-N-methylcarbamoyl)-phenyl]-5-pyridyl]-N,N'-dimethylethylenediamine

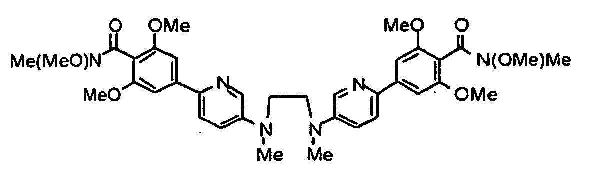

Following the procedures of Example 1, an oil was obtained from N,N'-bis(2-trifluoromethanesulfonyloxy-5-pyridyl)-N,N'-dimethylethylenediamine (76.0 mg, 0.140 mmol) synthesized in Referential Example 1 and 3,5-dimethoxy-4-(N-methoxy-N-methylcarbamoyl)phenylboric acid (91.0 mg, 0.340 mmol) synthesized in Referential Example 7. Chloroform-hexane was added to the oil and the resulting precipitate was collected by filtration, whereby the title compound was obtained as yellow crystalline powder (melting point: 217.0-220.0°C) (34.0 mg, yield: 36%).
¹H-NMR (CDCl₃) (mixture of geometrical isomers at the amide, data of primary isomer) δ: 3.04(s, 6H), 3.40(s, 6H), 3.51(s, 6H), 3.68(s, 4H), 3.91(s, 12H), 7.01(dd, J=2.5, 8.6Hz, 2H), 7.15(s, 4H), 7.59(d, J=8.6Hz, 2H), 8.23(d, J=2.5Hz, 2H).

### Example 26

### N,N'-Bis[2-(4-t-butoxycarbonyl-3,5-dimethoxyphenyl)-5-pyridyl]-N,N'-dimethylethylenediamine

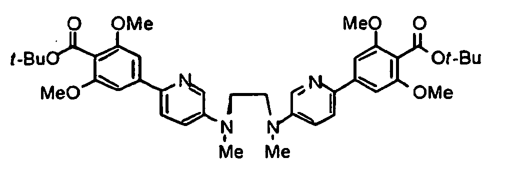

Following the procedures of Example 1, crude crystals were obtained from N,N'-bis(2-trifluoromethanesulfonyloxy-5-pyridyl)-N,N'-dimethylethylenediamine (167.0 mg, 0.300 mmol) synthesized in Referential Example 1 and 4-t-butoxy-carbonyl-3,5-dimethoxyphenylboric acid (187.0 mg, 0.660 mmol). The crude crystals were recrystallized from methylene chloride-diethyl ether-hexane, whereby the title compound was obtained as colorless crystalline powder (melting point: 211.0-213.0°C) (195.0 mg, yield: 90%).
¹H-NMR (CDCl₃) δ: 1.59(s, 18H), 3.02(s, 6H), 3.66(s, 4H), 3.90(s, 12H), 6.98(dd, J=3.1, 8.7Hz, 2H), 7.11(s, 4H), 7.55(d, J=8.7Hz, 2H), 8.22(d, J=3.1Hz, 2H).

### Example 27

### N,N'-Bis[2-(4-carboxy-3,5-dimethoxyphenyl)-5-pyridyl]-N,N'-dimethylethylenediamine ditrifluoroacetate

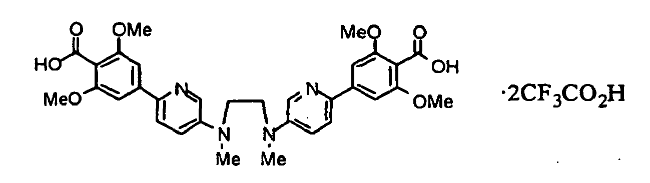

To a solution of N,N'-bis[2-(4-t-butoxycarbonyl-3,5-dimethoxyphenyl)-5-pyridyl]-N,N'-dimethylethylenediamine (72.0 mg, 0.100 mmol), which had been synthesized in Example 26, in methylene chloride (1.0 mL), trifluoroacetic acid (1.0 mL) was added, and the resulting mixture was stirred for 3 hours. Under ice cooling, diethyl ether was added to the reaction mixture and the resulting precipitate was collected by filtration, whereby the title compound was obtained as pale yellow crystalline powder [melting point: 235.0 (decomposed)](72.0 mg, yield: 86%).
¹H-NMR (DMSO-d₆, 120°C) (neither ammonium salt NH⁺ protons nor carboxylic acid CO₂H protons were observed) δ: 3.03(s, 6H), 3.68(s, 4H), 3.84(s, 12H), 7.19(dd, J=3.1, 8.7Hz, 2H), 7.22(s, 4H), 7.76(d, J=8.7Hz, 2H), 8.18(d, J=3.1Hz, 2H).

### Example 28

### N,N'-Bis[2-(4-ethoxycarboxy-3,5-dimethoxyphenyl)-5-pyridyl]-N,N'-dimethylethylenediamine

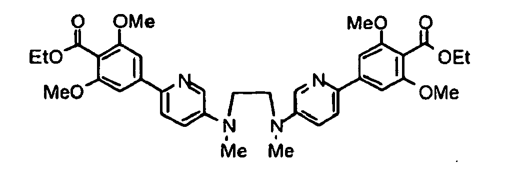

To a solution of N,N'-bis[2-(4-carboxy-3,5-dimethoxy-phenyl)-5-pyridyl]-N,N'-dimethylethylenediamine ditrifluoroacetate (42.0 mg, 0.050 mmol), which had been synthesized in Example 27, in methylene chloride (5.0 mL), dimethylformamide (0.1 mL, 1.29 mmol) and oxalyl chloride (0.21 mL, 2.5 mmol) were added. After the resulting mixture was stirred at room temperature for 30 minutes, ethanol (2.0 mL, 35 mmol) and N, N-diisopropylethylamine (0.51 mL, 3.0 mmol) were added. The reaction mixture was stirred for 1 hour, and then concentrated under reduced pressure. The residue was purified by chromatography on a silica gel column to obtain crude crystals. The crude crystals were recrystallized from methylene chloride-diethyl ether-hexane, whereby the title compound was obtained as pale yellow crystalline powder (melting point: 206.0-208.0°C) (30.0 mg, yield: 86%)
¹H-NMR (CDCl₃) δ: 1.38(t, J=7.0Hz, 6H), 3.03(s, 6H), 3.67(s, 4H), 3.91(s, 12H), 4.40(q, J=7.0Hz, 4H), 7.00(dd, J=2.9, 8.7Hz, 2H), 7.14(s, 4H), 7.57(d, J=8.7Hz, 2H), 8.23(d, J=2.9Hz, 2H).

### Example 29

### N,N'-Bis[2-(4-acetyl-3,5-dimethoxyphenyl)-5-pyridyl]-N,N'-dimethylethylenediamine dimethanesulfonate

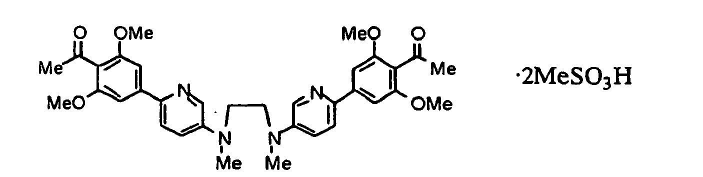

Under a nitrogen gas atmosphere, crude N,N'-bis(2-chloro-5-pyridyl)-N,N'-dimethylethylenediamine(7 8.0 mg, approx. 0.25 mmol) synthesized in Referential Example 9 and tetrakis(triphenylphosphine)palladium, (116.0 mg, 0.100 mmol) were added a solution of (4-acetyl-3,5-dimethoxyphenyl)-trimethyltin (235.0 mg, 0.760 mmol), which had been synthesized in Referential Example 8, in toluene (10 mL). After stirred at 130°C for 22 hours, a saturated aqueous solution of potassium fluoride was added to the reaction mixture, followed by stirring for 1 hour. Insoluble matter was filtered off using Celite. The residue was washed with chloroform, and the water layer in the filtrate was extracted with chloroform. The washing and the organic layer were combined, dried over anhydrous sodium sulfate, and then concentrated under reduced pressure. The residue was purified by chromatography on a silica gel column, whereby N,N'-bis[2-(4-acetyl-3,5-dimethoxyphenyl)-5-pyridyl]-N,N'-dimethylethylenediamine was obtained as pale yellow crystalline powder (41.4 mg, yield: 23% based on N,N'-bis[2-hydroxy-5-pyridyl]-N,N'-dimethylethylenediamine ditrifluoroacetate).

To a solution of N,N'-bis[2-(4-acetyl-3,5-dimethoxy-phenyl)-5-pyridyl]-N,N'-dimethylethylenediamine (76.4 mg, 0.128 mmol) in chloroform (5.0 mL), a 1.0 M methanol solution of methanesulfonic acid (0.32 mL, 0.32 mmol) was added, and the reaction mixture was concentrated under reduced pressure. Chloroform-diethyl ether was added to the residue and the resulting precipitate was collected by filtration, whereby the title compound was obtained as yellow crystalline powder (melting point: 261.0-265.0°C) (75.1 mg, yield: 74%).
¹H-NMR (CDCl₃) (ammonium salt NH⁺ protons were not observed) δ: 2.49(s, 6H), 2.86(s, 6H), 3.35(s, 6H), 3.80(s, 4H), 3.92(s, 12H), 7.03(s, 4H), 7.72(br d, J=9.2Hz, 2H), 7.94 (d, J=9.2Hz, 2H), 8.82 (br s, 2H).

### Example 30

### 1,4-Bis[2-(3,4,5-trimethoxyphenyl)-5-pyridyl]hexahydro-1,4-diazepine dimethanesulfonate

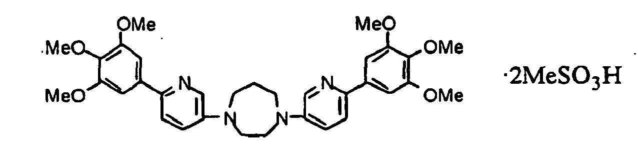

Following the procedures of Example 7, 1,4-bis[2-(3,4,5-trimethoxyphenyl)-5-pyridyl]hexahydro-1,4-diazepine was obtained as yellow amorphous powder (174.0 mg, yield: 59%) from 5-chloro-2-(3,4,5-trimethoxypheny)pyridine (335.0 mg, 1.20 mmol) synthesized in Referential Example 2 and hexahydro-1,4-diazepine (50.0 mg, 0.500 mmol).

To a solution of 1,4-bis[2-(3,4,5-trimethoxyphenyl)-5-pyridyl]hexahydro-1,4-diazepine (88.0 mg, 0.150 mmol) in methanol-methylene chloride (1:1, 4.0mL), a 1.0 M aqueous solution of methanesulfonic acid (0.30 mL, 0.30 mmol) was added, and the reaction mixture was concentrated under reduced pressure. Ethanol (5.0 mL) was added to the residue, and the resulting mixture was concentrated under reduced pressure. The residue was recrystallized from methanol-methylene chloride-diethyl ether, whereby the title compound was obtained as yellow prisms (melting point: 246.0-248.0°C) (93.0 mg, yield: 79%).
¹H-NMR (DMSO-d₆, 120°C) (ammonium salt NH⁺ protons were not observed) δ: 2.04(tt, J=5.8, 5.8Hz, 2H), 2.42(s, 6H), 3.64(dd, J=5.8, 5.8Hz, 4H), 3.75(s, 6H), 3.83(s, 4H), 3.84(s, 12H), 7.14(s, 4H), 7.52(dd, J=3.1, 9.0Hz, 2H), 7.82(d, J=9.0Hz, 2H), 8.14(d, J=3.1Hz, 2H).

### Example 31

### 1,4-Bis[2-(3,4,5-trimethoxyphenyl)-5-pyridyl]piperazine dimethanesulfonate

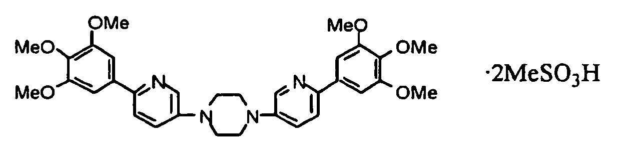

Following the procedures of Example 7, 1,4-bis[2-(3,4,5-trimethoxyphenyl)-5-pyridyl]piperazine was obtained as pale yellow needles (174.0 mg, yield: 61%) from 5-chloro-2-(3,4,5-trimethoxypheny)pyridine (335.0 mg, 1.20 mmol) synthesized in Referential Example 2 and piperazine (43.0 mg, 0.500 mmol).

To a solution of 1,4-bis[2-(3,4,5-trimethoxyphenyl)-5-pyridyl]piperazine (143.0 mg, 0.250 mmol) in methanol-methylene chloride (1:2, 6.0 mL), a 1.0 M aqueous solution of methanesulfonic acid (0.50 mL, 0. 50 mmol) was added, and the reaction mixture was concentrated under reduced pressure. Ethanol (5.0 mL) was added to the residue, and the resulting mixture was concentrated under reduced pressure. The residue was recrystallized from methanol-chloroform-diethyl ether, whereby the title compound was obtained as yellow needles [melting point: 262.0°C (decomposed) (171.0 mg, yield: 89%).
¹H-NMR (CDCl₃) (data of the free base of the title compound) δ: 3.46(s, 8H), 3.89(s, 6H), 3.96(s, 12H), 7.19(s, 4H), 7.32(dd, J=3.1, 8.7Hz, 2H), 7.63(d, J=8.7Hz, 2H), 8.43(d, J=3.1Hz, 2H).

### Example 32

### 1,3-Bis[4-[2-(3,4,5-trimethoxyphenyl)-5-pyridyl]-1-piperazinyl]propane tetrahydrochloride

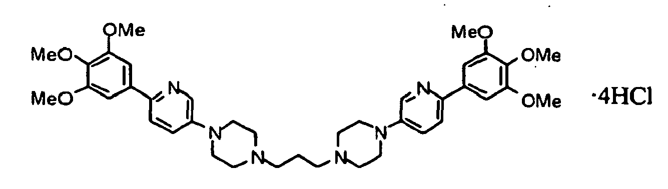

Following the procedures of Example 7, 1,3-bis[4-[2-(3,4,5-trimethoxyphenyl)-5-pyridyl]-1-piperazinyl]propane was obtained as a pale yellow oil (44.0 mg, yield: 25%) from 5-chloro-2-(3,4,5-trimethoxypheny)pyridine (166.0 mg, 0. 600 mmol) synthesized in Referential Example 2 and 1,3-bis(1-piperazinyl)propane (90.0 mg, 0.250.mmol).

To a solution of 1,3-bis[4-[2-(3,4,5-trimethoxy-phenyl)-5-pyridyl]-1-piperazinyl]propane (44.0 mg, 0.060 mmol) inmethanol-methylene chloride (1:2, 3.0mL), 1.0 M hydrochloric acid (0.30 mL, 0. 30 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder [melting point: 264.°C (decomposed)] (35.0 mg, yield: 69%).
¹H-NMR (CDCl₃) (ammonium salt NH⁺ protons were not observed) δ: 1.72-1.87(m, 2H), 2.46-2.56(m, 4H), 2.61-2.71(m, 8H), 3.24-3.35(m, 8H), 3.88(s, 6H), 3.95(s, 12H), 7.16(s, 4H), 7.24(dd, J=2.9, 8.7Hz, 2H), 7.58(d, J=8.7Hz, 2H), 8.37(d, J=2.9Hz, 2H).

### Example 33

### N,N'-Bis[2-(3,4,5-trimethoxyphenyl)-5-pyridyl]ethylenediamine

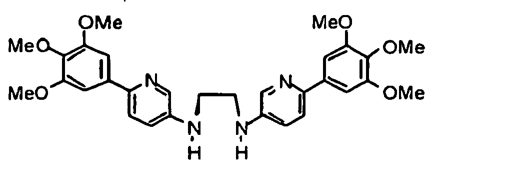

Following the procedures of Example 7, the title compound was obtained as pale yellow amorphous powder (21.0 mg, yield: 7%) from 5-chloro-2-(3,4,5-trimethoxypheny)pyridine (335.0 mg, 1.20 mmol) synthesized in Referential Example 2 and ethylenediamine (30.0 mg, 0.500 mmol).
¹H-NMR (CDCl₃) δ: 3.50(s, 4H), 3.88(s, 6H), 3. 95 (s, 12H), 4.01-4.09 (br s, 2H), 7.01(dd, J=2.9, 8.5Hz, 2H), 7.14(s, 4H), 7.53(d, J=8.5Hz, 2H), 8.16(d, J=2.9Hz, 2H)

### Example 34

### Cis-N,N'-bis[2-(3,4,5-trimethoxyphenyl)-5-pyridyl]-1,2-cyclohexanediamine

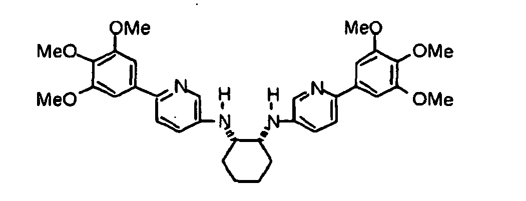

Following the procedures of Example 7, the title compound was obtained as pale yellow amorphous powder (37.0 mg, yield: 12%) from5-chloro-2- (3, 4, 5-trimethoxypheny)pyridine (166.0mg, 0.600 mmol) synthesized in Referential Example 2 and cis-1,2-cyclohexanediamine (24.0 mg, 0.200 mmol).
¹H-NMR (CDCl₃) δ: 1.53-1.72(m, 6H), 1.79-1.91(m, 2H), 3.75-3.85(m, 2H), 3.88(s, 6H), 3.94(s, 12H), 3.94-4.02(m, 2H), 6.96(dd, J=2.6, 8.5Hz, 2H), 7.13(s, 4H), 7.48(d, J=8.5Hz, 2H), 8.13(d, J=2.6Hz, 2H).

### Example 35

### Trans-N,N'-bis[2-(3,4,5-trimethoxyphenyl)-5-pyridyl]-1,4-cyclohexanediamine

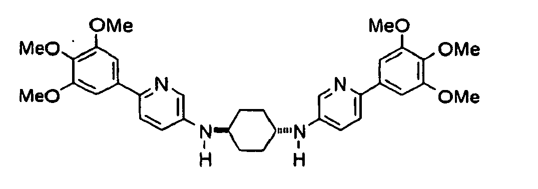

Following the procedures of Example 7, crude crystals was obtained (66.0 mg, yield: 21%) from 5-chloro-2-(3,4,5-trimethoxypheny)pyridine (335.0 mg, 1.20 mmol) synthesized in Referential Example 2 and trans-1, 4-cyclohexanediamine (57.0 mg, 0.500 mmol). The crude crystals were recrystallized from chloroform-diethyl ether-hexane, whereby the title compound was obtained as pale brown crystalline powder (melting point: 264.0-267.0°C)(27.0 mg, yield: 9%).
¹H-NMR [CD₃OD-CDCl₃ (1:10)] (amine NH protons were not observed) δ: 1.34-1.46(m, 4H), 2.19-2.31(m, 4H), 3.27-3.35(m, 2H), 3.87(s, 6H), 3.95(s, 12H), 7.00(dd, J=2.9, 8.7Hz, 2H), 7.07(s, 4H), 7.51(d, J=8.7Hz, 2H), 8.03(d, J=2.9Hz, 2H).

### Example 36

### N,N'-Bis[2-(3,4,5-trimethoxyphenyl)-5-pyridyl]-N,N'-diethylethylenediamine dimethanesulfonate

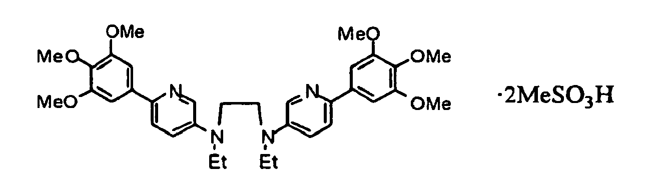

Following the procedures of Example 7, N,N'-bis[2-(3,4,5-trimethoxyphenyl)-5-pyridyl]-N,N'-diethylethylene-diamine was obtained as pale yellow crystalline powder (100.0 mg, yield: 33%) from 5-chloro-2-(3,4,5-trimethoxypheny)-pyridine (335.0 mg, 1.20 mmol) synthesized in Referential Example 2 and N,N'-diethylethylenediamine (58.0 mg, 0.500 mmol).

To a solution of N,N'-bis[2-(3,4,5-trimethoxy-phenyl)-5-pyridyl]-N,N'-diethylethylenediamine (100.0 mg, 0.165 mmol) in methanol-chloroform (1:1, 6.0 mL), a 1.0 M aqueous solution of methanesulfonic acid (0.33 mL, 0.33 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (melting point: 254.0-256.0°C).
¹H-NMR (DMSO-d₆, 120°C) (ammonium salt NH⁺ protons were not observed) δ: 1.17(t, J=6.8Hz, 6H), 2.41(s, 6H), 3.50(q, J=6.8Hz, 4H), 3.66(s, 4H), 3.76(s, 6H), 3.86(s, 12H), 7.18(s, 4H), 7.45(dd, J=2.9, 9.0Hz, 2H), 7.85(d, J=9.0Hz, 2H), 8.09(d, J=2.9Hz, 2H).

### Example 37

### N,N'-Bis[2-(3,4,5-trimethoxyphenyl)-5-pyridyl]-N,N'-dimethyl-1,3-propanediamine dimethanesulfonate

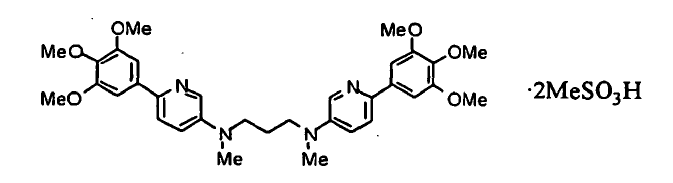

Following the procedures of Example 7, crude N,N'-bis[2-(3,4,5-trimethoxyphenyl)-5-pyridyl]-N,N'-dimethyl-1,3-propanediamine was obtained as a pale brown oil (275.0 mg) from 5-chloro-2-(3,4,5-trimethoxypheny)pyridine (335.0 mg, 1.20 mmol) synthesized in Referential Example 2 and N,N'-dimethyl-1,3-propanediamine (65.0 mg, 0.500 mmol).

To a solution of the crude N,N'-bis[2-(3,4,5-trimethoxyphenyl)-5-pyridyl]-N,N'-dimethyl-1,3-propane-diamine (275.0 mg, approx. 0.46 mmol) in methanol-chloroform (1:1, 6.0 mL), a 1.0 M aqueous solution of methanesulfonic acid (0.93 mL, 0.93 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow needles (melting point: 216.0-219.°C) (255.0 mg, yield: 70% based on N,N'-dimethyl-1,3-propanediamine).
¹H-NMR (DMSO-d₆, 120°C) (ammonium salt NH⁺ protons were not observed) δ: 1.94(tt, J=7.3Hz, 2H), 2.43(s, 6H), 3.05(s, 6H), 3.52(t, J=7.3Hz, 4H), 3.76(s, 6H), 3.86(s, 12H), 7.15(s, 4H), 7.53(dd, J=3.1, 9.2Hz, 2H), 7.88(d, J=9.2Hz, 2H), 8.10(d, J=3.1Hz, 2H).

### Example 38

### N,N'-Bis[2-(3,4,5-trimethoxyphenyl)-5-pyridyl]-N,N'-diethyl-1,3-propanediamine dimethanesulfonate

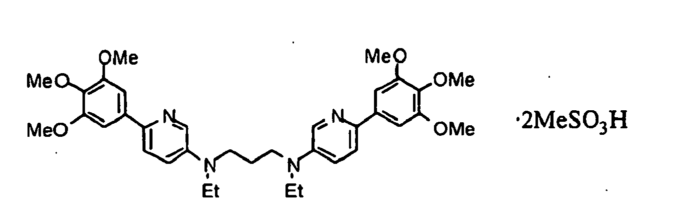

Following the procedures of Example 7, crude N,N'-bis[2-(3,4,5-trimethoxyphenyl)-5-pyridyl]-N,N'-diethyl-1,3-propanediamine was obtained as pale brown crystalline powder (160.0 mg) from 5-chloro-2-(3,4,5-trimethoxypheny)pyridine (335.0 mg, 1.20 mmol) synthesized in Referential Example 2 and N,N'-diethyl-1,3-propanediamine (65.0 mg, 0.500 mmol).

To a solution of the crude N,N'-bis[2-(3,4,5-trimethoxyphenyl)-5-pyridyl]-N,N'-diethyl-1,3-propane-diamine (160.0 mg, approx. 0.26 mmol) in methanol-chloroform (1:1, 6.0 mL), a 1.0 M aqueous solution of methanesulfonic acid (0.51 mL, 0.51 mmol) was added, and the reaction mixture was concentrated under reduced pressure. Ethanol (5.0 mL) was added to the residue, and the resulting mixture was concentrated under reduced pressure. The residue was recrystallized from methanol-methylene chloride-diethyl ether, whereby the title compound was obtained as yellow crystalline powder (melting point: 239.0-242.0°C) (92.0 mg, yield: 45% based on N,N'-diethyl-1,3-propanediamine).
¹H-NMR (DMSO-d₆, 120°C) (ammonium salt NH⁺ protons were not observed) δ: 1.14-1.21(m, 6H), 1.88-1.97(m, 2H), 2.43(s, 6H), 3.44-3.54(m, 8H), 3.76(s, 6H), 3.86(s, 12H), 7.16(s, 4H), 7.48-7.54(m, 2H), 7.83-7.90(m, 2H), 8.08-8.14 (m, 2H).

### Example 39

### N,N'-Bis[2-(3,4,5-trimethoxyphenyl)-5-pyridyl]-N,N',2,2-tetramethyl-1,3-propanediamine dimethane-sulfonate

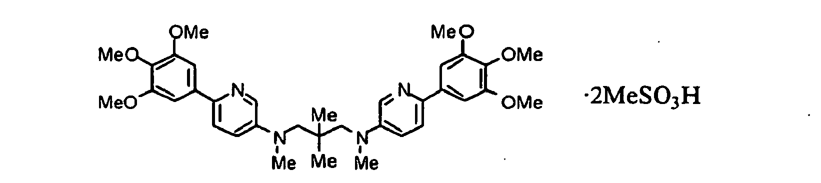

Following the procedures of Example 7, N,N'-bis[2-(3,4,5-trimethoxyphenyl)-5-pyridyl]-N,N',2,2-tetramethyl-1,3-propanediamine was obtained as a pale yellow oil (210.0 mg, yield: 68%) from 5-chloro-2-(3,4,5-trimethoxypheny)pyridine (335.0 mg, 1.20 mmol) synthesized in Referential Example 2 and N,N',2,2-tetramethyl-1,3-propanediamine (65.0 mg, 0.500 mmol).

To a solution of N,N'-bis[2-(3,4,5-trimethoxyphenyl)-5-pyridyl]-N,N',2,2-tetramethyl-1,3-propanediamine (210.0 mg, 0.340 mmol) in methanol-chloroform (1:1, 6.0 mL), a 1.0 M aqueous solution of methanesulfonic acid (0.68 mL, 0.68 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (melting point: 123.0-128.0°C)(161.0 mg, yield: 59%).
¹H-NMR (DMSO-d₆, 120°C) (ammonium salt NH⁺ protons were not observed) δ: 1.08(s, 6H), 2.44(s, 6H), 3.15(s, 6H), 3.49 (s, 4H), 3.76(s, 6H), 3.87(s, 12H), 7.18 (s, 4H), 7.65(dd, J=3.1, 9.2Hz, 2H), 7.90(d, J=9.2Hz, 2H), 8.19(d, J=3.1Hz, 2H).

### Example 40

### 2-Benzyloxy-N,N'-bis[2-(3,4,5-trimethoxyphenyl)-5-pyridyl]-N,N'-dimethyl-1,3-propanediamine

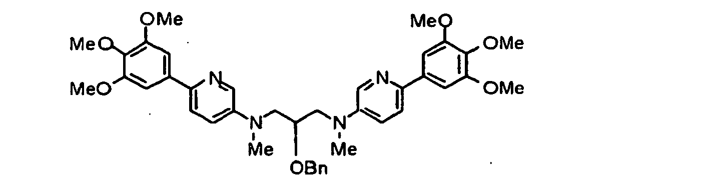

Following the procedures of Example 7, the title compound was obtained as pale yellow amorphous powder (69.0 mg, yield: 59%) from 5-chloro-2-(3,4,5-trimethoxypheny)pyridine (113.0 mg, 0.400 mmol) synthesized in Referential Example 2 and 2-benzyloxy-N,N'-dimethyl-1,3-propanediamine (35.0 mg, 0.160 mmol) synthesized in Referential Example 10.
¹H-NMR (CDCl₃) δ: 3.04(s, 6H), 3.49-3.51(m, 4H), 3.88(s, 6H), 3.93(s, 12H), 4.03-4.11(m, 1H), 4.52(s, 2H), 7.00(dd, J=3.1, 8.7Hz, 2H), 7.11-7.16(m, 2H), 7.16(s, 4H), 7.23-7.26(m, 3H), 7.49(d, J=8.7Hz, 2H), 8.21(d, J=3.1Hz, 2H).

### Example 41

### 2-Hydroxy-N,N'-bis[2-(3,4,5-trimethoxyphenyl)-5-pyridyl]-N,N'-dimethyl-1,3-propanediamine

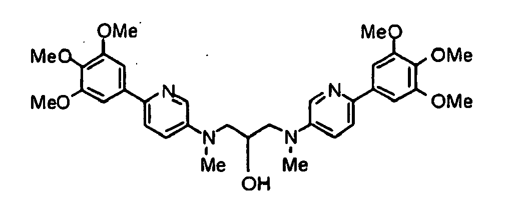

To a solution of 2-benzyloxy-N,N'-bis[2-(3,4,5-trimethoxyphenyl)-5-pyridyl]-N,N'-dimethyl-1,3-propane-diamine (40.0 mg, 0.050 mmol), which had been synthesized in Example 40, in methanol (5.0 mL), 1.0 M hydrochloric acid (2.5 mL, 2.5 mmol) and 10% palladium on charcoal (25.0 mg) were added, and the resulting mixture was stirred at 45°C for 15 hours under a hydrogen gas atmosphere. Insoluble matter was filtered off using Celite. A saturated aqueous solution of sodium hydrogencarbonate was added to the filtrate to basify the same, and the resulting mixture was extracted with chloroform. The organic layer was washed with water, dried over anhydrous sodium sulfate, and then concentrated under reduced pressure. The residue was purified by chromatography on a silica gel column, whereby the title compound was obtained as pale yellow amorphous powder (27.0 mg, yield: 89%).
¹H-NMR (CDCl₃) (alcoholic OH protons were not observed) δ: 3.08(s, 6H), 3.40-3.55(m, 4H), 3.88(s, 6H), 3.94(s, 12H), 4.26-4.35(m, 1H), 7.12(dd, J=2.9, 9.0Hz, 2H), 7.14(s, 4H), 7.55(d, J=9.0Hz, 2H), 8.25(d, J=2.9Hz, 2H).

### Example 42

### N,N-Bis[N-[2-(3,4,5-trimethoxyphenyl)-5-pyridyl]-N-methyl-2-aminoethyl]methylamine trihydrochloride

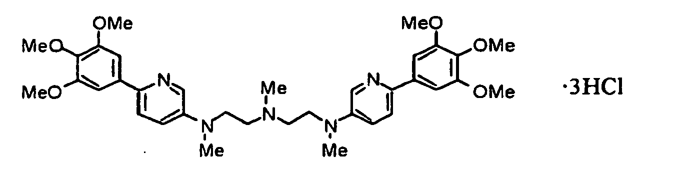

To a solution of methyliminodiacetic acid (74.0 mg, 0.503 mmol) in methylene chloride (2.5 mL), dimethylformamide (25 mg, 0.34 mmol) and oxalyl chloride (0.22 mL, 2.3 mmol) were added. The reaction mixture was stirred at room temperature for 2 hours and then concentrated under reduced pressure. To a solution of the residue in methylene chloride (5 mL), N,N-diisopropylethylamine (0.90 mL, 5.2 mmol) and 5-amino-2-(3,4,5-trimethoxyphenyl)pyridine (262 mg, 1.00 mmol), which had been synthesized in Referential Example 11, were added under ice cooling. After stirred under ice cooling for 2 hours, a saturated aqueous solution of sodium hydrogencarbonate was added, and the resulting mixture was extracted with chloroform. The organic layer was dried over anhydrous sodium sulfate and then concentrated under reduced pressure. The residue was purified by chromatography on a silica gel column, whereby N,N-bis[N-[2-(3,4,5-trimethoxyphenyl)-5-pyridyl]carbamoylmethyl]methylamine was obtained as a colorless oil (94.5 mg, yield: 30%).

Under a nitrogen gas atmosphere, lithium aluminum hydride (23.1 mg, 0.609 mmol) was added to a solution of N,N-bis[N-[2-(3,4,5-trimethoxyphenyl)-5-pyridyl]carbamoylmethyl]-methylamine (88.1 mg, 0.139 mmol) in anhydrous tetrahydrofuran (5.0 mL), and the resulting mixture was stirred at 65°C for 1 hour. Methanol (0.2 mL, 4.9 mmol) was added to the reaction mixture under ice cooling, and an ice bath was then removed. Water (0.2 mL), diethyl ether (20 mL) and anhydrous magnesium sulfate (1.5 g) were added, and the thus-obtained mixture was stirred at room temperature for 2 hours. Insoluble matter was filtered off using Celite, and the filtrate was concentrated under reduced pressure. The residue was purified by chromatography on a silica gel column, whereby N,N-bis[N-[2-(3,4,5-trimethoxyphenyl)-5-pyridyl]-2-aminoethyl]-methylamine was obtained as a colorless oil (58.2 mg, yield: 69%).

To a solution of N,N-bis[N-[2-(3,4,5-trimethoxy-phenyl)-5-pyridyl]-2-aminoethyl]methylamine (58.2 mg, 0.0964 mmol) in tetrahydrofuran (1.0 mL), paraformaldehyde (67.4mg, 2.24 mmol) and sodium borohydride (46.4 mg, 1.23 mmol) were added under ice cooling, followed by the gradual addition of trifluoroacetic acid (1.0 mL) over about 15 minutes. The ice bath was removed, and the mixture was stirred at room temperature for 16 hours. Paraformaldehyde (33.2 mg, 1.11 mmol) and sodium borohydride (24.0 mg, 0.634 mmol) were added in several portions, and the resulting mixture was stirred at room temperature for further 6 hours. Under ice cooling, a 2.5 M aqueous solution of sodium hydroxide (5.OmL) was added to the reaction mixture. Themixture was extracted with chloroform, and the organic layer was dried over anhydrous sodium sulfate and then concentrated under reduced pressure. The residue was purified by preparative thin-layer chromatography on silica gel, whereby N,N-bis[N-[2-(3,4,5-trimethoxyphenyl)-5-pyridyl]-N-methyl-2-aminoethyl]methyl-amine was obtained as a colorless oil (37.2 mg, yield: 61%).

To a solution of N,N-bis[N-[2-(3,4,5-trimethoxy-phenyl)-5-pyridyl]-N-methyl-2-aminoethyl]methyl-amine (42.0 mg, 0.059 mmol) in ethanol (10 mL), 1.0 M hydrochloric acid (0.30 mL, 0.30 mmol) was added, and the reaction mixture was concentrated under reduced pressure. Ethanol (10mL) was added to the residue, and the resultingmixture was concentrated under reduced pressure. The residue was recrystallized from ethanol-diethyl ether, whereby the title compound was obtained as yellow crystalline powder [melting point: 187.0°C (decomposed)] (37.1 mg, yield: 75%).
¹H-NMR (CDCl₃) (ammonium salt NH⁺ protons were not observed) δ: 3.07(br s, 3H), 3.19(br s, 6H), 3.49-3.70(m, 4H), 3.87(br s, 6H), 3.96(br s, 12H), 4.03-4.25(m, 4H), 7.17(br s, 4H), 7.83-7.97(m, 2H), 7.94-8.08(m, 2H), 8.16-8.30 (m, 2H).

### Example 43

### N,N-Bis[N-[2-(3,4,5-trimethoxyphenyl)-5-pyridyl]-N-methyl-3-aminopropyl]methylamine

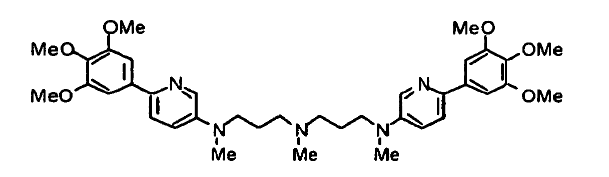

Following the procedures of Example 7, the title compound was obtained as pale yellow amorphous powder (94.0 mg, yield: 28%) from 5-chloro-2-(3,4,5-trimethoxypheny)pyridine (335.0 mg, 1.20 mmol) synthesized in Referential Example 2 and N,N-bis(N-methyl-3-aminopropyl)methylamine (87.0 mg, 0.500 mmol).
¹H-NMR (CDCl₃) δ: 1.75-1.85(m, 4H), 2.23(s, 3H), 2.35-2.45(m, 4H), 2.99(s, 6H), 3.39-3.48(m, 4H), 3.87(s, 6H), 3.93(s, 12H), 7.04(dd, J=2.9, 8.7Hz, 2H), 7.14(s, 4H), 7.54(d, J=8.7Hz, 2H), 8.20(d, J=2.9Hz, 2H).

### Example 44

### Bis[N-[2-(3,4,5-trimethoxyphenyl)-5-pyridyl]-2-aminoethyl]ether dimethanesulfonate

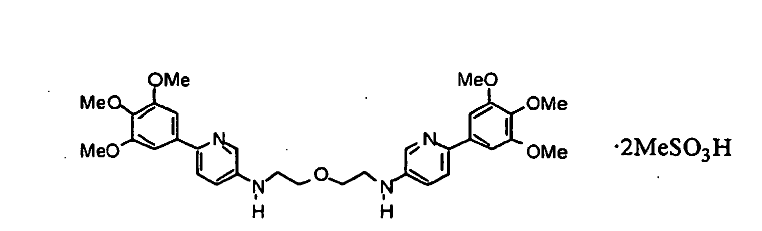

To a solution of 5-amino-2-(3,4,5-trimethoxyphenyl) -pyridine (203.6 mg, 0.783 mmol), which had been synthesized in Referential Example 11, in methylene chloride (4 mL), N,N-diisopropylamine (0.20 mL, 1.1 mmol) and diglycolyl chloride (75.8 mg, 0.443 mmol) were added under ice cooling. After stirred under ice cooling for 30 minutes, water was added to the reaction mixture. The resulting solution was extracted with methanol-chloroform (1:20). The organic layer was dried over anhydrous sodium sulfate and then concentrated under reduced pressure. The residue was purified by chromatography on a silica gel column, whereby bis[N-[2-(3,4,5-trimethoxy-phenyl)-5-pyridyl] carbamoylmethyl]ether was obtained as a colorless oil (224.3 mg, yield: 93%).

Under a nitrogen gas atmosphere, lithium aluminum hydride (33.2 mg, 0.875 mmol) was added to a solution of bis[N-[2-(3,4,5-trimethoxyphenyl)-5-pyridyl]carbamoylmethyl]ether (121.3 mg, 0.196 mmol) in anhydrous tetrahydrofuran (6 mL), and the resulting mixture was stirred at 65°C for 2 hours. Methanol (0.2 mL, 4.9 mmol) was added to the reaction mixture under ice cooling, and an ice bath was then removed. Water (0.2 mL), diethyl ether (20 mL) and anhydrous magnesium sulfate (1.5 g) were added, and the thus-obtained mixture was stirred at room temperature for 1 hour. Insoluble matter was filtered off using Celite, and the filtrate was concentrated under reduced pressure. The residue was purified by chromatography on a silica gel column, whereby bis[N-[2-(3,4,5-trimethoxy-phenyl)-5-pyridyl]-2-aminoethyl] ether was obtained as a colorless oil (98.2 mg, yield: 85%).

To a solution of bis[N-[2-(3,4,5-trimethoxyphenyl)-5-pyridyl]-2-aminoethyl] ether (53.1 mg, 0.0900 mmol) in ethanol (5.0 mL), a 1.0 M aqueous solution of methanesulfonic acid (0.20 mL, 0.20 mmol) was added, and the reaction mixture was concentrated under reduced pressure. Ethanol (10 mL) was added to the residue, and the thus-obtained mixture was concentrated under reduced pressure. The residue was suspended in ethanol-diethyl ether and the resultingprecipitate was collected by filtration, whereby the title compound was obtained as pale brown amorphous powder (49.9 mg, yield: 71%).
¹H-NMR (CDCl₃) (ammonium salt NH⁺ protons were not observed) δ: 2.90(s, 6H), 3.38(br t, J=4.4Hz, 4H), 3.77(br t, J=4.4Hz, 4H), 3.89(s, 6H), 3.93(s, 12H), 6.93(s, 4H), 7.44(d, J=9.1Hz, 2H), 7.73(br d, J=9.1Hz, 2H), 8.38(br s, 2H).

### Example 45

### Bis[N-[2-(3,4,5-trimethoxyphenyl)-5-pyridyl]-N-methyl-2-aminoethyl]ether dimethanesulfonate

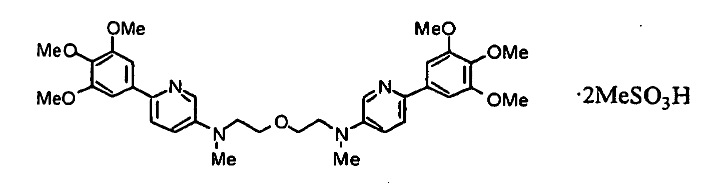

To a solution of bis[N-[2-(3,4,5-trimethoxyphenyl)-5-pyridyl]-2-aminoethyl] ether (23.6 mg, 0.0400 mmol) in tetrahydrofuran (0.5 mL), paraformaldehyde (25.9 mg, 0.862 mmol) and sodium borohydride (18.0 mg, 0.476 mmol) were added under ice cooling. After a solution of trifluoroacetic acid (0.25 mL) in tetrahydrofuran (0.50 mL) was gradually added over about 10 minutes, an ice bath was removed and the mixture was stirred at room temperature for 30 minutes. Paraformaldehyde (25.0 mg, 0.833 mmol) and sodium borohydride (17.8 mg, 0.47 mmol) were added in several portions, and the resulting mixture was stirred for 20 hours. Under ice cooling, a 2.5 M aqueous solution of sodium hydroxide (5.0 mL) was added to the reaction mixture, and the mixture was extracted with chloroform. The organic layer was dried over anhydrous sodium sulfate and then concentrated under reduced pressure. The residue was purified by preparative thin-layer chromatography on silica gel, whereby bis[N-[2-(3,4,5-trimethoxyphenyl)-5-pyridyl]-N-methyl-2-aminoethyl] ether was obtained as a colorless oil (18.8 mg, yield: 76%).

To a solution of bis[N-[2-(3,4,5-trimethoxyphenyl)-5-pyridyl]-N-methyl-2-aminoethyl]ether (47.5 mg, 0.0769 mmol) in ethanol (5.0 mL), a 1.0 M aqueous solution of methanesulfonic acid (0.17 mL, 0.17 mmol) was added, and the reaction mixture was concentrated under reduced pressure. Ethanol (10 mL) was added to the residue, and the resulting mixture was concentrated under reduced pressure. The residue was recrystallized from ethanol-diethyl ether, whereby the title compound was obtained as yellow crystalline powder (melting point: 165.5-169.0°C) (38.8 mg, yield: 62%).
¹H-NMR (CDCl₃) (ammonium salt NH⁺ protons were not observed) δ: 2.89(s, 6H), 3.07(s, 6H), 3.63(br t, J=4.9Hz, 4H), 3.77(br t, J=4.9Hz, 4H), 3.88(s, 6H), 3.94(s, 12H), 6.98(s, 4H), 7.74(br dd, J=2.9, 9.3Hz, 2H), 7.87(br d, J=9.3Hz, 2H), 8.40(br s, 2H).

### Example 46

### N,N'-Bis[2-(3,4,5-trimethoxyphenyl)-5-pyridyl]-N,N'-dimethyl-3-(1-pyrrolidinyl)-1,5-pentanediamine trihydrochloride

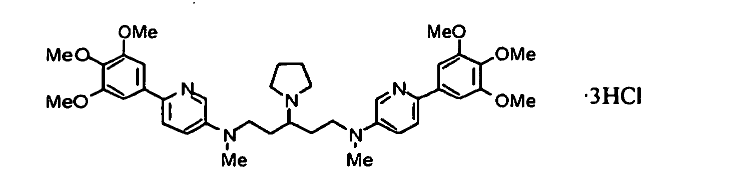

Following the procedures of Example 7, N,N'-bis[2-(3,4,5-trimethoxyphenyl)-5-pyridyl]-N,N'-dimethyl-3-(1-pyrrolidinyl)-1,5-pentanediamine was obtained as a colorless oil (49.6 mg, yield: 27%) from 2-(3,4,5-trimethoxypheny)-5-chloropyridine (182.0 mg, 0.635 mmol) synthesized in Referential Example 2 and N,N'-dimethyl-3-(1-pyrrolidinyl)-1,5-pentanediamine (54.0 mg, 0.217 mmol).

To a solution of N,N'-bis[2-(3,4,5-trimethoxyphenyl)-5-pyridyl]-N,N'-dimethyl-3-(1-pyrrolidinyl)-1,5-pentanediamine (49.6 mg, 0.0724 mmol) in ethanol (5.0mL), 1.0 M hydrochloric acid (0.40 mL, 0.40 mmol) was added, and the reaction mixture was concentrated under reduced pressure. Ethanol (10 mL) was added to the residue, and the resulting mixture was concentrated under reduced pressure, whereby the title compound was obtained as pale yellow amorphous powder (46.0 mg, yield: 71%).
¹H-NMR (CDCl₃) (ammonium salt NH⁺ protons were not observed) δ: 2.00-2.35(m, 8H), 3.10-3.25(m, 2H), 3.17(br s, 6H), 3.65-3.80(m, 3H), 3.82-3.98(m, 4H), 3.90(s, 6H), 3.99(s, 12H), 7.24(s, 4H), 7.79(br d, J=8.8Hz, 2H), 7.90(br d, J=8.8Hz, 2H), 8.20(br s, 2H).

### Example 47

### 2-Dimethylaminomethyl-1,4-bis[2-(3,4, 5-trimethoxyphenyl)-5-pyridyl]piperazine trihydrochloride

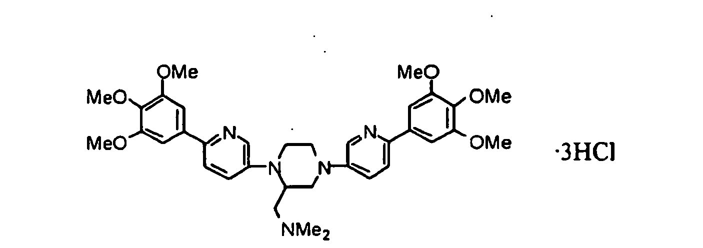

Following the procedures of Example 7, 2-dimethyl-aminomethyl-1,4-bis[2-(3,4,5-trimethoxyphenyl)-5-pyridyl]-piperazine was obtained as a colorless oil (32.3 mg, yield: 20%) from 2-(3,4,5-trimethoxypheny)-5-chloropyridine (198.9 mg, 0.712 mmol) synthesized in Referential Example 2 and 2-(N,N'-dimethylaminomethyl)piperazine (37.0 mg, 0.259 mmol).

To a solution of 2-dimethylaminomethyl-1,4-bis[2-(3,4,5-trimethoxyphenyl)-5-pyridyl]piperazine (32.3 mg, 0.0512 mmol) in ethanol (5.0 mL), 1.0 M hydrochloric acid (0.20 mL, 0.20 mmol) was added, and the reaction mixture was concentrated under reduced pressure. Ethanol (10 mL) was added to the residue, and the resulting mixture was concentrated under reduced pressure. The residue was recrystallized from ethanol-diethyl ether, whereby the title compound was obtained as pale yellow crystalline powder [melting point: 199.5°C (decomposed)] (25.7 mg, yield: 68%).
¹H-NMR (CDCl₃) (data of the free base of the title compound) δ: 2.21(br d, J=11.5Hz, 1H), 2.24-2.33(m, 1H), 2.27(s, 6H), 2.95(dd, J=11.5, 11.5Hz, 1H), 3.14(ddd, J=3.5, 11.5, 11.5Hz, 1H), 3.18(dd, J=3.5, 11.5Hz, 1H), 3.34(ddd, J=3.5, 11.5, 11.5Hz, 1H), 3.57(br ddd, J=3.5, 3.5, 11.5Hz, 1H), 3.74(br d, J=11.5 Hz, 1H), 3.90(s, 6H), 3.97(s, 12H), 4.11(br d, J=11.5Hz, 1H), 7.19(s, 2H), 7.19(s, 2H), 7.26(dd, J=2.9, 8.8Hz, 1H), 7.33(dd, J=2.9, 8.8Hz, 1H), 7.61(d, J=8.8Hz, 1H), 7.62(d, J=8.8Hz, 1H), 8.38(d, J=2.9Hz, 1H), 8.43(d, J=2.9Hz, 1H).

### Example 48

### N,N'-Bis[2-(3,4,5-trimethoxyphenyl)-5-pyridyl]-4,4'-bipiperidine dimethanesulfonate

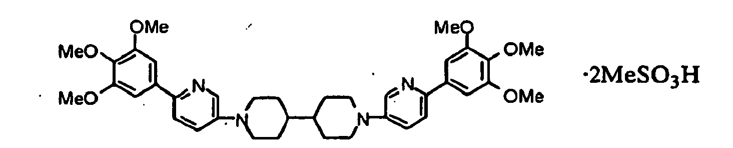

Following the procedures of Example 7, N,N'-bis[2-(3,4,5-trimethoxyphenyl)-5-pyridyl]-4,4'-bipiperidine was obtained as pale yellow crystalline powder (228.0 mg, yield: 69%) from 5-chloro-2-(3,4,5-trimethoxypheny)pyridine (335.0 mg, 1.20 mmol) synthesized in Referential Example 2 and 4,4'-bipiperidine (84.0 mg, 0.500 mmol).

To a solution of N,N'-bis[2-(3,4,5-trimethoxyphenyl)-5-pyridyl]-4,4'-bipiperidine (228.0 mg, 0.340 mmol) in methanol-chloroform(1:1, 6.0 mL), a 1.0 M aqueous solution of methanesulfonic acid (0.70 mL, 0.70 mmol) was added, and the reaction mixture was concentrated under reduced pressure. Ethanol (5.0 mL) was added to the residue, and the resulting mixture was concentrated under reduced pressure. The residue was recrystallized from methanol-methylene chloride-diethyl ether, whereby the title compound was obtained as yellow needles (melting point: 226.0-228.0°C) (170.0 mg, yield: 59%).
¹H-NMR (DMSO-d₆, 120°C) (ammonium salt NH⁺ protons were not observed) δ: 1.35-1.50(m, 6H), 1.81-1.92(m, 4H), 2.44(s, 6H), 2.81-2.97(m, 4H), 3.77(s, 6H), 3.88(s, 12H), 3.90-3.92(m, 4H), 7.20(s, 4H), 7.73(dd, J=2.9, 9.0Hz, 2H), 7.93(d, J=9.0Hz, 2H), 8.25(d, J=2.9Hz, 2H).

### Example 49

### N,N'-Bis[2-(3,4,5-trimethoxyphenyl)-N,N'-dimethyl-1,6-hexanediamine dimethanesulfonate

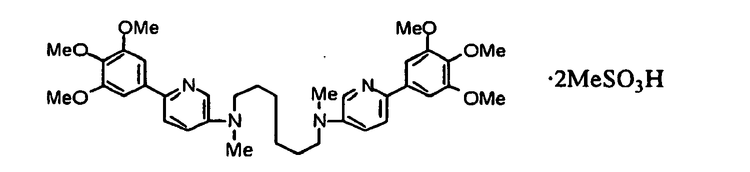

Following the procedures of Example 7, N,N'-bis[2-(3,4,5-trimethoxyphenyl)-5-pyridyl]-N,N'-dimethyl-1,6-hexanediamine was obtained as pale yellow crystalline powder (243.0 mg, yield: 75%) from 5-chloro-2-(3,4,5-trimethoxy-pheny)pyridine (335.0 mg, 1.20 mmol) synthesized in Referential Example 2 and N,N'-dimethyl-1,6-hexanediamine (72.0 mg, 0.500 mmol).

To a solution of N,N'-bis[2-(3,4,5-trimethoxyphenyl)-5-pyridyl]-N,N'-dimethyl-1,6-hexanediamine (243.0 mg, 0.370 mmol) in methanol-methylene chloride (6:1, 3.5 mL), a 1.0 M aqueous solution of methanesulfonic acid (0.76 mL, 0.76 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow needles (melting point: 192.0-195.0°C) (251.0 mg, yield: 81%).
¹H-NMR (DMSO-d₆, 120°C) (ammonium salt NH⁺ protons were not obs erved) δ: 1.37-1.45(m, 4H), 1.56-1.67(m, 4H), 2.42(s, 6H), 3.02(s, 6H), 3.40-3.47(m, 4H), 3.76(s, 6H), 3.87(s, 12H), 7.17(s, 4H), 7.50(dd, J=3.1, 9.0Hz, 2H), 7.89 (d, J=9.0Hz, 2H), 8.04(d, J=3.1Hz, 2H).

### Example 50

### N,N'-Bis[2-(3,5-dimethoxyphenyl)-5-pyridyl]-N,N'-diethylethylenediamine dimethanesulfonate

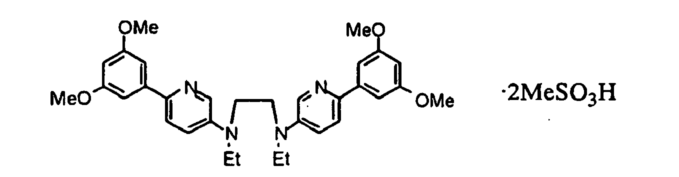

Following the procedures of Example 1, N,N'-bis[2-(3,5-dimethoxyphenyl)-5-pyridyl]-N,N'-diethylethylene-diamine was obtained as a colorless oil (55.0 mg, yield: 42%) from N,N'-bis(2-trifluoromethanesulfonyloxy-5-pyridyl)-N,N'-diethylethylenediamine (136.0 mg, 0.240 mmol), which had been synthesized by similar procedures as in Referential Example 1, and 3,5-dimethoxyphenylboric acid (80.0 mg, 0.530 mmol).

To a solution of N,N'-bis[2-(3,5-dimethoxyphenyl)-5-pyridyl]-N,N'-diethylethylenediamine (55.0 mg, 0.100 mmol) in methanol (2.0 mL), a 1.0 M aqueous solution of methanesulfonic acid (0.20 mL, 0.20 mmol) was added, and the reaction mixture was concentrated under reduced pressure. Ethanol (5.0mL) was added to the residue, and the resulting mixture was concentrated under reduced pressure. The residue was recrystallized from methanol-diethyl ether, whereby the title compound was obtained as yellow crystalline powder (melting point: 280.0-282.0°C) (48.0 mg, yield: 64%).
¹H-NMR [CD₃OD-CDCl₃, (1:10)] (ammonium salt NH⁺ protons were not observed) δ: 1.26(t, J=6.8Hz, 6H), 2.83(s, 6H), 3.64(q, J=6.8Hz, 4H), 3.77(s, 4H), 3.89(s, 12H), 6.60(t, J=2.2Hz, 2H), 6.93(d, J=2.2Hz, 4H), 7.75(dd, J=2.9, 10.3Hz, 2H), 7.92(d, J=10.3Hz, 2H), 8.45(d, J=2.9Hz, 2H).

### Example 51

### N,N'-Diethyl-N,N'-bis[2-(4-isopropoxy-3,5-dimethoxyphenyl)-5-pyridyl]ethylenediamine dimethanesulfonate

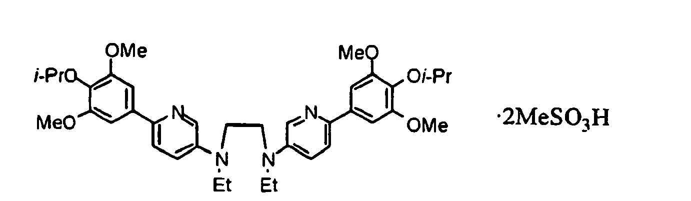

Following the procedures of Example 1, N,N'-diethyl-N,N'-bis[2-(4-isopropoxy-3,5-dimethoxyphenyl)-5-pyridyl]-ethylenediamine was obtained as a colorless oil (129.0 mg, yield: 98%) from N,N'-bis(2-trifluoromethanesulfonyloxy-5-pyridyl)-N,N'-diethylethylenediamine (113.0 mg, 0.200 mmol), which had been synthesized by similar procedures as in Referential Example 1, and 4-isopropoxy-3,5-dimethoxy-phenyl-boric acid (106.0 mg, 0.440 mmol).

To a solution of N,N'-diethyl-N,N'-bis[2-(4-isopropoxy-3,5-dimethoxyphenyl)-5-pyridyl]ethylenediamine (129.0 mg, 0.200 mmol) in methanol (2.0 mL), a 1.0 M aqueous solution of methanesulfonic acid (0.40 mL, 0.40 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as yellow crystalline powder (melting point: 260.0-261.0°C) (102.0 mg, yield: 60%).
¹H-NMR (CDCl₃) (ammonium salt NH⁺ protons were not observed) δ: 1.27(t, J=6.8Hz, 6H), 1.32(d, J=6.1Hz, 12H), 2.85(s, 6H), 3.72(s, 4H), 3.76(q, J=6.8Hz, 4H), 3.94(s, 12H), 4.47(qq, J=6.1, 6.1Hz, 2H), 7.07(s, 4H), 7.69(dd, J=2.9, 9.3Hz, 2H), 7.88(d, J=9.3Hz, 2H), 8.72(d, J=2.9Hz, 2H).

### Example 52

### N,N'-Bis[2-(3, 5-dimethoxyphenyl)-5-pyridyl]-N,N'-dimethyl-1,3-propanediamine dimethanesulfonate

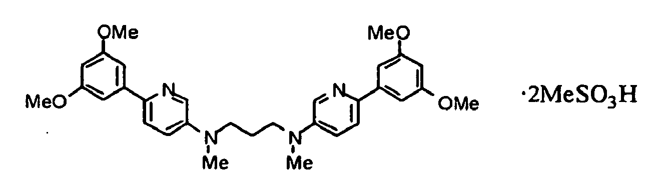

Following the procedures of Example 1, N,N'-bis[2-(3,5-dimethoxyphenyl)-5-pyridyl]-N,N'-dimethyl-1,3-propanediamine was obtained as a colorless oil (35.0 mg, yield: 35%) from N,N'-bis(2-trifluoromethanesulfonyloxy-5-pyridyl)-N,N'-dimethyl-1,3-propanediamine (95.0 mg, 0.190 mmol), which had been synthesized by similar procedures as in Referential Example 1, and 3,5-dimethoxyphenylboric acid (63.0 mg, 0.420 mmol).

To a solution of N,N'-bis[2-(3,5-dimethoxyphenyl)-5-pyridyl]-N,N'-dimethyl-1,3-propanediamine (35.0 mg, 0.070 mmol) in methanol (2.0 mL), a 1.0 M aqueous solution of methanesulfonic acid (0.15 mL, 0.15 mmol) was added, and the reaction mixture was concentrated under reduced pressure. Ethanol (5.0 mL) was added to the residue, and the resulting mixture was concentrated under reduced pressure. The residue was recrystallized from ethanol, whereby the title compound was obtained as yellow crystalline powder (melting point: 228.0-231.0°C) (21.0 mg, yield: 43%).
¹H-NMR (CDCl₃)(ammonium salt NH⁺ protons were not observed) δ: 2.07(tt, J=7.3, 7.3Hz, 2H), 2.90(s, 6H), 3.21(s, 6H), 3.58(t, J=7.3Hz, 4H), 3.85(s, 12H), 6.78(t, J=2.2Hz, 2H), 6.86(d, J=2.2Hz, 4H), 7.81(dd, J=3.2, 9.3Hz, 2H), 7.91(d, J=9.3Hz, 2H), 8.71(d, J=3.2Hz, 2H).

### Example 53

### N,N'-Bis[2-(4-isopropoxy-3,5-dimethoxy-phenyl)-5-pyridyl]-N,N'-dimethyl-1,3-propanediamine dimethane-sulfonate

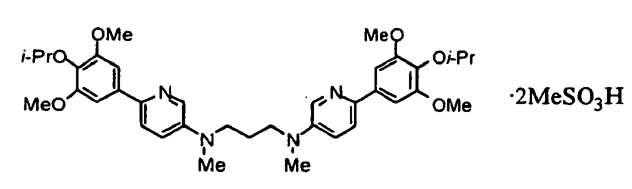

Following the procedures of Example 1, N,N'-bis[2-(4-isopropoxy-3,5-dimethoxyphenyl)-5-pyridyl]-N,N'-dimethyl-1,3-propanediamine was obtained as a colorless viscous oil (27.0 mg, yield: 33%) from N,N'-bis(2-trifluoromethane-sulfonyloxy-5-pyridyl)-N,N'-dimethyl-1,3-propanediamine (61.0 mg, 0.125 mmol), which had been synthesized by similar procedures as in Referential Example 1, and 4-isopropoxy-3,5-dimethoxyphenylboric acid (66.0 mg, 0.275 mmol).

To a solution of N,N'-bis[2-(4-isopropoxy-3,5-dimethoxyphenyl)-5-pyridyl]-N,N'-dimethyl-1,3-propane-diamine (27.0 mg, 0.040 mmol) in methanol (2.0 mL), a 1.0 M aqueous solution of methanesulfonic acid (0.10 mL, 0.10 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained asyellowcrystallinepowder (meltingpoint: 191.0-192.0°C) (15.0 mg, yield: 42%).
¹H-NMR (CDCl₃) (ammonium salt NH⁺ protons were not observed) δ: 1.31(d, J=6.1Hz, 12H), 1.96(tt, J=7.6, 7.6Hz, 2H), 3.01(s, 6H), 3.45(t, J=7.6 Hz, 4H), 3.91(s, 12H), 4.40(qq, J=6.1Hz, 2H), 7.14(s, 4H), 7.48(dd, J=2.9, 7.8Hz, 2H), 7.54(d, J=7.8Hz, 2H), 8.20(d, J=2.9Hz, 2H).

### Example 54

### N,N'-Bis[2-(4-hydroxy-3,5-dimethoxyphenyl)-5-pyridyl]-N,N'-dimethyl-1,3-propanediamine dimethanesulfonate

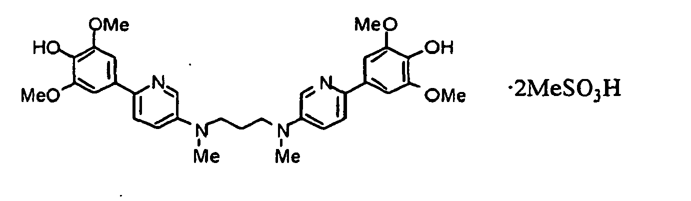

Following the procedures of Example 1, N,N'-bis[2-[4-(4-methoxybenzyloxy)-3,5-dimethoxyphenyl]-5-pyridyl]-N,N'-dimethyl-1,3-propanediamine was obtained as a pale brown oil (75.0 mg, yield: 23%) from N,N'-bis(2-trifluoromethane-sulfonyloxy-5-pyridyl)-N,N'-dimethyl-1,3-propanediamine (220.0 mg, 0.400 mmol) synthesized by similar procedures as in Referential Example and 4-(4-methoxybenzyloxy)-3,5-dimethoxyphenylboric acid (308.0 mg, 0.970 mmol) synthesized by similar procedures as in Referential Example 3.

To a solution of N,N'-bis[2-[4-(4-methoxybenzyloxy)-3,5-dimethoxyphenyl]-5-pyridyl]-N,N'-dimethyl-1,3-propane-diamine (72.0 mg, 0.089 mmol) in methylene chloride (1.0 mL), trifluoroacetic acid (1. 0 mL) was added. The reaction mixture was stirred at room temperature for 30 minutes, and then concentrated under reduced pressure. A solution of the residue in chloroform was washed with a saturated aqueous solution of sodium hydrogencarbonate and brine, dried over anhydrous sodium sulfate, and then concentrated under reduced pressure. The residue was purified by chromatography on a silica gel column, whereby N,N'-bis[2-(4-hydroxy-3,5-dimethoxyphenyl)-5-pyridyl]-N,N'-dimethyl-1, 3-propanediamine was obtained as a pale yellow oil (43.0 mg, yield: 86%).

To a solution of N,N'-bis[2-(4-hydroxy-3,5-dimethoxyphenyl) -5-pyridyl]-N,N'-dimethyl-1,3-propane-diamine (43.0 mg, 0.076 mmol) in methanol-chloroform (1:2, 3.0 mL), a 1.0 M aqueous solution of methanesulfonic acid (0.16 mL, 0.16 mmol) was added, and the reaction mixture was concentrated under reduced pressure. Ethanol (5.0 mL) was added to the residue, and the resulting mixture was concentrated under reduced pressure. The residue was recrystallized from ethanol-diethyl ether, whereby the title compound was obtained as yellow crystalline powder (melting point: 198.0-202.0°C)(17.0 mg, yield: 30%).
¹H-NMR (DMSO-d₆, 120°C) (neither ammonium salt NH⁺ protons nor phenolic OH protons were observed) δ: 1.93(tt, J=7.0, 7.0Hz, 2H), 2.41(s, 6H), 3.05(s, 6H), 3.52(t, J=7.0Hz, 4H), 3.85(s, 12H), 7.15(s, 4H), 7.59(dd, J=2.9, 9.2Hz, 2H), 7.88(d, J=9.2Hz, 2H), 8.09(d, J=2.9Hz, 2H).

### Example 55

### N,N'-Bis[2-(3-methoxy-4,5-methylenedioxyphenyl)-5-pyridyl]-N,N'-dimethyl-1,3-propanediamine dimethane-sulfonate

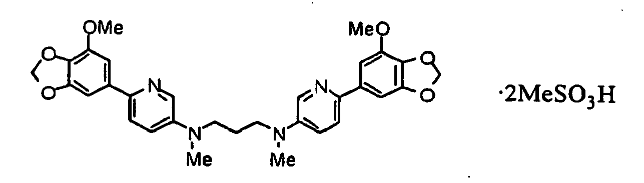

Following the procedures of Example 1, N,N'-bis[2-(3-methoxy-4,5-methylenedioxyphenyl)-5-pyridyl]-N,N'-dimethyl-1,3-propanediamine was obtained as a colorless oil (46.0 mg, yield: 52%) from N,N'-dimethyl-N,N'-bis(2-trifluoromethane-sulfonyloxy-5-pyridyl)-1,3-propanediamine (78.0 mg, 0.160 mmol), which had been synthesized by similar procedures as in Referential Example 1, and 3-methoxy-4,5-methylenedioxy-phenylboric acid (66.0 mg, 0.340 mmol).

To a solution of N,N'-bis[2-(3-methoxy-4,5-methylene-dioxyphenyl)-5-pyridyl]-N,N'-dimethyl-1,3-propanediamine (46.0 mg, 0.080 mmol) in methanol (2.0 mL), a 1.0 M aqueous solution of methanesulfonic acid (0.20 mL, 0.20 mmol) was added, and the reaction mixture was concentrated under reduced pressure. Ethanol (5.0 mL) was added to the residue, and the resulting mixture was concentrated under reduced pressure. Diethyl ether was added to a solution of the residue in methanol and insoluble matter was then filtered off, whereby the title compound was obtained as yellow amorphous powder (29.0 mg, yield: 46%).
¹H-NMR [CD₃OD-CDCl₃ (1:10)] (ammonium salt NH⁺ protons were not observed) δ: 2.04(tt, J=7.3, 7.3Hz, 2H), 2.86(s, 6H), 3.17(s, 6H), 3.58(t, J=7.3Hz, 4H), 3.99(s, 6H), 6.08(s, 4H), 6.87(d, J=1.7Hz, 2H), 6.97(d, J=1.7Hz, 2H), 7.78(dd, J=3.2, 9.5Hz, 2H), 7.88(d, J=9.5Hz, 2H), 8.38(d, J=3.2Hz, 2H).

### Example 56

### N,N'-Diethyl-N,N'-bis[2-(4-isopropoxy-3,5-dimethoxyphenyl)-5-pyridyl]-1,3-propanediamine dimethanesulfonate

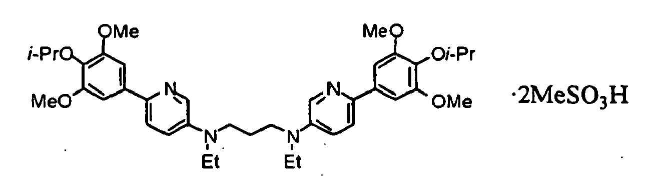

Following the procedures of Example 1, N,N'-diethyl-N,N'-bis[2-(4-isopropoxy-3,5-dimethoxyphenyl)-5-pyridyl]-1,3-propanediamine was obtained as a pale yellow oil (78.0 mg, yield: 58%) from N,N'-diethyl-N,N'-bis(2-trifluoromethane-sulfonyloxy-5-pyridyl)-1,3-propanediamine (116.0 mg, 0.200 mmol), which had been synthesized by similar procedures as in Referential Example 1, and 4-isopropoxy-3,5-dimethoxy-phenylboric acid (96.0 mg, 0.400 mmol).

To a solution of N,N'-diethyl-N,N'-bis[2-(4-isopropoxy-3,5-dimethoxyphenyl)-5-pyridyl]-1,3-propane-diamine (78.0 mg, 0.110 mmol) in methanol-chloroform (2:1, 3.0 mL), a 1.0 M aqueous solution of methanesulfonic acid (0.24 mL, 0.24 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether-hexane, whereby the title compound was obtained as yellow scales (melting point: 173.0-175.0°C) (66.0 mg, yield: 66%).
¹H-NMR (DMSO-d₆, 120°C) (ammonium salt NH⁺ protons were not obs erved) δ: 1.18(t, J=7.0Hz, 6H), 1.23(d, J=6.0Hz, 12H), 1.93(tt, J=7.0, 7.0Hz, 2H), 2.42(s, 6H), 3.47-3.56(m, 4H), 3.50(q, J=7.0Hz, 4H), 3.85(s, 12H), 4.38(qq, J=6.0, 6.0 Hz, 2H), 7.15(s, 4H), 7.56(dd, J=3.1, 9.0Hz, 2H), 7.90(d, J=9.0Hz, 2H), 8.09(d, J=3.1Hz, 2H).

### Example 57

### N,N-Bis[N-[2-(3,5-dimethoxy-4-(2,2,2-trifluoroethoxy)-phenyl)-5-pyridyl]-N-methyl-3-aminopropyl]methylamine trihydrochloride

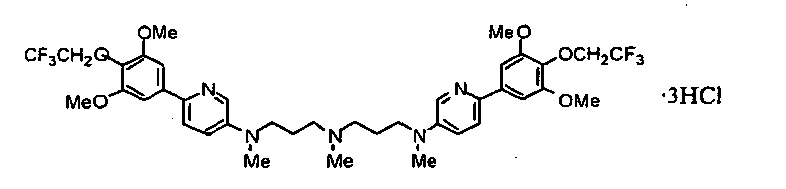

To a solution of 5-amino-2-[3,5-dimethoxy-4-(2,2,2-trifluoroethoxy)phenyl] pyridine (melting point" 176.0-177.5°C) (28.2 g, 85.4 mmol), which had been synthesized by similar procedures as in Referential Example 11, in methylene chloride (550 mL), N,N-diisopropylethylamine (18.0 mL, 103 mmol) was added. To the resulting solution held under stirring and ice cooling, acryloyl chloride (7.00 mL, 86.0 mmol) was added dropwise over about 10 minutes. A saturated aqueous solution of sodium hydrogencarbonate was added, and the resulting mixture was extracted with chloroform. The organic layer was dried over anhydrous sodium sulfate and then concentrated under reduced pressure. Chloroform-hexane (1:2, 300 mL) was added to the residue and the resulting precipitate was collectedby filtration, whereby crude 5-acryloylamino-2-[3,5-dimethoxy-4-(2,2,2-trifluoroethoxy) phenyl]pyridine was obtained as colorless needles (melting point: 207.0-209.0°C) (38.2 g).

To a 30% ethanol solution of methylamine (100 mL, 970 mmol), the crude 5-acryloylamino-2-[3,5-dimethoxy-4-(2,2,2-trifluoroethoxy)phenyl]pyridine (19.2 g, approx. 50.2 mmol) obtained by the above-described procedures was added. After stirred at room temperature for 1 hour, the reaction mixture was concentrated under reduced pressure. Chloroform (100 mL) was added to the residue, and the resulting mixture was again concentrated under reduced pressure. To a solution of the residue in chloroform (100 mL), the crude 5-acryloylamino-2-[3,5-dimethoxy-4-(2,2,2-trifluoroethoxy) phenyl]pyridine (16.0 g, approx. 41.8 mmol) obtained by the above-described procedures was added, and the thus-obtained mixture was stirred at 60°C for 12 hours. The solidified reaction mixture was dissolved in chloroform (600 mL), and the resulting solution was heated at 80°C for 2 hours to distill off the solvent. The residue was again dissolved in chloroform (600 mL) and the solution so obtained was concentrated to about 200 mL. Under stirring at 80°C, hexane (100 mL) was added to the solution. The resulting mixture was allowed to cool down and the precipitated crystals were collected by filtration, whereby N,N-bis[2-[N-[2-[3,5-dimethoxy-4-(2,2,2-trifluoroethoxy)-phenyl]-5-pyridyl]carbamoyl] ethyl] methylamine was obtained as colorless crystalline powder [melting point: 99.0°C (decomposed)][22.67 g, yield: 72% based on 5-amino-2-[3,5-dimethoxy-4-(2,2,2-trifluoroethoxy)phenyl] pyridine].

Under a nitrogen gas atmosphere, lithium aluminum hydride (4.28 g, 113 mmol) was added to an ice-cooled solution of N,N-bis[2-[N-[2-[3,5-dimethoxy-4-(2,2,2-trifluoro-ethoxy) phenyl]-5-pyridyl]carbamoyl]ethyl]methylamine (22.50 g, 28.3 mmol) in anhydrous tetrahydrofuran (500 mL). An ice bath was removed, and the reaction mixture was stirred at 65°C for 1 hour. Under ice cooling, methanol (20 mL, 490 mmol) was added to the reaction mixture, and the ice bath was removed. Water (20 mL), diethyl ether (2 L) and anhydrous magnesium sulfate (130 g) were added, and the thus-obtained mixture was stirred overnight at room temperature. Insoluble matter was filtered off using Celite, and the filtrate was concentrated under reduced pressure. The residue was purified by chromatography on a silica gel column, whereby N,N-bis[N-[2-[3,5-dimethoxy-4-(2,2,2-trifluoroethoxy)phenyl]-5-pyridyl]-3-aminopropyl]methylamine was obtained as a colorless oil (20.32 mg, yield: 94%).

To a solution of N,N-bis[N-[2-[3,5-dimethoxy-4-(2,2,2-trifluoro-ethoxy) phenyl]-5-pyridyl]-3-aminopropyl]-methylamine (2.11 g, 2.75 mmol) in acetonitrile (10 mL), formalin 2.1 mL (28 mmol) and sodium cyanoborohydride (0.55 g, 8.8 mmol) were added. The resulting mixture was added dropwise over about 10 minutes to a solution of acetic acid (0.6 mL) in acetonitrile (0.5 mL) over about 10 minutes, and the resulting mixture was stirred at room temperature for 1 hour. 1.0 M sodium hydroxide (100 mL) was added dropwise, the reaction mixture was extracted with methylene chloride, and the organic layer was concentrated under reduced pressure. A solution of the residue in pyridine (15 mL) was stirred at 45°C for 1 hour, and was then concentrated under reduced pressure. Toluene was added to the residue, and the thus-obtained mixture was concentrated under reduced pressure. The residue was purified by chromatography on a silica gel column, whereby N,N-bis[N-[2-[3,5-dimethoxy-4-(2,2,2-trifluoroethoxy)phenyl]-5-pyridyl]-N-methyl-3-aminopropyl]methylamine was obtained as a colorless oil (984 mg, yield: 45%).

To a solution of N,N-bis[N-[2-[3,5-dimethoxy-4-(2,2,2-trifluoroethoxy)phenyl]-5-pyridyl]-N-methyl-3-aminopropyl]methylamine (3.62 g, 4.55 mmol) in ethanol (50 mL), concentrated hydrochloric acid (1.4 mL, 17 mmol) was added, and the reaction mixture was concentrated under reduced pressure. Ethanol (50mL) was added to the residue, and the resulting mixture was concentrated under reduced pressure. Ethyl acetate (20 mL) was then added to the residue, followed by concentration of the resultant mixture under reduced pressure. The residue was suspended in ethyl acetate and the resulting suspension was filtered, whereby the title compound was obtained as yellow crystalline powder [melting point: 140°C (decomposed)] (3.55 g, yield: 86%).
¹H-NMR (CD₃OD) (ammonium salt NH⁺ protons were not observed) δ: 2.17(br dddd, J=7.3, 7.3, 7.3, 7.3Hz, 4H), 2.94(s, 3H), 3.20(s, 6H), 3.23-3.32(m, 2H), 3.34-3.44(m, 2H), 3.65(dt, J=14.3, 7.3Hz, 2H), 3.70(dt, J=14.3, 7.3Hz, 2H), 3.97 (s, 12H), 4 45 (q, ³J_{HF}=8.8Hz, 4H), 7.15(s, 4H), 8.00(br dd, J=3.0, 9.5Hz, 2H), 8.12(br d, J=3.0Hz, 2H), 8.14 (br d, J=9.5Hz, 2H).

### Example 58

### N,N-Bis[N-[2-(3,5-dimethoxy-4-methylthiophenyl)-5-pyridyl]-N-methyl-3-aminopropyl]methylamine trihydro-chloride

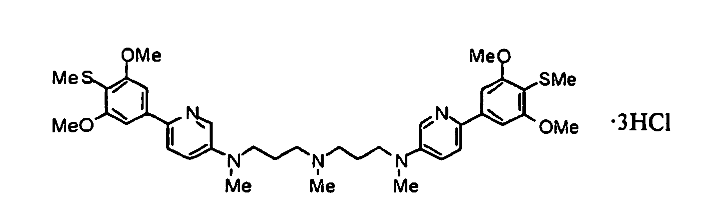

Following the procedures of Example 57, from 5-amino-2-(3,5-dimethoxy-4-methylthiophenyl)pyridine (182.0 mg, 0.660 mmol) synthesized by similar procedures as in Referential Example 11, N,N-bis[2-[N-[2-(3,5-dimethoxy-4-methylthio-phenyl)-5-pyridyl]carbamoyl]ethyl]methylamine was obtained as a colorless viscous oil [136.0 mg, yield: 61% based on 5-amino-2-(3,5-dimethoxy-4-methylthiophenyl)pyridine]. From N,N-bis[2-[N-[2-(3,5-dimethoxy-4-methylthiophenyl)-5-pyridyl]carbamoyl]ethyl]methylamine (671.0 mg, 0.970 mmol), N,N-bis[N-[2-(3,5-dimethoxy-4-methylthiophenyl)-5-pyridyl]-3-aminopropyl]methylamine was obtained as a colorless oil (508.0 mg, yield: 79%). From N,N-bis[N-[2-(3,5-dimethoxy-4-methylthiophenyl)-5-pyridyl]-3-aminopropyl]methylamine (67.0 mg, 0.100 mmol), N,N-bis[N-[2-(3,5-dimethoxy-4-methylthiophenyl)-5-pyridyl]-N-methyl-3-aminopropyl]methyl-amine was obtained as a colorless oil (44.0 mg, yield: 64%). From N,N-bis[N-[2-(3,5-dimethoxy-4-methylthiophenyl)-5-pyridyl]-N-methyl-3-aminopropyl]methylamine (67.0 mg, 0.100 mmol), the title compound was obtained as yellow amorphous powder (42.0 mg, yield: 52%).
¹H-NMR (CDCl₃) (data of the free base of the title compound) δ: 1.74-1.85(m, 4H), 2.23(s, 3H), 2.35-2.44(m, 4H), 2.39(s, 6H), 3.01(s, 6H), 3.45(t, J=7.3Hz, 4H), 3.98(s, 12H), 7.04(dd, J=2.9, 8.8Hz, 2H), 7.15(s, 4H), 7.59(d, J=8.8Hz, 2H), 8.22(d, J=2.9Hz, 2H).

### Test 1 (Evaluation of IgE antibody production inhibiting activity)

From a mouse (Balb/C, male, 8 weeks old), the spleen was excised. The spleen was shredded in 10% FBS/RPMI 1640, and was then disintegrated into single cells through a 70-mesh screen. Those single cells were hemolyzed with the Gey' s solution, and using RPMI 1640 medium/25 M HEPES/0.3% BSA, a spleen cell suspension (1 x 10⁷ cells/mL) was prepared. After an aliquot of the suspension was reacted with a rat anti-mouse Thy-1,2 monoclonal antibody (product of Cedarlane Laboratories Limited) at 4°C for 1 hour, centrifugation was conducted. Precipitated cells were suspended again (1 x 10⁷ cells/mL, RPMI/HEPES/BSA) . After the suspension was next reacted with a low cytotoxic rabbit complement (product of Cedarlane Laboratories Limited) at 37°C for 1 hour, dead cells were removed by specific gravity centrifugation so that a B cell fraction was obtained as viable cells.

Using a 96-well plate, the B cells (2x10⁵ cells/0.2mL/well) was incubated together with LPS (E. coli 026:B6, product of DIFCO Laboratories, Inc.) for 1 day. Mouse IL-4 (product of Genzyme Corp.) was then added, followed by further incubation for 6 days.

The IgE antibody production inhibiting activity of each drug was calculated by adding the drug Day 1 of the incubation and assaying the quantity of IgE in the culture supernatant after the incubation. Inhibition activity (IC₅₀) is presented in Table 1.

Further, the water solubility (%) of each compound was also determined. The results are presented in Table 1.

**Table 1**

| Compound | IC₅₀ (µM) | Water solubility |
|---|---|---|
| (Example No.) | | (%) |
| 7 | 0.04 | 5 |
| 14 | 0.04 | 0.1 |
| 30 | 0.10 | 0.1 |
| 36 | 0.03 | 10 |
| 37 | 0.10 | 10 |
| 39 | 0.08 | 10 |
| 41 | 0.05 | 0.1* |
| 42 | 0.10 | 10 |
| 43 | 0.10 | 10* |
| 44 | 0.10 | 10 |
| 47 | 0.05 | 0.1 |
| 49 | 0.04 | 10 |
| 57 | 0.10 | 1 |

| | | |
|---|---|---|
| * Solubility in 1 M hydrochloric acid | | |

### Industrial Applicability

The bis(2-aryl-5-pyridyl) derivatives (1) according to the present invention and salts thereof have excellent IgE antibody production inhibiting activity and are useful as medicines for the prevention or treatment of allergic immune diseases.

## Claims

1. A bis(2-aryl-5-pyridyl) derivative represented by the following formula (1) or a salt thereof: 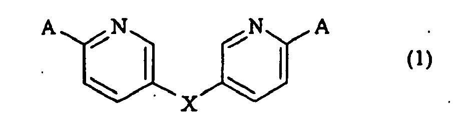 wherein A represents a substituted or unsubstituted aromatic hydrocarbon group, and X represents a group selected from the following formulas (2) to (4): 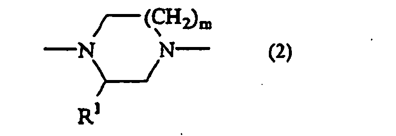 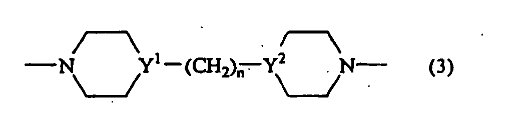 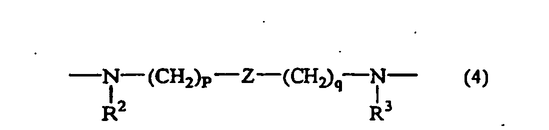 wherein in the formula (2), R¹ represents a hydrogen atom or a di(lower alkyl)amino(lower alkyl) group, and m stands for an integer of 1 or 2; in the formula (3), Y¹ and Y² represent a nitrogen atom or a CH group, and n stands for an integer of 0 to 6; in the formula (4), R² and R³ represent a hydrogen atom or a lower alkyl group, Z represents a single bond, a substituted methylene group, a substituted imino group, an oxygen atom or a cycloalkylene group, and p and q stand for an integer of 0 to 6; and in which the lower alkyl moiety is a linear, branched or cyclic alkyl group having 1 to 6 carbon atoms.

2. A bis(2-aryl-5-pyridyl) derivative or a salt thereof according to claim 1, wherein in the formula (1), A is a substituted or unsubstituted phenyl group.

3. A bis(2-aryl-5-pyridyl) derivative or a salt thereof according to claim 1, wherein in the formula (1), X is a group represented by the formula (4).

4. A bis(2-aryl-5-pyridyl) derivative or a salt thereof according to claim 1, wherein said aromatic hydrocarbon group is substituted by 1 to 3 substituents selected from lower alkyl groups, lower alkoxy groups, halogeno(lower alkoxy) groups, lower alkoxy(lower alkoxy) groups, hydroxy(lower alkoxy) groups, carboxyl groups, (lower alkoxy)carbonyl groups, unsubstituted or (lower alkyl)- and/or (lower alkoxy)-substituted carbamoyl groups, lower alkanoyl groups, formyl groups, lower alkanoyloxy groups, halogen atoms, hydroxy groups, cyano groups, (lower alkyl) thio groups, amino groups, mono- or di-(lower alkyl)amino groups, (lower alkyl)sufonylamino groups, pyrrolidinyl groups, and alkylenedioxy groups; in which the lower alkyl moiety is a linear, branched or cyclic alkyl group having 1 to 6 carbon atoms.

5. A medicine comprising as an active ingredient a bis(2-aryl-5-pyridyl) derivative or a salt thereof according to any one of claims 1-4.

6. A medicine according to claim 5, which is a preventive or therapeutic for an allergic immune disease.

7. A medicine according to claim 5, which is a preventive or therapeutic for asthma, atopic dermatitis, allergic rhinilis, inflammatory colitis, contact skin disease or an allergic eye disease.

8. Use of bis(2-aryl-5-pyridyl)derivative or a salt thereof according to any one of claims 1-4 for the manufacture of a medicine for preventing or treating allergic immune diseases.

9. The use according to claim 8, wherein the medicine is a preventive or therapeutic for asthma, atopic dermatitis, allergic rhinitis, inflammatory colitis, contact skin diseases or allertic eye diseases.

10. An IgE antibody production inhibitor which is a bis(2-aryl-5-pyridyl) derivative or a salt thereof according to any one of claims 1-4.

11. A medicinal composition comprising a bis(2-aryl-5-pyridyl) derivative or a salt thereof according to any one of claims 1-4 and a pharmacologically acceptable carrier.

## Patentansprüche

1. Bis(2-aryl-5-pyridyl)-Derivat der folgenden Formel (1) oder ein Salz davon: 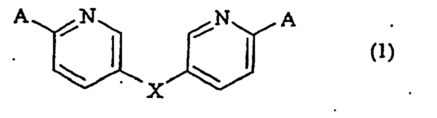 worin A eine substituierte oder unsubstituierte aromatische Kohlenwasserstoffgruppe bedeutet, und X eine Gruppe bedeutet, ausgewählt aus den folgenden Formeln (2) bis (4): 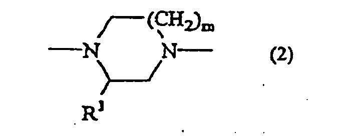 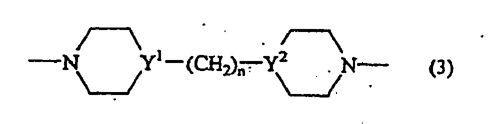 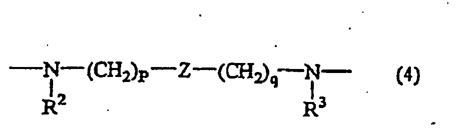 worin in Formel (2) R¹ ein Wasserstoffatom oder eine Di(niederalkyl)amino(niederalkyl)gruppe bedeutet, und m eine ganze Zahl von 1 oder 2 ist; in der Formel (3) Y¹ und Y² ein Stickstoffatom oder eine CH-Gruppe bedeuten, und n für eine ganze Zahl von 0 bis 6 steht; in der Formel (4) R² und R³ ein Wasserstoffatom oder eine Niederalkylgruppe bedeuten, Z eine Einfachbindung, eine substituierte Methylengruppe, eine substituierte Iminogruppe, ein Sauerstoffatom oder eine Cycloalkylengruppe bedeutet, und p und q für eine ganze Zahl von 0 bis 6 stehen; und worin der Niederalkylrest eine lineare, verzweigte oder cyclische Alkylgruppe mit 1 bis 6 Kohlenstoffatomen ist.

2. Bis(2-aryl-5-pyridyl)-Derivat oder ein Salz davon nach Anspruch 1, worin in der Formel (1) A eine substuierte oder unsubstituierte Phenylgruppe ist.

3. Bis(2-aryl-5-pyridyl)-Derivat oder ein Salz davon nach Anspruch 1, worin in Formel (1) X eine durch die Formel (4) repräsentierte Gruppe ist.

4. Bis(2-aryl-5-pyridyl)-Derivat oder ein Salz davon nach Anspruch 1, worin die aromatische Kohlenwasserstoffgruppe substituiert ist durch ein bis drei Substituenten, ausgewählt aus Niederalkylgruppen, Niederalkoxygruppen, Halogen(niederalkoxy)gruppen, Niederalkoxy(niederalkoxy)gruppen, Hydroxy(niederalkoxy)gruppen, Carboxylgruppen, (Niederalkoxy)carbonylgruppen, unsubstituierten oder (Niederalkyl)- und/oder (Niederalkoxy)-substituierten Carbamoylgruppen, Niederalkanoylgruppen, Formylgruppen, Niederalkanoyloxygruppen, Halogenatomen, Hydroxygruppen, Cyangruppen, (Niederalkyl)thiogruppen, Aminogruppen, Mono- oder Di(niederalkyl)aminogruppen, (Niederalkyl)sulfonylaminogruppen, Pyrrolidinylgruppen und Alkylendioxygruppen; worin der Niederalkylrest eine lineare, verzweigte oder cyclische Alkylgruppe mit 1 bis 6 Kohlenstoffatomen ist.

5. Arzneimittel, das als aktiven Bestandteil ein Bis(2-aryl-5-pyridyl)-Derivat oder ein Salz davon nach einem der Ansprüche 1 bis 4 aufweist.

6. Arzneimittel nach Anspruch 5, das ein Prophylaktikum oder Therapeutikum für eine allergische Immunerkrankung ist.

7. Arzneimittel nach Anspruch 5, das ein Prophylaktikum oder Therapeutikum für Asthma, atopische Dermatitis, allergische Rhinitis, entzündliche Colitis, Kontakt-Dermatose oder eine allergische Augenerkrankung ist.

8. Verwendung eines Bis(2-aryl-5-pyridyl)-Derivats oder eines Salzes davon nach einem der Ansprüche 1 bis 4 zur Herstellung eines Arzneimittels zur Behandlung oder Vorbeugung allergischer Immunerkrankungen.

9. Verwendung nach Anspruch 8, worin das Arzneimittel ein Prophylaktikum oder Therapeutikum für Asthma, atopische Dermatitis, allergische Rhinitis, entzündliche Colitis, Kontakt-Dermatosen oder allergische Augenerkrankungen ist.

10. IgE-Antikörper-Produktionsinhibitor, der ein Bis(2-aryl-5-pyridyl)-Derivat oder ein Salz davon nach einem der Ansprüche 1 bis 4 ist.

11. Arzneimittelzusammensetzung, die ein Bis(2-aryl-5-pyridyl)-Derivat oder ein Salz davon nach einem der Ansprüche 1 bis 4 und einen pharmakologisch annehmbaren Träger umfasst.

## Revendications

1. Dérivé bis(2-aryl-5-pyridyle) représenté par la formule (1) suivante ou sel de celui-ci : 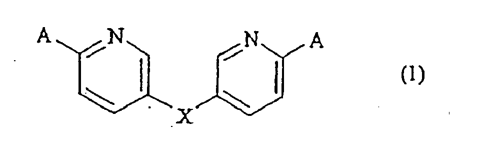 où A représente un groupe hydrocarboné aromatique substitué ou non substitué, et X représente un groupe choisi parmi les formules (2) à (4) suivantes : 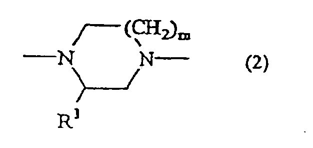 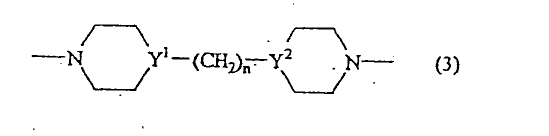 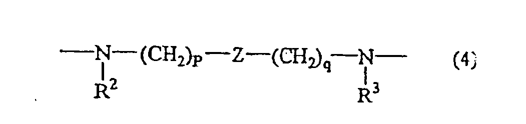 où dans laquelle dans la formule (2), R¹ représente un atome d'hydrogène ou un groupe di(alkyl inférieur)aminoalkyle inférieur, et m représente l'entier 1 ou 2 ; dans la formule (3), Y¹ et Y² représentent un atome d'azote ou un groupe CH, et n représente un entier de 0 à 6 ; dans la formule (4), R² et R³ représentent un atome d'hydrogène ou un groupe alkyle inférieur, Z représente une liaison simple, un groupe méthylène substitué, un groupe imino substitué, un atome d'oxygène ou un groupe cycloalkylène, et p et q représentent un entier de 0 à 6 ; le fragment alkyle inférieur étant un groupe alkyle linéaire, ramifié ou cyclique ayant de 1 à 6 atome(s) de carbone.

2. Dérivé bis(2-aryl-5-pyridyle) ou sel de celui-ci selon la revendication 1, pour lequel dans la formule (1), A est un groupe phényle substitué ou non substitué.

3. Dérivé bis(2-aryl-5-pyridyle) ou sel de celui-ci selon la revendication 1, pour lequel dans la formule (1), X est un groupe représenté par la formule (4).

4. Dérivé bis(2-aryl-5-pyridyle) ou sel de celui-ci selon la revendication 1, dans lequel ledit groupe hydrocarboné aromatique est substitué par 1 à 3 substituant(s) choisi(s) parmi des groupes alkyle inférieurs, des groupes alkoxy inférieurs, des groupes halogénoalkoxy inférieurs, des groupes (alkoxy inférieur)alkoxy inférieurs, des groupes hydroxyalkoxy inférieurs, des groupes carboxyle, des groupes (alkoxy inférieur)carbonyle, des groupes carbamoyle non substitués ou substitués par alkyle inférieur et/ou alkoxy inférieur, des groupes alcanoyle inférieurs, des groupes formyle, des groupes alcanoyloxy inférieurs, des atomes d'halogène, des groupes hydroxy, des groupes cyano, des groupes (alkyl inférieur)thio, des groupes amino, des groupes mono- ou di-(alkyl inférieur)amino, des groupes (alkyl inférieur)sulfonylamino, des groupes pyrrolidinyle, et des groupes alkylènedioxy ; le fragment alkyle inférieur étant un groupe alkyle linéaire, ramifié ou cyclique ayant de 1 à 6 atome(s) de carbone.

5. Médicament comprenant, en tant qu'ingrédient actif, un dérivé bis(2-aryl-5-pyridyle) ou un sel de celui-ci selon l'une quelconque des revendications 1-4.

6. Médicament selon la revendication 5, qui est un produit préventif ou thérapeutique pour une maladie immune allergique.

7. Médicament selon la revendication 5, qui est un produit préventif ou thérapeutique pour l'asthme, la dermatite atopique, la rhinite allergique, la colite inflammatoire, une maladie de peau par contact ou une maladie allergique des yeux.

8. Utilisation du dérivé bis(2-aryl-5-pyridyle) ou d'un sel de celui-ci selon l'une quelconque des revendications 1-4 pour la fabrication d'un médicament pour prévenir ou traiter des maladies immunes allergiques.

9. Utilisation selon la revendication 8, dans laquelle le médicament est un produit préventif ou thérapeutique pour l'asthme, la dermatite atopique, la rhinite allergique, la colite inflammatoire, des maladies de peau par contact ou des maladies allergiques des yeux.

10. Inhibiteur de la production d'anticorps IgE qui est un dérivé bis(2-aryl-5-pyridyle) ou un sel de celui-ci selon l'une quelconque des revendications 1-4.

11. Composition médicamenteuse comprenant un dérivé bis(2-aryl-5-pyridyle) ou un sel de celui-ci selon l'une quelconque des revendications 1-4 et un véhicule pharmacologiquement acceptable.
